# EUROPEAN PATENT SPECIFICATION

(11) **EP 3 733 658 B1**
(45) Date of publication and mention of the grant of the patent: **22.02.2023**
(21) Application number: 18897200.4
(22) Date of filing: 21.12.2018
(51) Int. Cl.: C07D 311/72, A61K 31/355, A61P 3/06, A61P 3/10

(54) **PRENYLATED BENZOPYRANES AS PPAR AGONISTS**
PRENYLIERTE BENZOPYRANE ALS PPAR-AGONISTEN
BENZOPYRANES PRÉNYLÉS AGONISTES DE PPAR

(30) Priority: 26.12.2017 ES 201731470
(43) Date of publication of application: 04.11.2020
(73) Proprietor: Universitat de València, 46010 Valencia (ES); Fundación Incliva, 46010 Valencia (ES)
(72) Inventor: CORTÉS MARTÍNEZ, Diego Miguel, 46980 Valencia (ES); SANZ FERRANDO, María Jesús, 46005 Valencia (ES); CABEDO ESCRIG, Nuria, 46010 Valencia (ES); BERMEJO DEL CASTILLO, Almudena, 46010 Valencia (ES); PIQUERAS RUIZ, Laura, 46010 Valencia (ES); COLLADO SÁNCHEZ, Aída, 46022 Valencia (ES); GOMES MARQUES, Patrice, 46010 Valencia (ES); VILA DASÍ, Laura, 46010 Valencia (ES)
(74) Representative: Manuel Illescas y Asociados, S.L.
(86) International application number: PCT/ES2018/070826
(87) International publication number: WO 2019/129909

(56) References cited:
- EP-A2- 0 421 419
- WO-A2-02/26729
- M. CARMEN GONZALEZ ET AL: "Polycerasoidin and Polycerasoidol, Two New Prenylated Benzopyran Derivatives from Polyalthia cerasoides", JOURNAL OF NATURAL PRODUCTS, vol. 58, no. 8, 1 August 1995 (1995-08-01) , pages 1278-1284, XP055622434, US ISSN: 0163-3864, DOI: 10.1021/np50122a022
- WAI SAN CHEANG ET AL: "The peroxisome proliferator-activated receptors in cardiovascular diseases: experimental benefits and clinical challenges : PPARs and vascular function", BRITISH JOURNAL OF PHARMACOLOGY, vol. 172, no. 23, 23 January 2015 (2015-01-23), pages 5512-5522, XP55744626, UK ISSN: 0007-1188, DOI: 10.1111/bph.13029
- TAN CHEK KUN et al.: "Synthetic and natural Peroxisome Proliferator-Activated Receptor (PPAR) agonists as candidates for the therapy of the metabolic syndrome", Expert Opinion on Therapeutic Targets MAR 2017, vol. 21, no. 3, 28 February 2017 (2017-02-28), pages 333-348, XP055622430, ISSN: 1472-8222, DOI: 10.1080/14728222.2017.1280467
- PEARCE B C et al.: "HYPOCHOLESTEROLEMIC ACTIVITY OF SYNTHETIC AND NATURAL TOCOTRIENOLS", Journal of Medicinal Chemistry 1992, vol. 35, no. 20, 30 November 1991 (1991-11-30), pages 3595-3606, XP002393508, ISSN: 0022-2623, DOI: doi:10.1021/jm00098a002
- GONZALEZ M CARMEN et al.: "Polycerasoidin and polycerasoidol, two new prenylated benzopyran derivatives from Polyalthia cerasoides", Journal of Natural Products (Lloydia) 1995, vol. 58, no. 8, 30 November 1994 (1994-11-30), pages 1278-1284, XP055622434, ISSN: 0163-3864, DOI: 10.1021/np50122a022
- GONZALEZ M CARMEN et al.: "Prenylated benzopyran derivatives from two Polyalthia species", Phytochemistry ( Oxford ) 1996, vol. 43, no. 6, 30 November 1995 (1995-11-30), pages 1361-1364, XP055622438, ISSN: 0031-9422, DOI: 10.1016/S0031-9422(96)00450-5
- SANZ M -J et al.: "A novel dual PPARa and PPARy agonist inhibit TNFa - induced mononuclear cell recruitment", Basic and Clinical Pharmacology and Toxicology, vol. 123, no. 2, 1 August 2018 (2018-08-01), page 44, ISSN: 1742-7843

## Description

### PURPOSE OF THE INVENTION

The present invention discloses compounds comprising a prenylated benzopyran core structure which display activity as agonists of PPARα, PPARγ, or dual activity as agonists of PPARα and PPARγ. The invention therefore refers to compounds with said prenylated benzopyran structure of formula I disclosed in the claims, the pharmaceutically acceptable salts thereof, first medical use, and the use of said prenylated benzopyran compounds in the treatment of diseases responding to the administration of PPARα and /or PPARγ agonists disclosed in the claims. Said diseases are disorders such as type 2 diabetes (T2D), obesity, metabolic syndrome, cardiovascular disorders, hypercholesterolemia, hypertriglyceridemia (serious and moderate), primary chylomicronemia, familial dysbetalipoproteinemia, hyperlipoproteinemia, inflammation and neurodegenerative diseases, as Parkinson's, Alzheimer's or amyotrophic lateral sclerosis (ALS).

### BACKGROUND OF THE INVENTION

Peroxisome proliferation activator receptors (PPAR) belong to the family of nuclear hormonal receptors. There are three types of PPAR: PPARα (NR1C1), PPARβ/δ (NUC1; NR1C2), and PPARγ (NR1C3). They all act as heterodimers with 9-cys-retinoic acid receptors (retinoid X receptor; RXR) and play a key role in the regulation of various metabolic pathways, including lipid biosynthesis and glucose metabolism, as well as in cell differentiation and proliferation and in apoptosis (Cheang *et al.,* 2015, Ahmadian *et al.,* 2013 and Tan *et al.,* 2017).

PPAR are considered powerful therapeutic tools for the control of various pathologies, such as type 2 diabetes (T2D), obesity, hyperlipidemia, cardiovascular disorders, inflammation and neurodegenerative diseases. PPAR are also important due to their involvement in the control of inflammatory processes, and can regulate inflammation, vascular function and vascular remodeling (Cheang *et al.,* 2015, Ahmadian *et al.,* 2013 and Tan *et al.,* 2017).

PPARα are mainly found in the liver, kidneys, heart, muscle and adipose tissue, and play an essential role in the oxidation of fatty acids and the metabolism of lipoproteins.

PPARγ are mainly present in adipose tissue, macrophages, monocytes, intestinal cells, skeletal muscle and endothelium. They are related to lipid metabolism, adipogenesis, glucose homeostasis and insulin sensitization.

PPAR ligands, and in particular agonists PPARα and PPARγ, inhibit the expression of inflammation genes and can interfere negatively with signaling pathways for proinflammatory transcription factors in vascular and inflammatory cells.

PPARα are activated by the fibrate compounds: fenofibrate, chlorfibrate, benzafibrate, and similar compounds such as WY-14643:

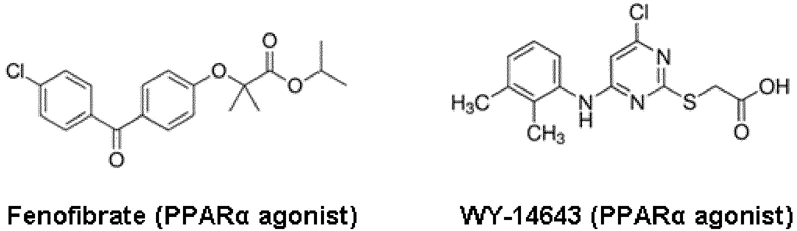

PPARα agonists can reduce triglyceride levels and increase HDL-c, and are highly effective in the treatment of hypertriglyceridemia.¹

Among the known PPARγ ligands of these receptors stand out thiazolidinedione (TZD) type agonists, among which are rosiglitazone and pioglitazone, which have been used clinically in the treatment of T2D due to their capacity to reduce glucose levels in blood and improve sensitivity to insulin (Ahmadian *et al.,* 2013).

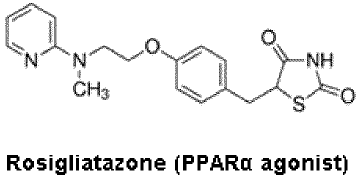

The development of PPARα or PPARγ agonists, or of dual PPARα/PPARγ agonists, has led to the synthesis of a new class of candidates with therapeutic potential (Cheang *et al.,* 2015 and Tan *et al.,* 2017). In fact, dual PPARα/γ agonists are considered to be more potent compounds in the correction of lipid and glucose homeostasis in T2D and metabolic syndrome than selective agonists (Tan *et al.,* 2017). Despite the clinical effectiveness of PPARα and PPARγ agonists in the treatment of dyslipidemias and T2D, respectively, the appearance of numerous adverse effects has limited the use of these selective agonists, requiring the withdrawal of numerous drugs. In particular, PPARα agonists can lead to increased levels of creatinine and transaminases, and more rarely myopathy, while PPARγ agonists are related to weight gain, edema and myocardial infarction (Cheang *et al*., 2015). The possibility of finding a single compound that can activate the two receptors PPARα and PPARγ, a dual agonist, is seen as a potential alternative in the treatment of T2D and metabolic syndrome with fewer side effects (Cheang *et al.,* 2015 and Tan *et al.,* 2017). The use of two PPARα/γ dual agonists belonging to the group of glitazars has been recently approved, saroglitazar in India (Lipaglyn^{™}, by the Zydus Cadila research centre) and lobeglitazone in Korea (Duvie^{™}, by Chong Kun Dang), for lipid and glycemic control (Tan *et al.,* 2017).

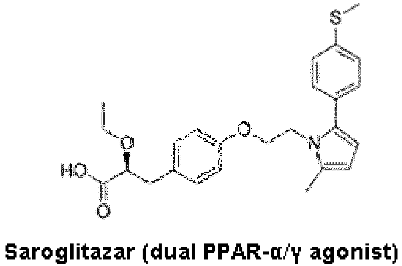

However, other dual agonists such as farglitazar and muraglitazar have been withdrawn from the market due to their side effects in edema, myocardial infarction, stroke and heart failure, as have aleglitazar (in clinical phase II), ragaglitazar and tesaglitazar, since they have shown carcinogenicity in rodent models and increased creatinine in plasma (Tan *et al.,* 2017). WO0226729 A2 discloses benzopyrancarboxylic acid derivatives which are PPARα and/or PPARγ agonists. Pearce et al (1991) disclose the use of tocotrienols in the treatment of hypercholesterolemia. Additionally, Sanz M-J et al. (2018) disclosed dual PPARα and PPARγ with a chromane moiety.

The development of hPPARα and hPPARy dual agonists allows combining the properties of PPARα agonists (fibrates) on the regulation of lipid metabolism, including reduction of triglycerides (TG), with the additional effect of the improved insulin sensitivity produced by PPARγ agonists (TZD). These hPPARα/γ dual ligands have generally shown greater effectiveness and lower toxicity in animals for the treatment of metabolic disorders than the use of selective compounds for each receptor. In fact, clinical studies show that PPARα/γ dual agonists reduce insulin resistance in type 2 diabetes, glycaemia and TG values in plasma, and participate in the control of vascular inflammation. Therefore, the search for PPARα/γ dual agonists is important for the treatment of various metabolic disorders such as metabolic syndrome, T2D, and cardiovascular diseases.

Consequently, there is a need to develop PPARα and /or γ agonists that would show a greater effectiveness than the currently available ones, and /or also would show lower toxicity.

### DESCRIPTION

The present invention is defined by the claims and refers to a group of PPARα and /or PPARγ agonist compounds included in said claims and pharmaceutical compositions comprising the same. The present invention also describes the PPARα and /or PPARγ agonists included in the claims, and pharmaceutical compositions comprising the same for use as a medicament, and for use in the treatment of a disease that responds to the administration of PPARα and /or PPARγ agonists independently selected from the group consisting of cardiovascular disorders, type 2 diabetes (T2D), obesity, metabolic syndrome, hypercholesterolemia, hypertriglyceridemia, primary chylomicronemia, hyperlipoproteinemia, familial dysbetalipoproteinemia, inflammation and neurodegenerative diseases.

The subject matter disclosed herein which is not part of the claims or, which is not part of embodiments of the invention, has been marked as being examples of the present disclosure. Said examples are included for illustration purposes only in order to aid in understanding the invention which is defined in the claims.

One example of the PPARα and /or PPARγ agonist compounds described herein refers to a compound of general formula I, stereoisomers and pharmaceutically acceptable salts thereof: 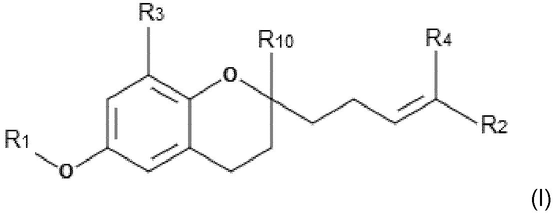 wherein
- R₁ is independently selected from H or a phenol protecting group;
- R₂ is independently selected from an alkyl-ester group with structure: 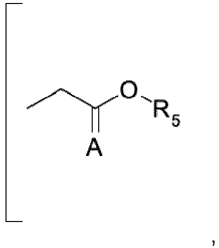
   a prenyl group derivative with structure: 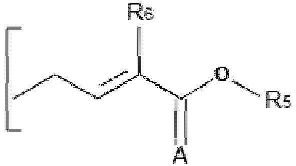
   or an acyl -C(O)OR^{b} group, wherein R^{b} is a C₁₋₆ alkyl group,
   wherein
      - A is independently selected from an O atom, a S atom or a NR₇ group, wherein R₇ is independently selected from H, OR₈ or a C₁₋₆ alkyl group, and wherein R₈ is a H group or a C₁₋₆ alkyl group;
      - R₅ is independently selected from H or a carboxylic acid protecting group;
      - R₆ is C₁₋₆ alkyl or OR₉, wherein R₉ is C₁₋₆ alkyl;
   and wherein
      - R₃ is H or a C₁₋₆ alkyl group;
      - R₄ is a C₁₋₆ alkyl group or an alkoxide;
      - R₁₀ is independently selected from a C₁₋₆ alkyl, allyl, alkylamine, alkylamide, alkylaryl, alkylether, haloalkyl, silyl, carbamate, alkylsulphone or haloalkylsulphone group.

In an example disclosed herein when R₁ is H and R₃ is methyl, R₅ is other than H or methyl, and when R₁ is methyl and R₃ is methyl, R₅ is other than H or methyl.

One example refers to a compound of general formula I, described above, to its enantiomers, E,Z isomers and pharmaceutically acceptable salts thereof.

For the purposes of the present discloseure, the term "phenol protecting group" is defined as a group substituting the H of the OH phenol group used to prevent the same from being reactive, or as a group that improves the pharmacodynamics and pharmacokinetics of the molecule. Non-limiting examples of phenol protecting groups, among others, are: C₁₋₆ alkyl (e.g. methyl, ethyl, propyl, etc.); allyl; alkylamine (e.g. methyl- isoquinolein); alkylamide (e.g. acetamide); alkylaryl (e.g. benzyl, p-fluorobenzyl, p-methoxybenzyl); alkyl-ether (e.g. 2-methoxyethoxymethyl); halo-alkyl (e.g. trifluoromethyl, fluoroethyl, chloroethyl); silyl (e.g. alkyltrimethylsilanes, allyltrimethylsilanes); carbamates C(O)NRₘRₚ, wherein Rₘ and/or Rₚ are each one and independently H, C₁₋₆ alkyl or aryl; an acyl group (e.g. benzoyl), an alkylsulphone or a halo-alkylsulphone (e.g. trifluoromethylsulphone).

In one example disclosed herein the group R₁ is a phenol protecting group independently selected from the group consisting of H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently: H, C₁₋₆ alkyl or aryl; acyl C(O)R^{a}, wherein R^{a} is an aryl group; alkylsulphone and halo-alkylsulphone.

In one example R₁ is independently selected from the group consisting of H, C₁₋₆ alkyl, allyl, alkylamide, alkylaryl, alkyl-ether, alkylbenzoyl, halo-alkyl, silyl, halo-alkylsulphone and an acyl group -C(O)R^{a}, wherein R^{a} is an aryl group.

In another example R₁ is independently selected from the group consisting of H, C₁₋₆ alkyl, alkylamide, alkylaryl, and an acyl group -C(O)R^{a}, wherein R^{a} is an aryl group.

For the purposes of the present disclosure, the term "carboxylic acid protecting group" is defined as a group substituting the H of the carboxylic acid group -COOH to prevent the same from being reactive, or as a group that improves the pharmacodynamics and pharmacokinetics of the molecule. Non-limiting examples of carboxylic acid protecting groups, among others, are: C₁₋₆ alkyl, allyl, alkylamide, alkylamine, alkylaryl, alkyl-ether, alkyl-ester, halo-alkyl, silyl, carbamate C(O)NRₘRₚ, wherein Rₘ and/or Rₚ are each one and independently H, C₁₋₆ alkyl or aryl, acyl C(O)R^{a}, wherein R^{a} is aryl, alkylsulphone or a halo-alkylsulphone group.

In one example R₅ is independently selected from H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl; alkylsulphone or halo-alkylsulphone.

In one example R₅ is independently selected from the group consisting of H, C₁₋₆ alkyl, alkylaryl and alkyl-ester.

In another example R₅ is independently selected from the group consisting of H, C₁₋₆ alkyl, aryl, alkylaryl, alkyl-ether, alkyl-ester, alkylsulphone, halo-alkyl, silyl and halo-alkylsulphone.

One example disclosed herein refers to a compound of general formula I, stereoisomers and pharmaceutically acceptable salts thereof: 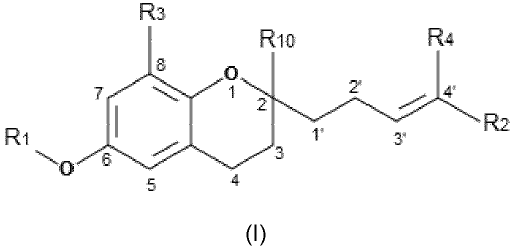 wherein
- R₁ is independently selected from H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl C(O)R^{a}, wherein R^{a} is an aryl group; alkylsulphone or halo-alkylsulphone;
- R₂ is independently selected from an alkyl-ester group with structure: 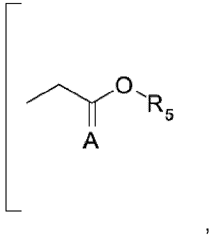
   a prenyl group derivative with structure: 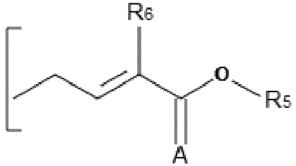
   or an acyl -C(O)OR^{b} group, wherein R^{b} is a C₁₋₆ alkyl group,
   wherein
      - A is independently selected from an O atom O, a S atom S or a NR₇ group, wherein R₇ is independently selected from H, OR₈ or a C₁₋₆ alkyl group; and wherein R₈ is an H or a C₁₋₆ alkyl group;
      - R₅ is independently selected from H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl; alkylsulphone or halo-alkylsulphone;
      - R₆ is C₁₋₆ alkyl or OR₉ wherein R₉ is C₁₋₆ alkyl;
   and wherein
      - R₃ is H or a C₁₋₆ alkyl group;
      - R₄ is a C₁₋₆ alkyl group or an alkoxide;
      - R₁₀ is independently selected from a C₁₋₆ alkyl, allyl, alkylamine, alkylamide, alkylaryl, alkylether, haloalkyl, silyl, carbamate, alkylsulphone or haloalkylsulphone group.

In an example disclosed herein when R₁ is H and R₃ is methyl, R₅ is other than H or methyl, and when R₁ is methyl and R₃ is methyl, R₅ is other than H or methyl.

For the purposes of the present disclosure, a compound of formula I includes all the stereoisomers of said formula I.

For the purposes of the present disclosure, the term "stereoisomer" refers to molecules with the same molecular formula and the same sequence of atoms bonded (same constitution), and with the same bonds between the atoms, but which differ in the three-dimensional orientation of their atoms in space. Enantiomers and diastereomers are two different types of stereoisomers.

For the purposes of the present disclosure the term "enantiomer" refers to two stereoisomers that are mirror images of each other and cannot be superimposed on each other.

Specifically, one example disclosed herein comprises the R and S enantiomers of the compounds of formula I, where said enantiomers are the 2 possible enantiomers with respect to the C-2 position of the benzopyran core, therefore including the R and S configurations of the radical R₁₀.

For the purposes of the present disclosure the term "diastereomer" refers to two stereoisomers that are not mirror images of each other. Diastereomers include meso compounds, E/Z isomers and non-enantiomeric optical isomers.

E/Z isomers refer to compounds comprising C=C double bonds, that is, alkenes or cycloalkenes. If in a C=C double bond the two higher priority substituents, according to the IUPAC (International Union of Pure and Applied Chemistry) rules, are on the same side of the bond, the arrangement is Z. However, if they are on opposite sides the arrangement is E.

One example disclosed herein refers to a compound of general formula I, described above, to its enantiomers, E,Z isomers and pharmaceutically acceptable salts thereof.

An example disclosed comprises the R enantiomer with respect to the C-2 position of the benzopyran core.

Another example disclosed comprises the S enantiomer with respect to the C-2 position of the benzopyran core.

One example disclosed comprises the E isomer with respect to the double bond of C-'3 position in the prenylated side chain.

One example disclosed comprises the Z isomer with respect to the double bond of C-'3 position in the prenylated side chain.

For the purposes of the present disclosure the term pharmaceutically acceptable salt refers to any salt which, when administered to a patient, can deliver (directly or indirectly) the compound in question to the patient. Said salts are preferably addition salts of organic or inorganic acids or, of organic or inorganic bases. Examples of addition salts of acids include, but are not limited to, addition salts of inorganic acids such as, e.g., chloride, bromide, iodide, sulphate, nitrate, phosphate, or addition salts of organic acids such as, e.g., acetate, trifluoroacetate, maleate, fumarate, citrate, oxalate, succinate or tartrate. Examples of addition salts of bases include, but are not limited to, addition salts of inorganic bases such as, e.g., sodium, potassium, calcium or ammonium, or addition salts of organic bases such as, e.g., ethylenediamine, ethanolamine, N,N-dialkylethanolamine, triethanolamine and aminoacid basic salts. The methods for forming the salts are convention methods of the art.

For the purposes of the present disclosure, the term "C₁₋₆ alkyl" refers to a hydrocarbon chain of 1 to 6 carbon atoms, fully saturated, that can be linear or branched.

For the purposes of the present disclosure, the term "halo-alkyl" refers to a hydrocarbon chain, linear or branched, in which at least one H has been substituted with a halogen atom.

For the purposes of the present disclosure, the term "allyl" refers to a hydrocarbon chain, linear or branched, which comprises at least one -CH=CH-CH₂- group.

For the purposes of the present disclosure, the term "aklylamide" refers to a hydrocarbon chain, linear or branched, substituted with an amide group.

For the purposes of the present disclosure, the term "alkylamine" refers to a hydrocarbon chain, linear or branched, substituted with an amine group.

For the purposes of the present disclosure the term "aryl" refers to a group derived from an aromatic hydrocarbon formed by removing one hydrogen atom from an aromatic ring in said aromatic hydrocarbon. For the purposes of the present disclosure said aryl groups can be optionally substituted. Non-limiting examples of substitutions are OH, alkoxide, alkyl, halogen, halogenated alkyl, cyano, thiol, amine or alkylamine.

For the purposes of the present disclosure the term "alkylaryl" refers to a hydrocarbon chain substituted with an aryl group, which is, in turn, defined as described above.

For the purposes of the present disclosure, the term "alkoxide" refers to an oxygen atom linked to a hydrocarbon chain, where said hydrocarbon chain may comprise an alkyl and/or an aryl group, as defined above.

For the purposes of the present disclosure the term "alkyl ether" refers to a saturated hydrocarbon chain substituted with an alkoxide group, which is defined as described above.

For the purposes of the present disclosure, the term "acyl" refers to a carbonyl group linked to a hydrocarbon chain, where said hydrocarbon chain may comprise an alkyl and/or an aryl group, as defined above.

For the purposes of the present disclosure the term "ester" refers to a group derived from a carboxylic acid -COOH, where the hydroxyl group -OH is replaced by an alkoxide group, as defined according to the present invention.

For the purposes of the present disclosure the term "alkyl ester" refers to a saturated hydrocarbon chain substituted with an ester group, as defined above.

In an example disclosed herein A is an O atom.

In another example disclosed herein A is a S atom.

In another example disclosed herein A is a NR₇ group, where R₇ is independently selected from H, OR₈ or a C₁₋₆ alkyl group, and wherein R₈ is an H group or a C₁₋₆ alkyl group. In one example R₇ is H, -OH or -OCH₃.

In an example disclosed hereinR₁ is H.

In another example disclosed R₁ is a C₁₋₆ alkyl group. In one example R₁ is a methyl or propyl group.

In another example disclosed R₁ is an alkylaryl group. In one example R₁ is a benzyl or 4-fluorobenzyl group.

In another example disclosed R₁ is an alkylamide group. In one example R₁ is an acetamide group.

In another example disclosed R₁ is an acyl group -C(O)R^{a}. In one example R1 is an acyl group -C(O)R^{a}, wherein R^{a} is an aryl group. In one example R^{a} is a phenyl group.

In one example R₃ is H.

In another example R₃ is methyl.

In one example R₄ is C₁₋₆ alkyl or an alkoxide group. In one example the alkoxide group is a halogenated alkoxide group.

In one example R₄ is methyl.

In one example R₄ is -OCH₂CF₃.

In one example R₅ is H.

In another example R₅ is C₁₋₆ alkyl. More preferably R₅ is methyl, ethyl, propyl, butyl or tert-butyl. In one example R₅ is methyl, ethyl or propyl.

In another example R₅ is alkylaryl. In one example R₅ is benzyl.

In another example R₅ is alkyl ester. In one example R₅ is -CH₂COOCH₃ or -CH(COOCH₃)₂.

In one example R₆ is methyl.

In another example R₆ is OR₉ wherein R₉ is C₁₋₆ alkyl. More preferably R₉ is ethyl.

In one example R₁₀ is C₁₋₆ alkyl. More preferably R₁₀ is methyl.

As disclosed in the claims, the invention refers to a compound selected independently from the group consisting of 3, 4, 4a, 4b, 4c, 6, 8, 9,10, 11, 12 and 13:

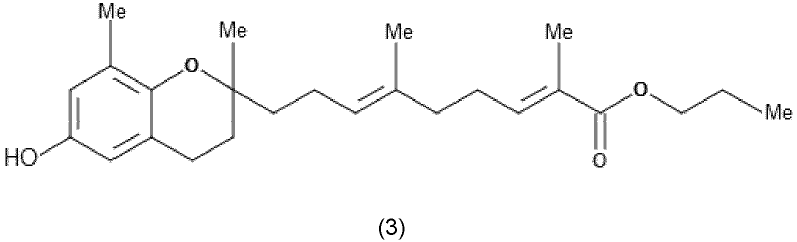

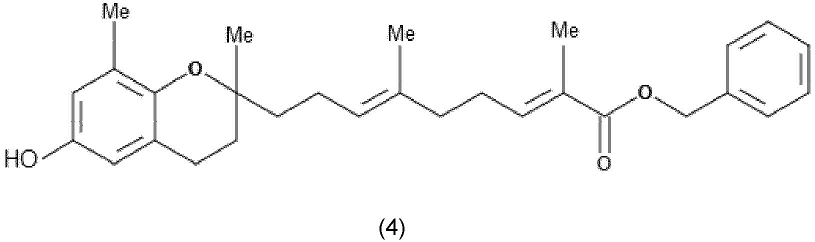

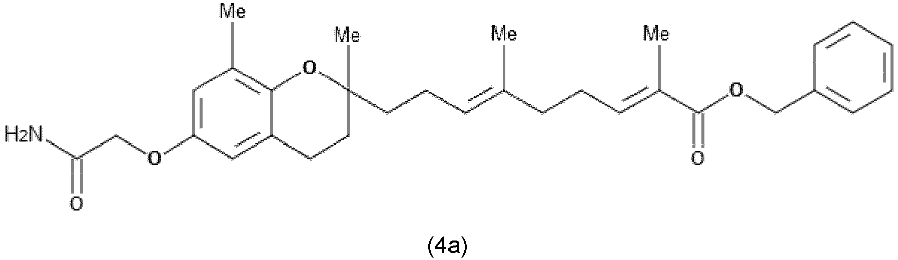

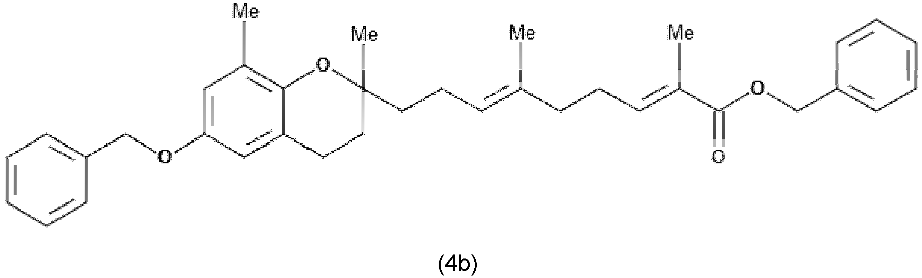

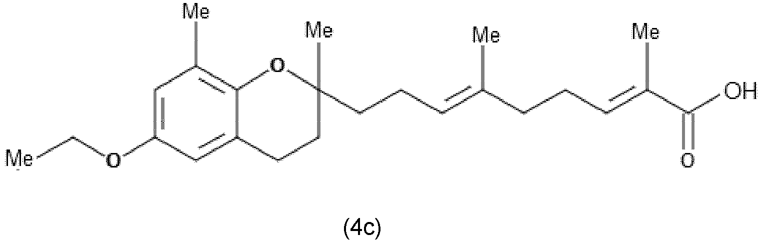

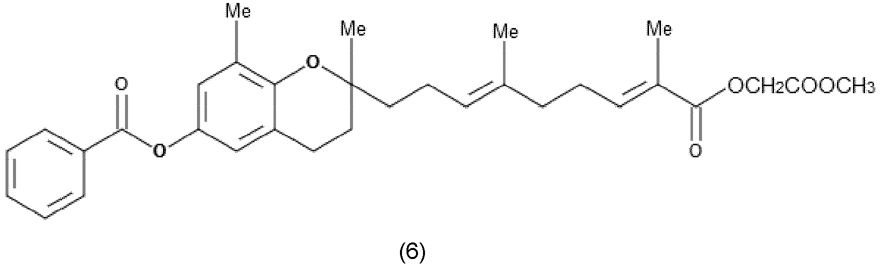

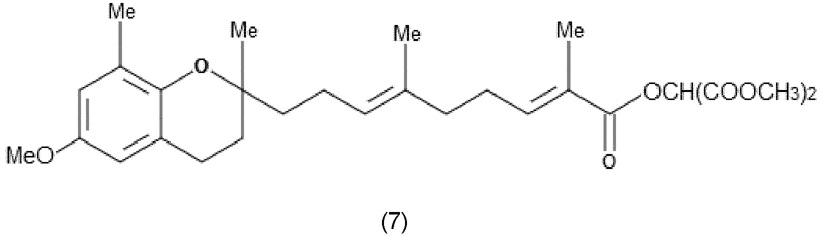

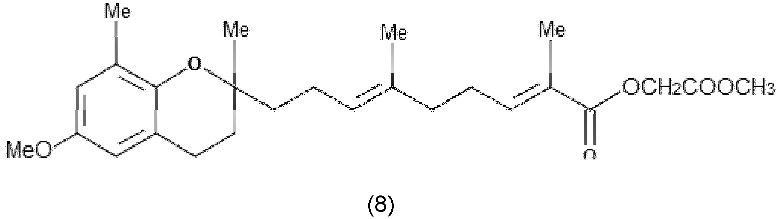

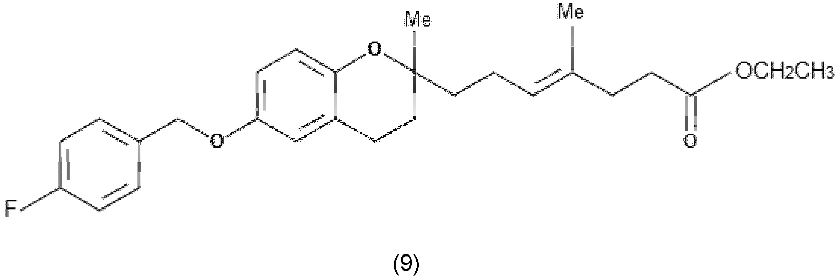

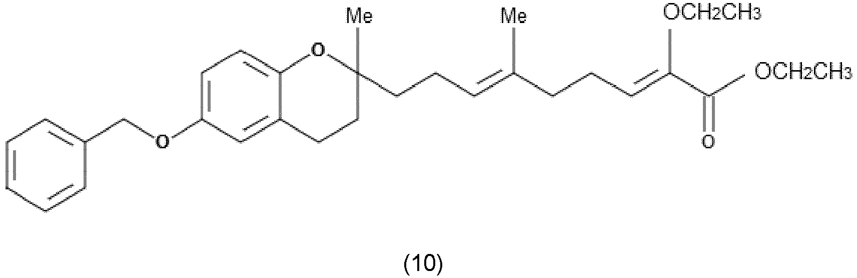

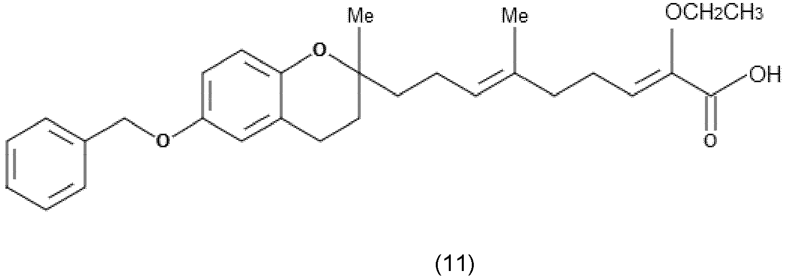

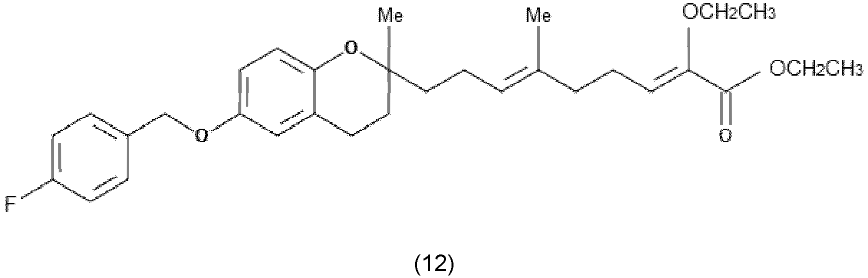

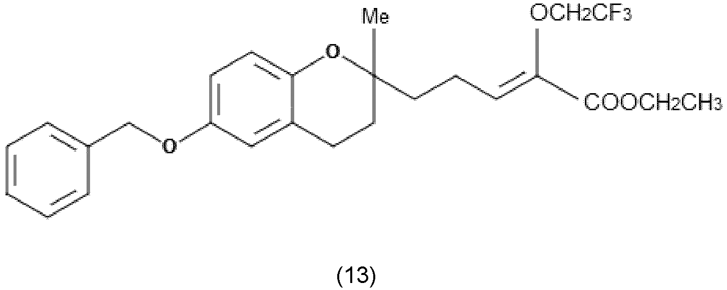

One example disclosed comprises the enantiomer R of the compounds 3, 4, 4a, 4b, 4c, 6, 7, 8, 9, 10, 11, 12 and 13, with respect to the position C-2 of the benzopyran core.

Another example disclosed comprises the enantiomer S of the compounds 3, 4, 4a, 4b, 4c, 6, 7, 8, 9, 10, 11, 12 and 13, with respect to the position C-2 of the benzopyran core.

One example disclosed comprises the isomer (*E*,*Z*) of the compounds 3, 4, 4a, 4b, 4c, 6, 7, 8, 10, 11 and 12, with respect to the positions C-'3 and C-'7 of the prenylated side chain.

Another example disclosed comprises the isomer (*E*,*E*) of the compounds 3, 4, 4a, 4b, 4c, 6, 7, 8,10, 11 and 12, with respect to the positions C-'3 and C-'7 of the prenylated side chain.

One example disclosed comprises the isomer (*Z*,*Z*) of the compounds 3, 4, 4a, 4b, 4c, 6, 7, 8, 10, 11 and 12, with respect to the positions C-'3 and C-'7 of the prenylated side chain.

Another example disclosed comprises the isomer (*Z*,*E*) of the compounds 3, 4, 4a, 4b, 4c, 6, 7, 8, 10, 11 and 12, with respect to the positions C-'3 and C-'7 of the prenylated side chain.

One example disclosed comprises the isomer *E* of the compounds 9 and 13, with respect to the position C-'3 of the prenylated side chain.

Another example disclosed comprises the isomer Z of the compounds 9 and 13, with respect to the position C-'3 of the prenylated side chain.

The compounds of formula I, stereoisomers and pharmaceutically acceptable salts thereof, with PPAR agonist activity, disclosed in the present document, comprise a common structure derived from the natural prenylated benzopyrans polycerasoidol and polycerasoidin (Gonzalez *et al.,* 1995 and 1996), natural isomers of compounds 1 and 2 described herein: The isomer (3*E*, 7*Z*) of compound 1 corresponds to the natural compound polycerasoidol, while the isomer (3*E*, 7*E*) is the natural compound isopolycerasoidol, and polycerasoidin corresponds to the isomer (3*E*,7*Z*) of compound 2:

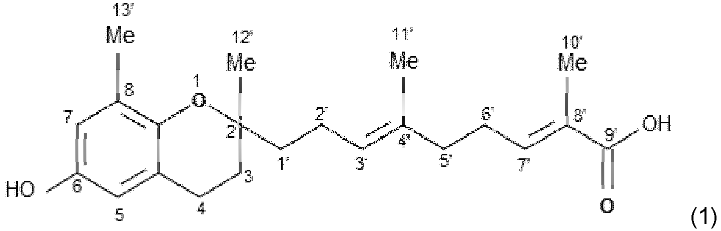

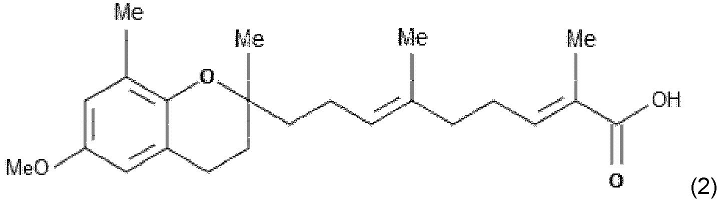

Said documents, by Gonzalez et al. (1995 and 1996), describe the isolation of the natural compounds polycerasoidol and polycerasoidin from the bark of the tree genus *Polyalthia,* indicating that some of the compounds of the family of prenylated hydroquinones to which they belong are known to have cytotoxic or antioxidant properties, without making reference to, or giving indications on, a possible activity as PPAR agonists. In the same sense, other documents disclose said compounds, or compounds with a similar structure, although said compounds also lack activity as PPAR agonists. In particular, documents Taha et al., 2015 and Karimian et al. (2015) describe the use of polycerasoidol, its methyl ester, and polycerasoidin as inducing apoptosis in breast cancer tumor cells. Zhao et al. (2010) analyses various metabolites derived from tocopherol and tocotrienols detected in urine, serum or liver, in human and mouse samples, among which polycerasoidol is described. Zafra-Polo et al. (1996) describes polyalthidin, related to the basic structure of the compounds of the invention, but which is described only with regard to its activity as an inhibitor of the mitochondrial respiratory chain. Lastly, document EP0421419A describes benzopyran core compounds with three prenylated chains, making reference to their use to reduce serum cholesterol levels. None of these documents describes or gives indications on the possible use of similar compounds having a prenylated benzopyran core as PPAR agonists.

One example comprises the enantiomer *R* of the compounds 1 and 2, with respect to the position C-2 of the benzopyran core.

Another example comprises the enantiomer S of the compounds 1 and 2, with respect to the position C-2 of the benzopyran core.

One example comprises the isomer (*E*,*E*) of the compounds 1 and 2, with respect to the positions C-'3 and C-`7 of the prenylated side chain.

Another example comprises the isomer (*E*,*Z*) of the compounds 1 and 2, with respect to the positions C-'3 and C-`7 of the prenylated side chain.

One example comprises the isomer (*Z*,*Z*) of the compounds 1 and 2, with respect to the positions C-'3 and C-`7 of the prenylated side chain.

Another example comprises the isomer (*Z*,*E*) of the compounds 1 and 2, with respect to the positions C-'3 and C-`7 of the prenylated side chain.

One example relates to a compound of formula I, stereoisomers and pharmaceutically acceptable salts thereof: 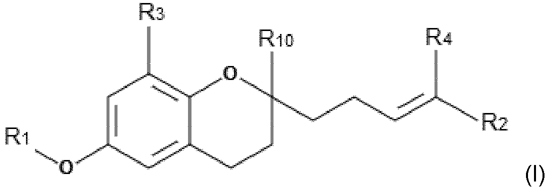
wherein R₃, R₁₀ and R₄ are methyl and R₂ is a derivative of a prenyl group with structure: 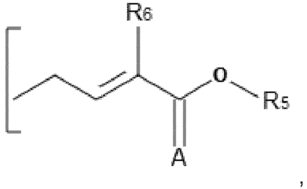 wherein A is an O atom and R₆ is methyl,
and wherein said compound of formula I is a compound of formula II, stereoisomers and pharmaceutically acceptable salts thereof: 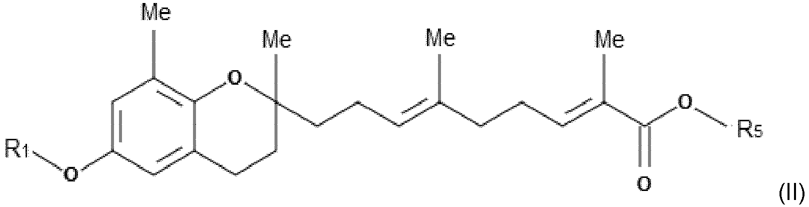
wherein R₁ and R₅ are defined according to the embodiments of the present invention described above.

Said compounds of formula II, therefore, include the compounds 1, 2 and derived compounds with different groups R₁ and R₅.

In one example, when R₁ is H, R₅ is other than H or methyl, and when R₁ is Me, R₅ is other than H or methyl.

One example comprises the *R* enantiomer of the compounds of formula II, with respect to the position C-2 of the benzopyran core.

Another example comprises the enantiomer R of the compounds of formula II, with respect to the position C-2 of the benzopyran core.

One example comprises the isomer (*E*,*Z*) of the compounds of formula II, with respect to the positions C-'3 and C-`7 of the prenylated side chain.

Another example comprises the isomer (*E*,*E*) of the compounds of formula II, with respect to the positions C-'3 and C-`7 of the prenylated side chain.

One example comprises the isomer (*Z*,*Z*) of the compounds of formula II, with respect to the positions C-'3 and C-`7 of the prenylated side chain.

Another example comprises the isomer (*Z*,*E*) of the compounds of formula II, with respect to the positions C-'3 and C-`7 of the prenylated side chain.

In one example, R₁ is H and R₅ is H.

In another example, R₁ is C₁₋₆ alkyl and R₅ is H. In one example R₁ is methyl and R₅ is H.

In another example, R₁ is H and R₅ is C₁₋₆ alkyl. In one example R₁ is H and R₅ is methyl or propyl.

In one example, R₁ is H and R₅ is alkylaryl. In one example R₁ is H and R₅ is benzyl.

In another example, R₁ is alkylamide and R₅ is alkylaryl. In one example R₁ is acetamide, and R₅ is benzyl.

In another example, R₁ and R₅ are alkylaryl. In one example R₁ and R₅ are benzyl.

In another example, R₁ is C₁₋₆ alkyl and R₅ is H. In one example R₁ is propyl and R₅ is H.

In another example, R₁ is an acyl group -C(O)R^{a}, wherein R^{a} is an aryl group, and R₅ is an alkyl ester group. In one example R₁ is a benzoyl group and R₅ is a -CH₂COOCH₃ group.

In another example, R₁ is a C₁₋₆ alkyl group and R₅ is an alkyl ester group. In one example R₁ is a methyl group and R₅ is a -CH₂COOCH₃ group or a -CH(COOCH₃)₂ group.

Both compounds 1 and 2 feature a similar structure and only differ in the functional group in position C-6, which is a hydroxyl group in compound 1 and a methoxy group in compound 2. Departing from compounds 1 and 2 the compounds 3, 4, 4a,4b, 4c, 6, 7 or 8 of formula I, of the present invention, can be synthesized by substituting the positions C-9' and C-6, as is described in Example 1 below.

One example of the compound of formula II, stereoisomers and pharmaceutically acceptable salts thereof, as described above, is a compound independently selected from compounds 1, 2, 3, 4, 4a, 4b, 4c, 6, 7 or 8.

A preferred embodiment of the present invention refers to a compound selected independently from the group consisting of 3, 4, 4a, 4b, 4c, 6, 7 and 8. In one example disclosed the compounds 3, 4, 4a, 4b, 4c, 6, 7 and 8 are (*E*,*Z*) isomers, with respect to the double bonds of the prenylated side chain in positions C-3' and C-7'.

Another example disclosed herein relates to a compound of formula I, stereoisomers and pharmaceutically acceptable salts thereof: 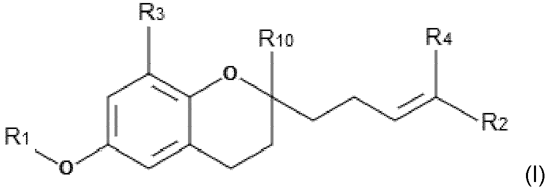
wherein R₃ is H, R₄ and R₁₀ are methyl and R₂ is a prenyl group derivative with structure: 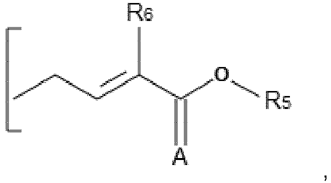 wherein A is an O atom,
and wherein said compound of formula I is a compound of formula III, stereoisomers and pharmaceutically acceptable salts thereof: 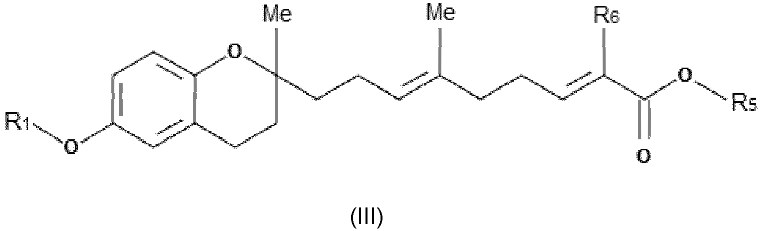
wherein R₁, R₅, and R₆ are defined according to the examples described in the present document.

Said compounds of formula III also present a structure derived from the compounds 1 and 2, but, unlike the compounds of formula II, feature a H instead of methyl as the R₃ group, and, in addition to the modifications in positions C-6 (-OR₁) and C-9' (-COOR₅), also feature modifications in position C-8' (R₆).

One example comprises the enantiomer R of the compounds of formula III, with respect to the position C-2 of the benzopyran core.

Another example comprises the enantiomer R of the compounds of formula III, with respect to the position C-2 of the benzopyran core.

One example comprises the isomer (*E*,*Z*) of the compounds of formula III, with respect to the positions C-'3 and C-`7 of the prenylated side chain.

Another example comprises the isomer (*E*,*E*) of the compounds of formula III, with respect to the positions C-'3 and C-`7 of the prenylated side chain.

One example comprises the isomer (*Z*,*Z*) of the compounds of formula III, with respect to the positions C-'3 and C-`7 of the prenylated side chain.

Another example comprises the isomer (*Z*,*E*) of the compounds of formula III, with respect to the positions C-'3 and C-`7 of the prenylated side chain.

In one example, R₁ is an alkylaryl group, R₆ is an -OR₉ group, wherein R₉ is C₁₋₆ alkyl, and R₅ is H or a C₁₋₆ alkyl group. More preferably R₁ is a benzyl group, R₆ is a -OCH₂CH₃ group, and R₅ is H or ethyl.

In one example R₁ is a 4-fluorobenzyl group, R₆ is a -OCH₂CH₃ group, and R₅ is ethyl.

One embodiment of present invention is a compound that is independently selected from compounds 10, 11 and 12, as disclosed in the claims:

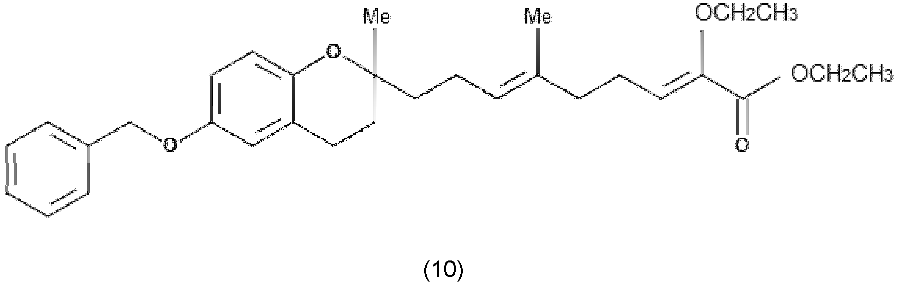

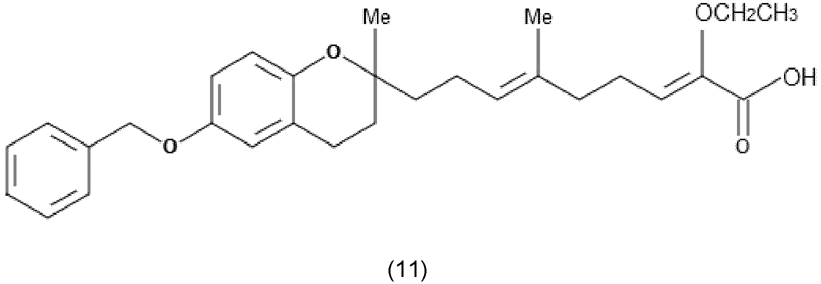

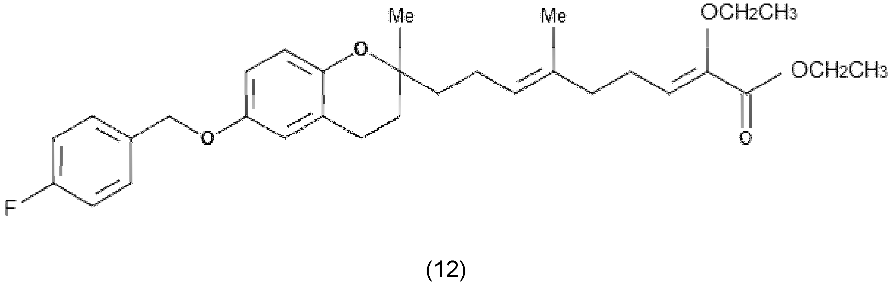

Another example disclosed herein refers to a compound of formula I, stereoisomers and pharmaceutically acceptable salts thereof: 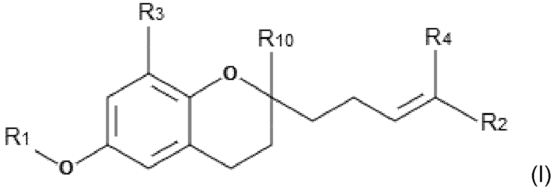
wherein R₃ is H, R₄ and R₁₀ are methyl and R₂ is an alkyl-ester group with structure: 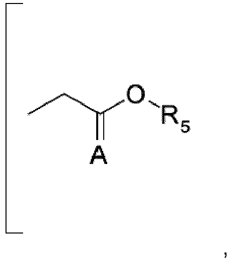 wherein A is an O atom,
and wherein said compound of formula I is a compound of formula IV, stereoisomers and pharmaceutically acceptable salts thereof: 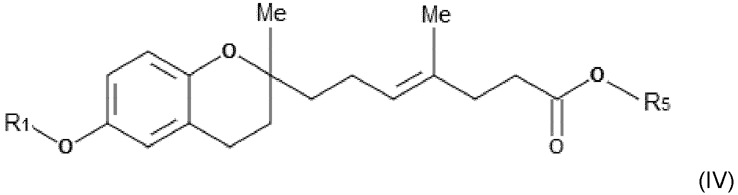
wherein R₁ and R₅ are defined according to the examples described herein.

Said compounds of formula IV also feature a structure derived from the compounds of formula I, 1 and 2. However, the compounds of formula IV are compounds of formula I wherein the radical R₃ is H, and not methyl as it is in the compounds 1 and 2, and the radical R₂ is an alkyl-ester group which, unlike in the compounds 1 and 2, does not feature a second prenyl group.

One example comprises the enantiomer R of the compounds of formula IV, with respect to the position C-2 of the benzopyran core.

Another example comprises the enantiomer *S* of the compounds of formula IV, with respect to the position C-2 of the benzopyran core.

One example comprises the isomer *E* of the compounds of formula IV, with respect to the double bond of the prenylated side chain.

Another example comprises the isomer *Z* of the compounds of formula IV, with respect to the double bond of the prenylated side chain.

In one example R₁ is alkylaryl and R₅ is C₁₋₆ alkyl. More preferably R₁ is 4-fluorobenzyl and R₅ is ethyl.

One compound of present invention is compound 9, as disclosed in the claims:

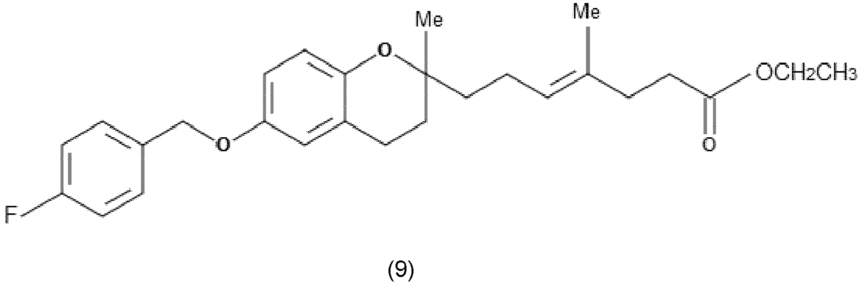

Another example disclosed herein refers to a compound of formula I, stereoisomers and pharmaceutically acceptable salts thereof: 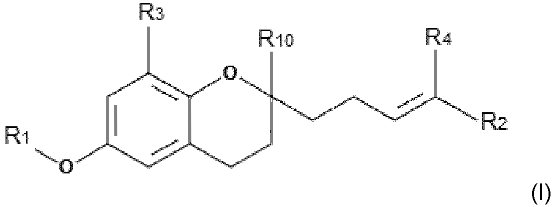
wherein R₃ is H, R₁₀ is methyl and R₂ is -C(O)OR^{b}, and wherein said compound of formula I is a compound of formula V, stereoisomers and pharmaceutically acceptable salts thereof: 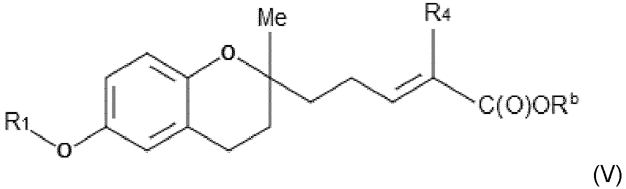
wherein R^{b} is a C₁₋₆ alkyl group, and R₁ and R₄ are defined according to the examples described herein.

Said compounds of formula V also feature a structure derived from the compounds of formula I, 1 and 2, but wherein said formula I the radical R₂ is an ester group, instead of a prenylated alkyl-ester group featured in compounds 1 and 2.

One example comprises the enantiomer *R* of the compounds of formula V, with respect to the position C-2 of the benzopyran core.

Another example comprises the enantiomer S of the compounds of formula V, with respect to the position C-2 of the benzopyran core.

One example comprises the isomer *E* of the compounds of formula V, with respect to the double bond of the prenylated side chain.

Another example comprises the isomer *Z* of the compounds of formula V, with respect to the double bond of the prenylated side chain.

In one example R₁ is alkylaryl, R^{b} is C₁₋₆ alkyl and R₄ is an alkoxide group. In one example R₁ is benzyl, R^{b} is ethyl and R₄ is -OCH₂CF₃.

One compound of present invention is compound 13, as disclosed in the claims:

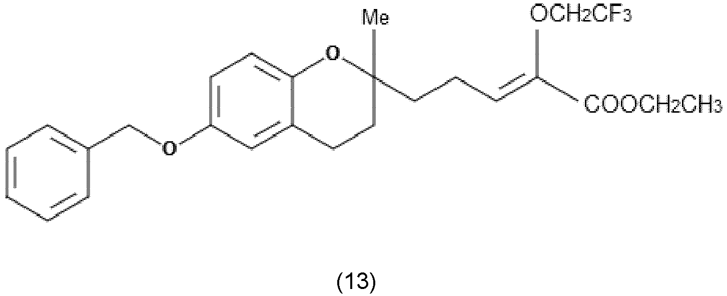

An example disclosed herein refers to a pharmaceutical composition comprising an effective amount of at least a compound of formula I, stereoisomers and pharmaceutically acceptable salts thereof, as described above, and at least one pharmaceutically acceptable excipient.

For the purposes of the present disclosure an effective amount is defined as an amount of the compound that provides an objectively identifiable improvement in the condition of the patient, recognized by a qualified observer, and where said patient is treated with a pharmaceutical composition comprising said amount of the compound.

For the purposes of the present disclosure, pharmaceutically acceptable excipients are inert ingredients such as, but not limited to, cosolvents, surfactants, oils, wetting agents, emollients, preservatives, stabilizers and antioxidants.

An example disclosed herein refers to a compound of formula I, stereoisomers and pharmaceutically acceptable salts thereof: 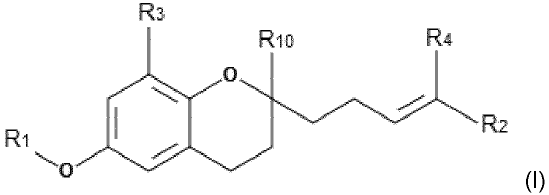 wherein
- R₁ is H or a phenol protecting group;
- R₂ is independently selected from an alkyl-ester group with structure: 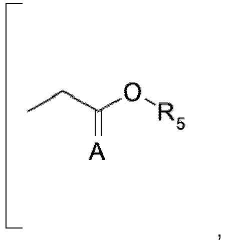
   a prenyl group derivative with structure: 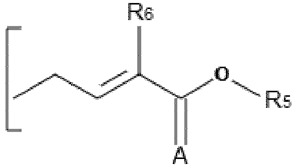
   or an acyl -C(O)OR^{b} group, wherein R^{b} is a C₁₋₆ alkyl group,
   wherein
      - A is independently selected from an O atom, a S atom or a NR₇ group, wherein R₇ is independently selected from H, OR₈ or a C₁₋₆ alkyl group, and wherein R₈ is H or a C₁₋₆ alkyl group;
      - R₅ is independently selected from H or a carboxylic acid protecting group;
      - R₆ is C₁₋₆ alkyl or OR₉, wherein R₉ is C₁₋₆ alkyl;
wherein
- R₃ is H or a C₁₋₆ alkyl group; and
- R₄ is a C₁₋₆ alkyl group or an alkoxide;
- R₁₀ is independently selected from a C₁₋₆ alkyl, allyl, alkylamine, alkylamide, alkylaryl, alkylether, haloalkyl, silyl, carbamate, alkylsulphone or haloalkylsulphone group; and
when R₁ is H and R₃ is methyl, R₅ is other than H or methyl, and when R₁ is methyl and R₃ is methyl, R₅ is other than H or methyl; or a pharmaceutical composition comprising the same, for use as a medicament.

Another example disclosed herein refers to a compound of formula I, stereoisomers and pharmaceutically acceptable salts thereof: 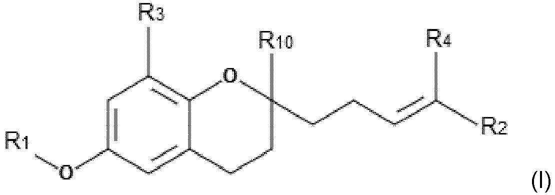 wherein
- R₁ is independently selected from the group consisting of H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl C(O)R^{a}, wherein R^{a} is an aryl group; alkylsulphone and halo-alkylsulphone;
- R₂ is independently selected from an alkyl-ester group with structure: 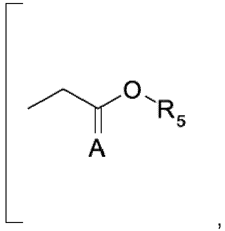
   a prenyl group derivative with structure: 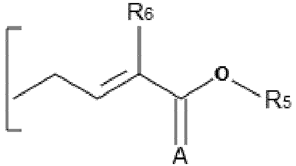
   or an acyl -C(O)OR^{b} group, wherein R^{b} is a C₁₋₆ alkyl group,
   wherein
      - A is independently selected from an O atom, a S atom or a NR₇ group, wherein R₇ is independently selected from H, OR₈ or a C₁₋₆ alkyl group, and wherein R₈ is H or a C₁₋₆ alkyl group;
      - R₅ is independently selected from H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl C(O)R^{a}, wherein R^{a} is an aryl group; alkylsulphone or halo-alkylsulphone;
      - R₆ is C₁₋₆ alkyl or OR₉ wherein R₉ is C₁₋₆ alkyl;
wherein
- R₃ is H or a C₁₋₆ alkyl group; and
- R₄ is a C₁₋₆ alkyl group or an alkoxide;
- R₁₀ is independently selected from a C₁₋₆ alkyl, allyl, alkylamine, alkylamide, alkylaryl, alkyléter, haloalkyl, silyl, carbamate, alkylsulphone or haloalkylsulphone group; and
when R₁ is H and R₃ is methyl, R₅ is other than H or methyl, and when R₁ is methyl and R₃ is methyl, R₅ is other than H or methyl; or a pharmaceutical composition comprising the same, for use as a medication.

An example of the compound of formula I, or of a composition comprising the same, for use as a medicament, as described above, comprises any of the enantiomers thereof or mixtures of the same, and any of the E/Z enantiomers thereof and mixtures of the same, and their pharmaceutically acceptable salts. One example comprises the enantiomer R. Another example comprises the enantiomer S. One example comprises the isomer E. Another example comprises the isomer Z.

A preferred embodiment of the invention refers to a compound independently selected from the group consisting of 3, 4, 4a, 4b, 4c, 6, 7, 8, 9, 10, 11, 12 and 13, or a pharmaceutical composition comprising the same, for use as a medicament, as disclosed in the claims.

The compounds of formula I described in the present disclosure are agonists of PPARα and/or PPARγ. Consequently, said compounds are useful in the treatment of diseases responding to the administration of PPARα and/or PPARγ agonists, that is, diseases mediated by PPARα and/or PPARγ agonists, which is to say diseases whose treatment benefits from the administration of PPARα and PPARγ agonists.

As indicated above, PPAR are considered to be powerful therapeutic tools for controlling cardiovascular disorders, type 2 diabetes (T2D), obesity, metabolic syndrome, hypercholesterolemia, hypertriglyceridemia, primary chylomicronemia, hyperlipoproteinemia, familial dysbetalipoproteinemia, inflammation and neurodegenerative diseases such as Parkinson's, Alzheimer's or amyotrophic lateral sclerosis (ALS) (Ahmadian *et al.,* 2013). Moreover, PPAR are also important due to their involvement in the control of inflammatory processes, and thus can regulate inflammation, vascular function, and vascular remodeling.

An example herein disclosed relates to a compound of formula I, stereoisomers and pharmaceutically acceptable salts thereof: 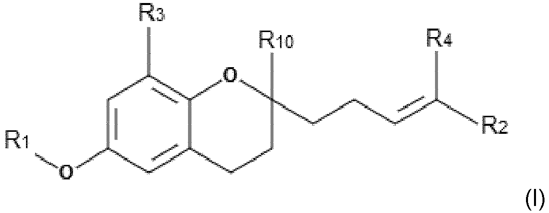 wherein
- R₁ is H or a phenol protecting group;
- R₂ is independently selected from an alkyl-ester group with structure: 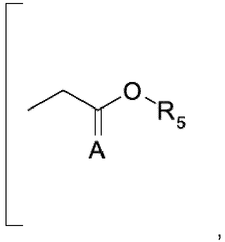
   a prenyl group derivative with structure: 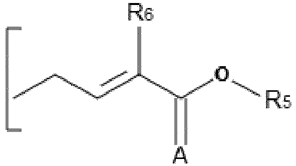
   or an acyl -C(O)OR^{b} group, wherein R^{b} is a C₁₋₆ alkyl group,
   wherein
      - A is independently selected from an O atom, a S atom or a NR₇ group, wherein R₇ is independently selected from H, OR₈ or a C₁₋₆ alkyl group, and wherein R₈ is H or a C₁₋₆ alkyl group;
      - R₅ is H or a carboxylic acid protecting group;
      - R₆ is C₁₋₆ alkyl or OR₉, wherein R₉ is C₁₋₆ alkyl;
and wherein
- R₃ is H or a C₁₋₆ alkyl group; and
- R₄ is a C₁₋₆ alkyl group or an alkoxide; and
- R₁₀ is independently selected from a C₁₋₆ alkyl, allyl, alkylamine, alkylamide, alkylaryl, alkylether, haloalkyl, silyl, carbamate, alkylsulphone or haloalkylsulphone group;
or a pharmaceutical composition comprising the same, for use in the treatment of a disease that responds to the administration of PPARα and/or PPARγ agonists.

For example the disease that responds to the administration of PPARα and/or PPARγ agonists is independently selected from cardiovascular disorders, type 2 diabetes (T2D), obesity, metabolic syndrome, hypercholesterolemia, hypertriglyceridemia, primary chylomicronemia, hyperlipoproteinemia, familial dysbetalipoproteinemia, inflammation and neurodegenerative diseases.

In one example the cardiovascular disorders refer to atherosclerosis.

In one example the neurodegenerative diseases are selected independently from Parkinson's, Alzheimer's or amyotrophic lateral sclerosis (ALS).

An example disclosed herein refers to a compound of formula I, stereoisomers and pharmaceutically acceptable salts thereof: 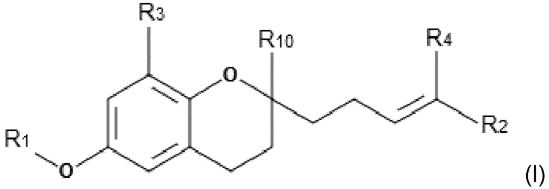 wherein
- R₁ is independently selected from the group consisting of H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl C(O)R^{a}, wherein R^{a} is an aryl group; alkylsulphone and halo-alkylsulphone;
- R₂ is independently selected from an alkyl-ester group with structure: 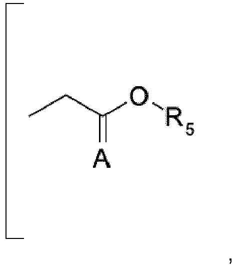
   a prenyl group derivative with structure: 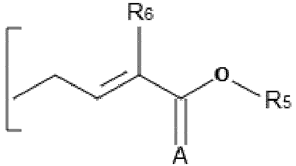
   or an acyl -C(O)OR^{b} group, wherein R^{b} is a C₁₋₆ alkyl group,
   wherein
      - A is independently selected from an O atom, a S atom or a NR₇ group, wherein R₇ is independently selected from H, OR₈ or a C₁₋₆ alkyl group, and wherein R₈ is H or a C₁₋₆ alkyl group;
      - R₅ is independently selected from H; C₁₋₆ alkyl; allyl; alkylamide; alkylamine; alkylaryl; alkyl-ether; halo-alkyl; silyl; C(O)NRₘRₚ, wherein Rₘ and/or Rₚ are each one and independently H, C₁₋₆ alkyl or aryl; acyl C(O)R^{a}, wherein R^{a} is is an aryl group; alkylsulphone or halo-alkylsulphone;
      - R₆ is C₁₋₆ alkyl or OR₉, wherein R₉ is C₁₋₆ alkyl;
and wherein
- R₃ is H or a C₁₋₆ alkyl group; and
- R₄ is a C₁₋₆ alkyl group or an alkoxide; and
- R₁₀ is independently selected from a C₁₋₆ alkyl, allyl, alkylamine, alkylamide, alkylaryl, alkylether, haloalkyl, silyl, carbamate, alkylsulphone or haloalkylsulphone group;
or a pharmaceutical composition comprising the same, for use in the treatment of a disease that responds to the administration of PPARα and/or PPARγ agonists.

One example refers to a compound of formula I or a pharmaceutical composition comprising the same, as described above, for use in the treatment of autoimmune diseases, such as type 2 diabetes (T2D).

Another example refers to a compound of formula I or a pharmaceutical composition comprising the same, as described above, for use in the treatment of atherosclerosis. Another exampple refers to a compound of formula I or a pharmaceutical composition comprising the same, as described above, for use in the treatment of atherogenesis.

Another example refers to a compound of formula I or a pharmaceutical composition comprising the same, as described above, for use in the treatment of inflammation and diseases related to inflammatory processes.

Another example refers to a compound of formula I or a pharmaceutical composition comprising the same, as described above, for use in the treatment of hypercholesterolemia.

Another example refers to a compound of formula I or a pharmaceutical composition comprising the same, as described above, for use in the treatment of hypertriglyceridemia.

Another example refers to a compound of formula I or a pharmaceutical composition comprising the same, as described above, for use in the treatment of metabolic syndrome.

Another example refers to a compound of formula I or a pharmaceutical composition comprising the same, as described above, for use in the treatment of obesity.

Another example refers to a compound of formula I or a pharmaceutical composition comprising the same, as described above, for use in the treatment of primary chylomicronemia.

Another example refers to a compound of formula I or a pharmaceutical composition comprising the same, as described above, for use in the treatment of hyperlipoproteinemia.

Another example refers to a compound of formula I or a pharmaceutical composition comprising the same, as described above, for use in the treatment of dysbetalipoproteinemia.

In one example herein disclosed when R₁ is H and R₃ is methyl, R₅ is other than H or methyl, and when R₁ is methyl and R₃ is methyl, R₅ is other than H or methyl.

An example of the compound of formula I, or of a composition comprising the same, for use in the treatment of a disease that responds to the administration of PPARα and/or PPARγ agonists, as described above, comprises any of the enantiomers thereof or mixtures of same, and any of the E/Z enantiomers thereof and mixtures of the same, and their pharmaceutically acceptable salts. One example comprises the enantiomer R. Another example comprises the enantiomer S. One example comprises the isomer E. Another example comprises the isomer Z.

Another example disclosed herein refers to a compound of formula I, stereoisomers and pharmaceutically acceptable salts thereof, that is independently selected from the group consisting of II, III, IV and V: 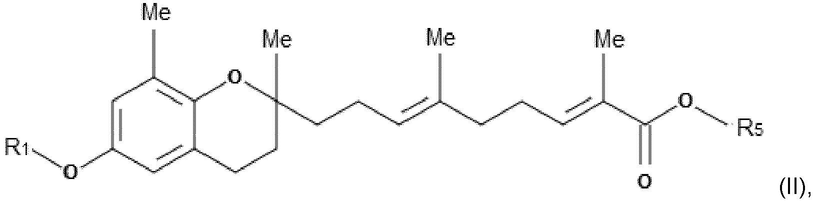 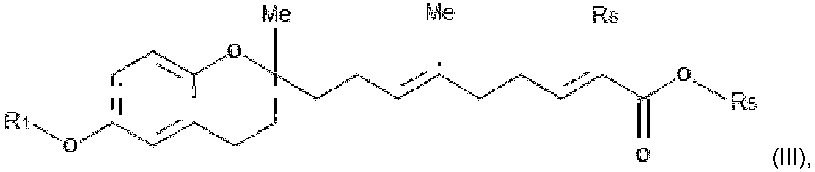 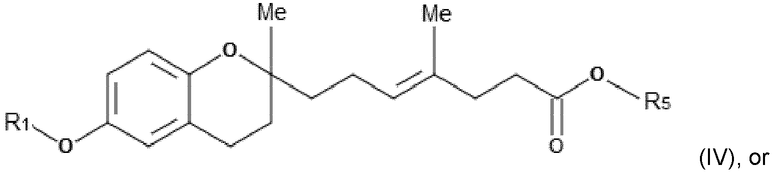 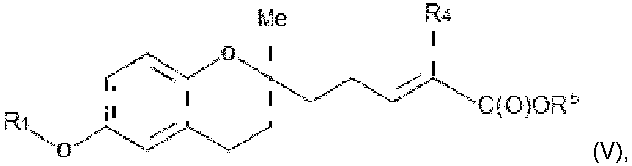 wherein R₁, R₄, R₅, R₆ and R^{b} are defined as described above, or a pharmaceutical composition comprising the same, for use in the treatment of a disease that responds to the administration of PPARα and/or PPARγ agonists.

An example of the compound of formula II, III, IV or V, or of a composition comprising one of said compounds, for use in the treatment of a disease that responds to the administration of PPARα and/or PPARγ agonists, as described above, comprises any of the enantiomers thereof or mixtures of the same, and any of the E/Z enantiomers thereof and mixtures of the same, and their pharmaceutically acceptable salts.

Another embodiment of the present invention refers to a compound independently selected from a compound 1, 2, 3, 4, 4a, 4b, 4c, 5, 6, 7, 8, 9, 10, 11, 12 or 13, or a pharmaceutical composition comprising the same, for use in the treatment of a disease that responds to the administration of PPARα and/or PPARγ agonists, wherein the disease that responds to the administration of PPARα and/or PPARγ agonists is independently selected from cardiovascular disorders, type 2 diabetes (T2D), obesity, metabolic syndrome, hypercholesterolemia, hypertriglyceridemia, primary chylomicronemia, hyperlipoproteinemia, familial dysbetalipoproteinemia, inflammation and neurodegenerative diseases, as disclosed in the claims.

Compound 5 refers to the compound of formula I:

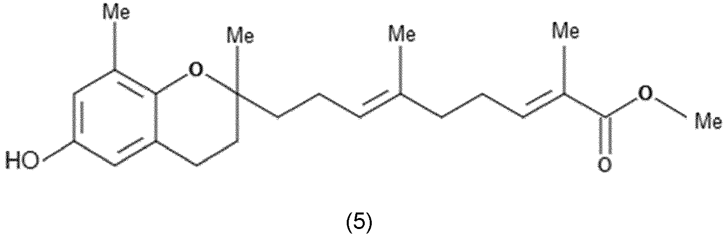

In one example the compounds 1, 2, 3, 4, 4a, 4b, 4c, 5, 6, 7, 8, 9, 10, 11, 12 and 13, for use in the treatment of a disease that responds to the administration of PPARα and/or PPARγ agonists, as described above, comprise the enantiomer R.

In another example the compounds 1, 2, 3, 4, 4a, 4b, 4c, 5, 6, 7, 8, 9, 10, 11, 12 and 13, for use in the treatment of a disease that responds to the administration of PPARα and/or PPARγ agonists, as described above, comprise the enantiomer S.

In one example the compounds 1, 2, 3, 4, 4a, 4b, 4c, 5, 6, 7, 8, 10, 11 and 12, for use in the treatment of a disease that responds to the administration of PPARα and/or PPARγ agonists, as described above, comprise the isomer (*E*,*Z*).

In another example the compounds 1, 2, 3, 4, 4a, 4b, 4c, 5, 6, 7, 8, 10, 11 and 12, for use in the treatment of a disease that responds to the administration of PPARα and/or PPARγ agonists, as described above, comprise the isomer (*E*,*E*).

In one example the compounds 1, 2, 3, 4, 4a, 4b, 4c, 5, 6, 7, 8, 10, 11 and 12, for use in the treatment of a disease that responds to the administration of PPARα and/or PPARγ agonists, as described above, comprise the isomer (*Z*,*Z*).

In another example the compounds 1, 2, 3, 4, 4a, 4b, 4c, 5, 6, 7, 8, 10, 11 and 12, for use in the treatment of a disease that responds to the administration of PPARα and/or PPARγ agonists, as described above, comprise the isomer (*Z*,E*)*.

In one example the compounds 9 and 13, for use in the treatment of a disease that responds to the administration of PPARα and/or PPARγ agonists, as described above, comprise the isomer *E*.

In another example the compounds 9 and 13, for use in the treatment of a disease that responds to the administration of PPARα and/or PPARγ agonists, as described above, comprise the isomer *Z*.

Another example relates to the use of the compounds of formula I in the treatment of diseases that respond to the administration of PPARα and/or PPARγ agonists.

Another example relates to a method of treatment comprising the administration of an effective amount of at least one compound of formula I, or of a pharmaceutical composition comprising the same, to a patient suffering from a disease that responds to the administration of PPARα and/or PPARγ agonists.

Another example refers to a method of treatment comprising the administration of an effective amount of at least one compound of formula I, selected independently from the group consisting of 1, 2, 3, 4c, 5, 7, 8, 9, 10, 11, 12 and 13, or of a pharmaceutical composition comprising the same, to a patient suffering from a disease that responds to the administration of PPARα and PPARγ dual agonists.

One example disclosed herein refers to the method for synthesizing the compounds of formula I, as described above, wherein said method comprises:
(a) condensing a 2,5-dihydroxyacetophenone and an alkylketoester to obtain a benzopyran-4-one of formula A; 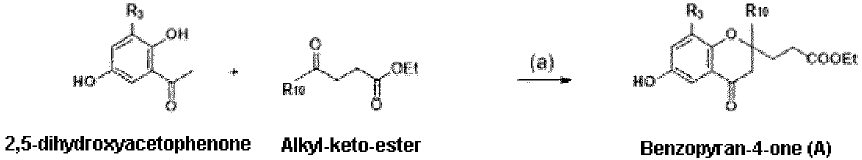
(b) reducing the ketone group of the benzopyran-4-one obtained in step (a);
(c) protecting the phenol group of the benzopyran-4-one to obtain a benzopyran ester of formula (B); 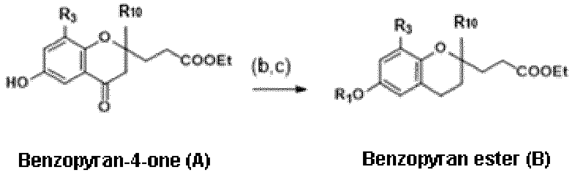
(d) reducing the ester group with suitable reagents to obtain an aldehyde; 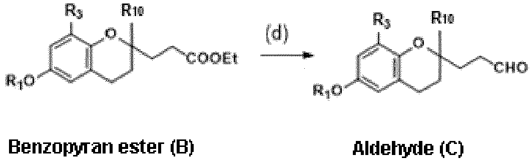
(e) reacting the aldehyde group (C), obtained in (d), with a Grignard reagent to obtain an allyl alcohol; 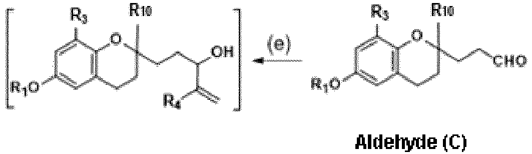 and subjecting said allyl alcohol to Johnson-Claisen transposition conditions to obtain a benzopyran ester (D); 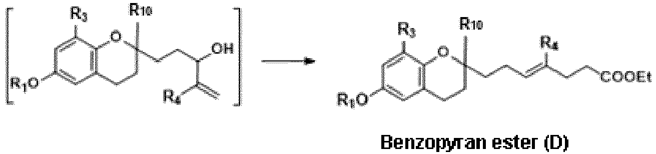 where optionally,
   - the aldehyde (C) obtained in step (d) is subjected to:
      (f) Horner-Wadsworth-Emmons olefination conditions using a phosphonate that comprises a -CH(COOR^{b})R₄ radical, or
      (g) Wittig olefination conditions using a phosphorus ylide comprising a - C(COOR^{b})R₄ radical; 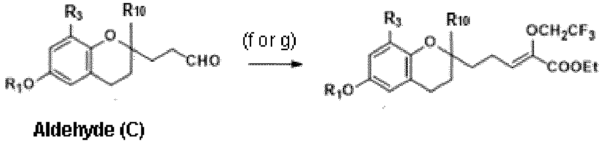
   - or wherein after performing step (e), the benzopyran ester (D) obtained is subjected to step (d): 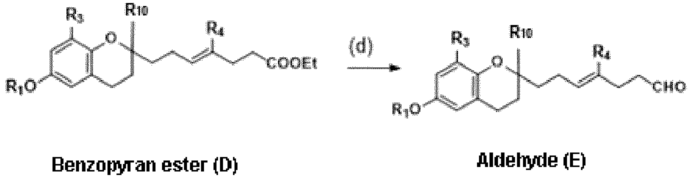 to obtain a new aldehyde (E); and wherein said new aldehyde (E) is subjected to
      (f)- Horner-Wadsworth-Emmons olefination conditions using a phosphonate comprising a -CH(COOR₅)R₆ radical, or
      (g) Wittig olefination conditions using a phosphorus ylide comprising a - C(COOR₅)R₆ radical;
      and wherein, optionally, the phenol group and/or the carboxyl group in the compound resulting from step (f) or (g) is subsequently unprotected;
and wherein R₁, R₃, R₄, R₅, R₆, R₁₀ and R^{b} are defined as previously described in the examples disclosed herein.

This synthetic method provides the compounds 1, 2, 3, 4, 4a, 4b, 4c, 5, 6, 7, 8, 9, 10, 11, 12 and 13 described above.

This synthetic method for the compounds of formula I can be summarized as follows:

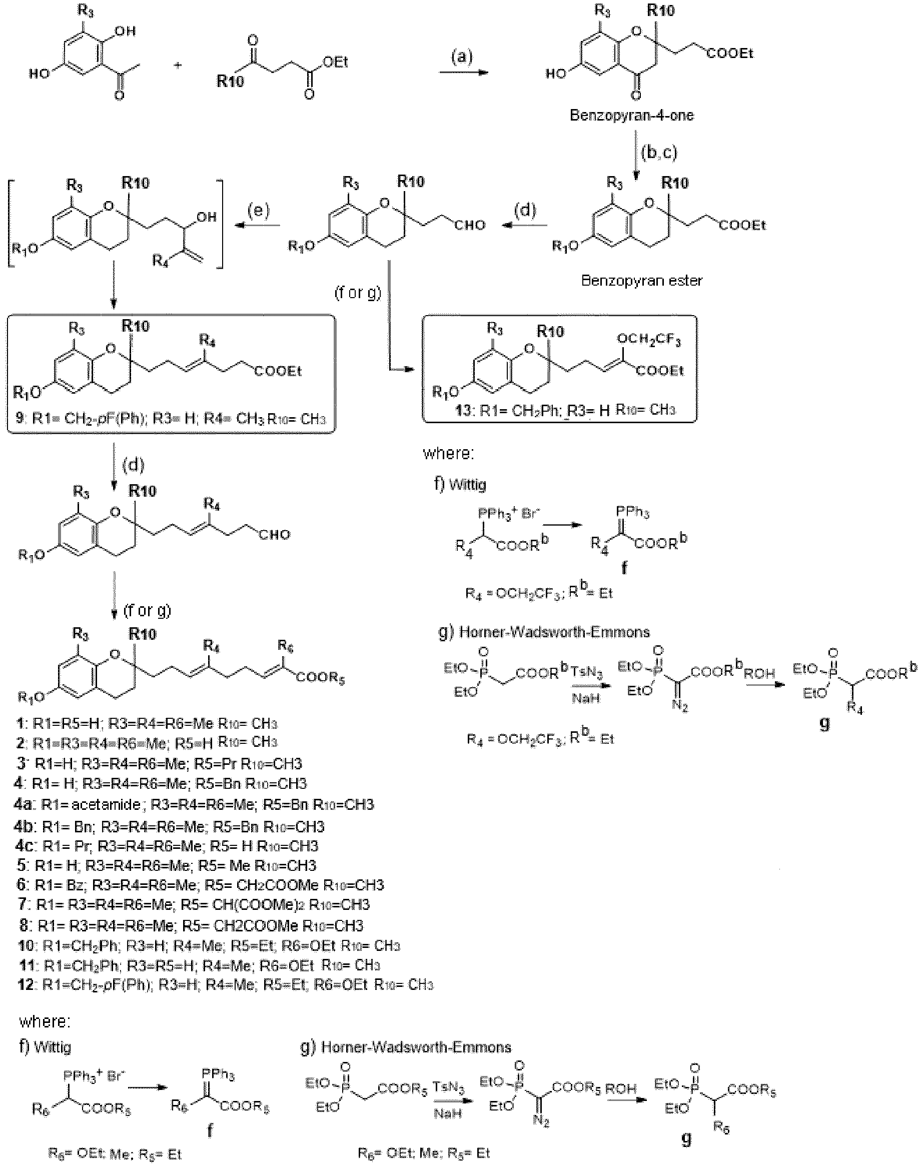

Reagents and conditions: (a) pyrrolidine, EtOH, 45°C, 24 h; (b) Zn, HCl-AcOH (2:1, v/v), amb temp, 1 h; (c) R₁X where X is a halogen, K₂CO₃, EtOH, reflux, 4 h; (d) 1M DIBAL-H (diisobutylaluminium hydride) in THF, -78 °C, 20 min; (e) 1) R₄-MgBr, - 78 °C, 3 h; 2) MeC(OR₆)₃, isobutyric acid (2 drops), 140°C, 2 h; (f) (g) tBuOK or NaH, THF, 0°C at amb temp, 16h.

The chromane core is therefore obtained by (a) condensation of 2,5-dihydroxy acetophenone and alkylketoester, producing benzopyran-4-one (Kabbe *et al.,* 1982). Then, (b) the carbonyl group is reduced by a Clemmensen reduction, and (c) the phenol group is protected. Elongation of the side chain is performed (d) by reducing the ethyl ester, forming the corresponding aldehyde. Compound **13** is obtained subjecting said aldehyde obtained in step (d) to olefination by the Wittig reaction (f) or the Horner-Wadsworth-Emmons reaction (g) (Bisceglia *et al.,* 2012 and Toshima *et al.,* 2001).

To obtain compound **9,** (e) the aldehyde obtained in step (d), is first treated with a Grignard reagent to generate an allyl alcohol which is then subjected to Johnson-Claisen transposition conditions (Sen *et al.,* 1990) to finally obtain the ester of compound **9.** The compounds **1-8** and **10-12** are obtained by a second elongation of the side chain again using the conditions of step (d) described above to reduce the ester group of compound 9, forming the corresponding aldehyde, wherein said aldehyde is then subjected to an olefination using the Wittig reaction (f) or the Horner-Wadsworth-Emmons reaction (g).

### DESCRIPTION OF THE FIGURES

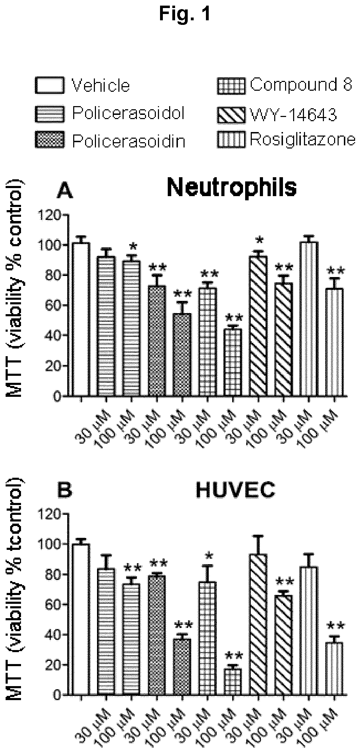

**Figure 1****.** Effect on cell viability by MTT test of the compounds polycerasoidol (1) and polycerasoidin (2) for use according to the present invention, compound 8 of the present invention, WY-14643 and rosiglitazone. In human neutrophils (**Fig. 1A**) and HUVEC (**Fig. 1B**). Data represented as mean ± SEM for n=5 independent experiments. *p <0.05 or **p <0.01 with respect to the vehicle group.

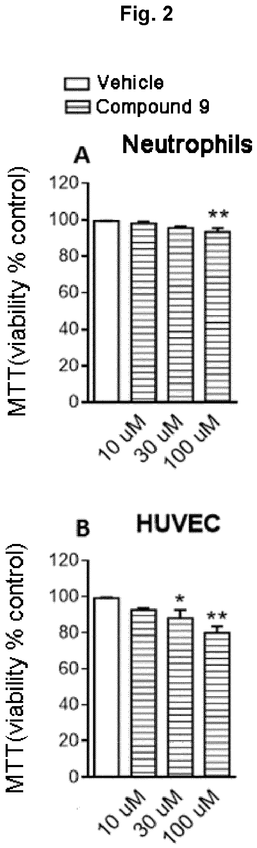

**Figure 2****.** Effect of synthetic analogue 9 of the present invention on cell viability by MTT test. In human neutrophils (**A**) and HUVEC (**B**). Data represented as mean ± SEM for n=5 independent experiments. *p <0.05 or **p <0.01 with respect to the vehicle group.

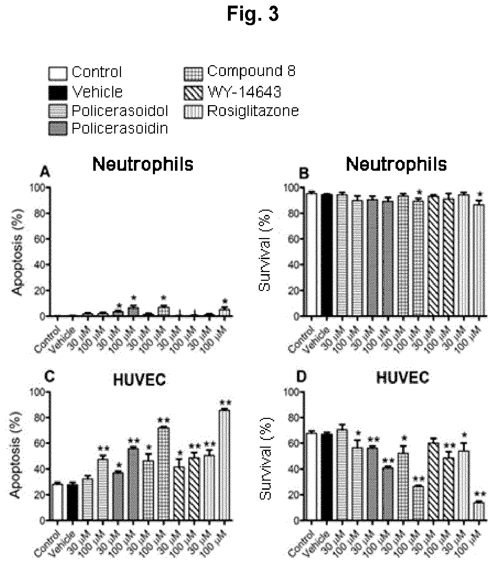

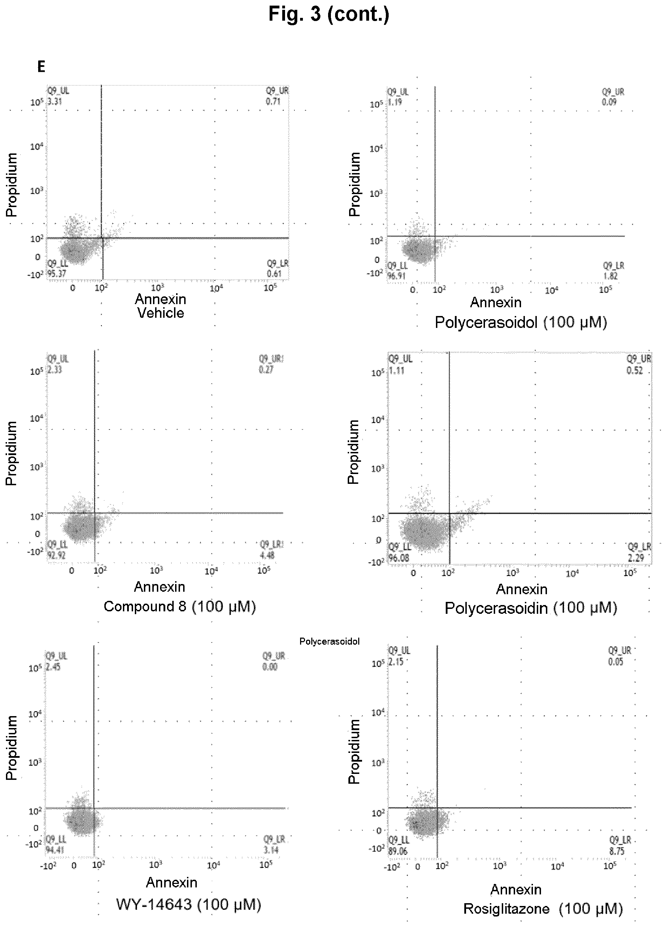

**Figure 3****.** Effect of the compounds polycerasoidol (1) and polycerasoidin (2) for use according to the present invention, compound 8 of the present invention, WY-14643 and rosiglitazone, on cell viability using flow cytometry. Percentage of apoptotic neutrophils (**Fig. 3A**), survival (**Fig. 3B**), apoptosis (**Fig. 3C**) and survival in HUVEC (**Fig. 3D**) after 24 h incubation with the compounds **1** and **2** for use according to the present invention, compound **8** of the present invention, WY-14643 and rosiglitazone. The apoptotic cells were quantified as a percentage of the total population of annexins V⁺/PI⁻ late apoptotic cells and/or necrotic cells such as annexin V⁺/PI⁺, and non-apoptotic viable cells as annexin V⁻/PI⁻. The columns correspond to the mean ± SEM for n= 3 independent experiments. The representative panels for flow cytometry have been included (**Fig. 3E**) showing the effects of the vehicle and compounds **1** and **2** for use according to the present invention, compound **8** of the present invention, WY-14643 and rosiglitazone on neutrophil apoptosis/survival. *p <0.05 or **p <0.01 with respect to the vehicle group.

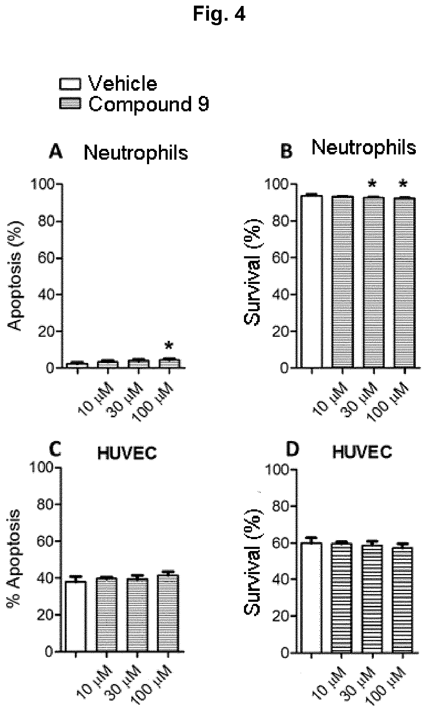

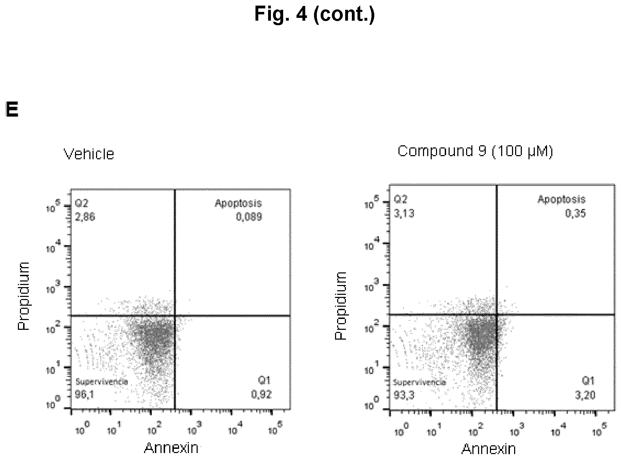

**Figure 4****.** Effect of compound 9 of the present invention on cell viability by flow cytometry. Percentage of neutrophil apoptosis (**Fig. 4A**), survival (**Fig. 4B**), apoptosis of HUVEC (**Fig. 4C**) and survival (**Fig. 4D**) after 24 h incubation. The apoptotic cells were quantified as a percentage of the total population of annexin V⁺/PI⁻ late apoptotic cells and/or necrotic cells such as annexin V⁺/PI⁺, and non-apoptotic viable cells as annexin V⁻/PI⁻. The columns correspond to the mean ± SEM for n=3 independent experiments. The representative panels for flow cytometry have been included (**Fig. 4E**) showing the effects of the vehicle and of compound **9** of the present invention on neutrophil apoptosis/survival. *p <0.05 or **p <0.01 with respect to the vehicle group.

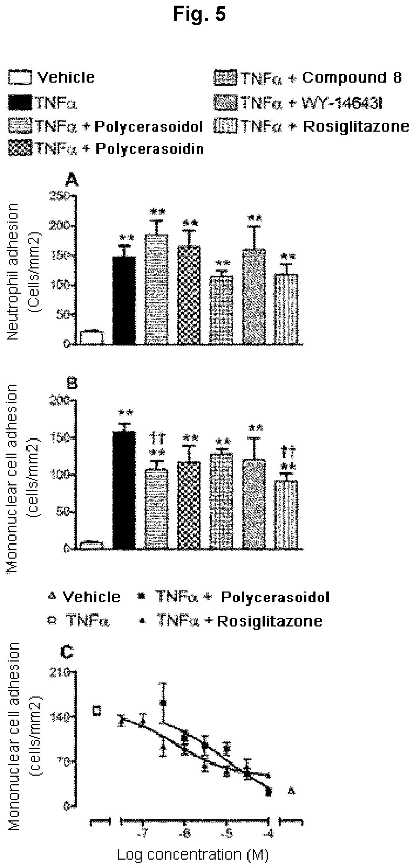

**Figure 5****.** The compound polycerasoidol (1) for use according to the present invention inhibits mononuclear cell adhesion to HUVEC induced by TNFα in physiological flow conditions. The cells were pretreated for 20 h with 1 µM of compounds polycerasoidol (1) and polycerasoidin (2) for use according to the present invention, compound 8 of the present invention, WY-14643 or rosiglitazone, prior to stimulation with TNFα (20 ng/mL, 24 h). Freshly isolated human neutrophils of mononuclear cells were perfused across the endothelial monolayers for 5 min at 0.5 dyn/cm² quantifying the adhesion of neutrophils (**Fig. 5A**) and mononuclear cells (**Fig. 5B**). Dose-response curves were obtained for the compound polycerasoidol (1) for use according to the present invention, and rosiglitazone, with HUVEC stimulated with TNFα (20 ng/mL, 24 h) and perfused with freshly isolated human mononuclear cells (**Fig. 5C**). The results are the mean ± SEM of 4-6 independent experiments. **p <0.01 with respect to the vehicle group and ^{††}p < 0.01 with respect to cells stimulated with TNFα.

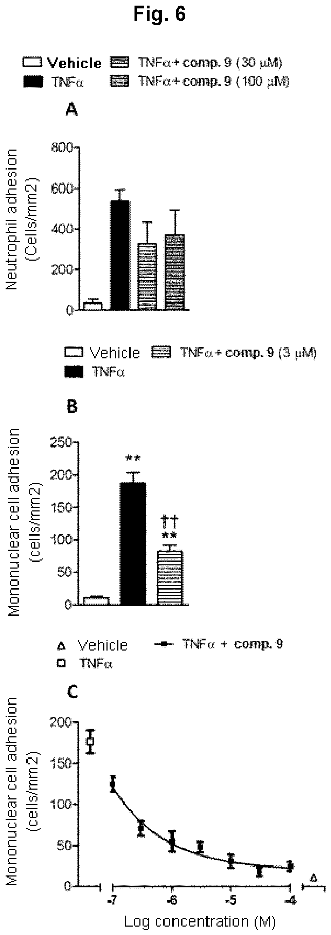

**Figure 6****.** Compound 9 of the present invention inhibits mononuclear cell adhesion to HUVEC induced by TNFα in physiological flow conditions. Some cells were pretreated for 20 h with compound **9** (3, 30 or 100 µM) of the present invention, prior to stimulation with TNFα (20 ng/mL, 24 h). Freshly isolated human neutrophils or mononuclear cells were perfused across the endothelial monolayers for 5 min at 0.5 dyn/cm² quantifying the adhesion of neutrophils (**Fig. 6A**) and mononuclear cells **(****Fig. 6B**). The concentration-response curves (0.1-100 µM) were made for compound 9 of the present invention with HUVEC stimulated with TNFα (20 ng/mL, 24 h) and perfused with freshly isolated human mononuclear cells (**Fig. 6C**). The results are the mean ± SEM for 4-6 independent experiments. **p< 0.01 with respect to the vehicle group ⁺⁺p< 0.01 with respect to cells stimulated with TNFα.

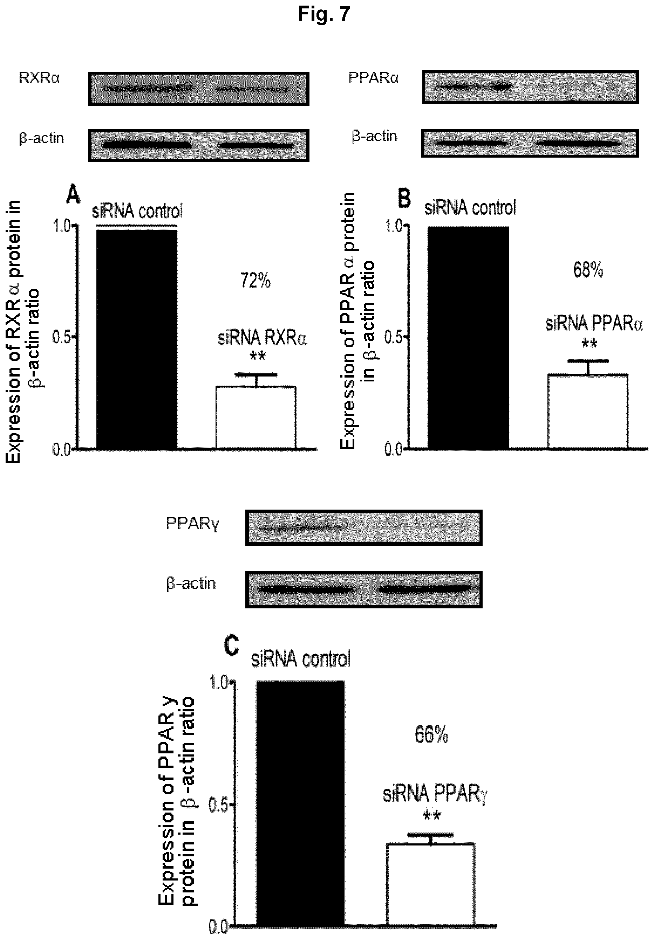

**Figure 7****.** Endothelial cells were transfected with control siRNA or specific siRNA for RXRα, PPARα or PPAR. 48 h after transfection, the protein expression of RXRα (**Fig. 7A**), PPARα (**Fig. 7B**) or PPARγ (**Fig. 7C**) was determined by Western blot. The results (mean ± SEM for n=4 independent experiments) are expressed with respect to β-actin. The images of the representative gels are also shown. **p <0.01 with respect to the values in the corresponding siRNA control group.

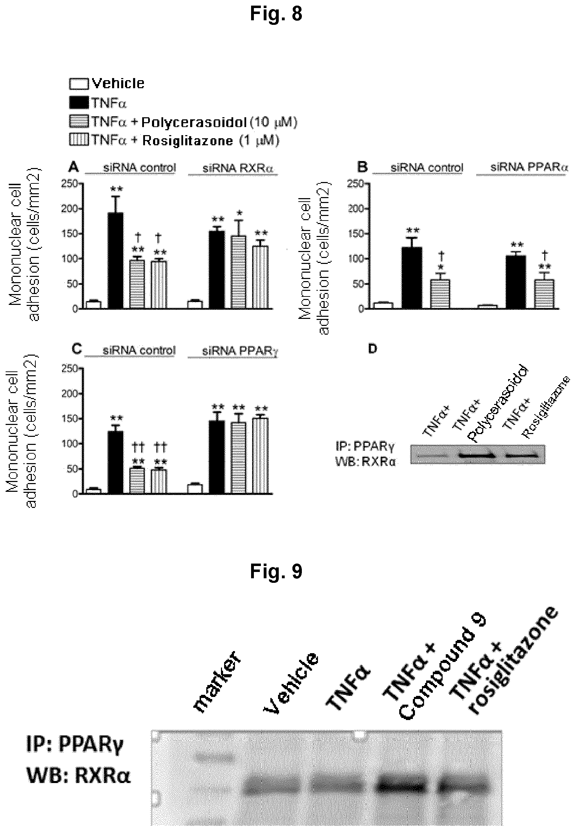

**Figure 8****.** The silencing of PPARγ or RXRα by siRNA stops the inhibiting effect of the compound polycerasoidol (1) for use according to the present invention, in the interactions between endothelial cells and mononuclear leukocytes induced by TNFα. HUVEC were transfected with control or specific siRNA for RXRα, PPARα or PPARγ. 48 h after transfection the cells were pretreated with polycerasoidol (10 µM) or rosiglitazone (1 µM) for 20 h and then stimulated with TNFα (20 ng/mL, 4h). The mononuclear cell adhesion was quantified. The results are the mean ± SEM of 3-4 independent experiments. *p <0.05 or **p <0.01 with respect to the corresponding vehicle group, ^{†}p <0.05 or ^{††}p <0.01 with respect to corresponding cells stimulated with TNFα (**Fig. 8A, 8B, 8C**). The RXRα/PPARγ interaction was measured by immunoprecipitation of PPARγ followed by Western blot for RXRα. Chemoluminescence films were quantified by image analysis. The blot represents n=3 independent experiments (**Fig**. **8D**).
**Figure 9****.** The RXRα/PPARγ interaction of compound 9 of the present invention was measured by immunoprecipitation of PPARγ followed by Western blot for RXRα. Chemoluminescence films were quantified by image analysis. The blot represents n=3 independent experiments.

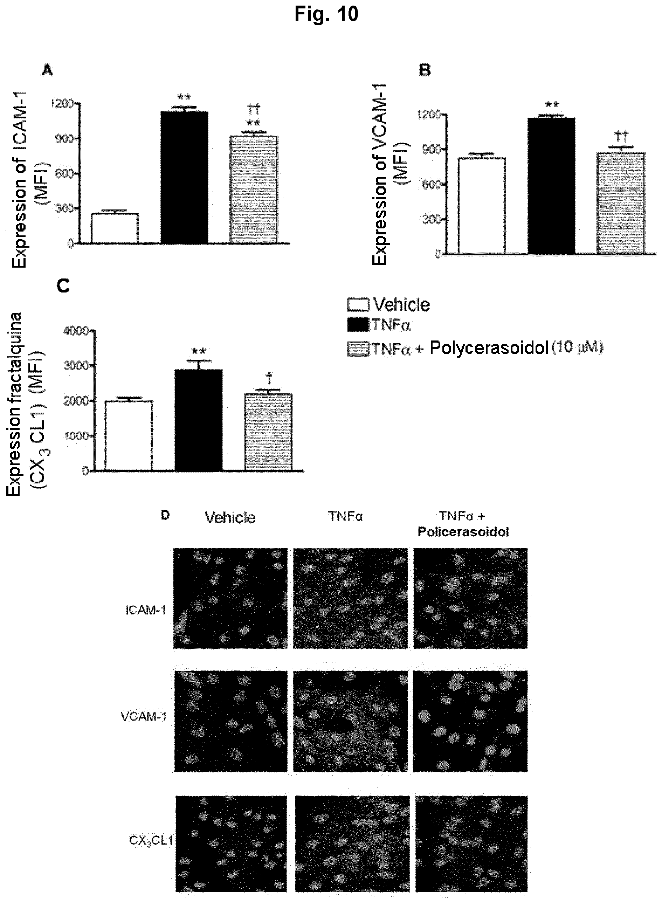

**Figure 10****.** The compound polycerasoidol (1) for use according to the present invention, inhibits the expression in HUVEC of ICAM-1, VCAM-1 and CX₃CL1 induced by TNFα. Some cells were pretreated with the compound polycerasoidol (10 µM) for use according to the present invention, 20 h, before stimulation with TNFα (20 ng/mL, 24 h for ICAM-1, VCAM-1 and CX₃CL1). Flow cytometry was used to determine the expression of ICAM-1 (**Fig. 10A**), VCAM-1 (**Fig. 10B**) and CX₃CL1 (**Fig. 10C**). The results (mean ± SEM) were expressed as the mean fluorescence intensity of n=3-6 independent experiments. Expression of ICAM-1, VCAM-1, and CX₃CL1 was also seen in HUVEC not permeabilized by inmunofluorescence, using alexa-fluor 488 (green) (Fig. 10E). The nuclei were counterstained with Hoechst dye (blue). *p <0.05 or **p <0.01 with respect to the vehicle group, ^{†}p <0.05 or ^{††}p <0.01 with respect to cells stimulated with TNFα.

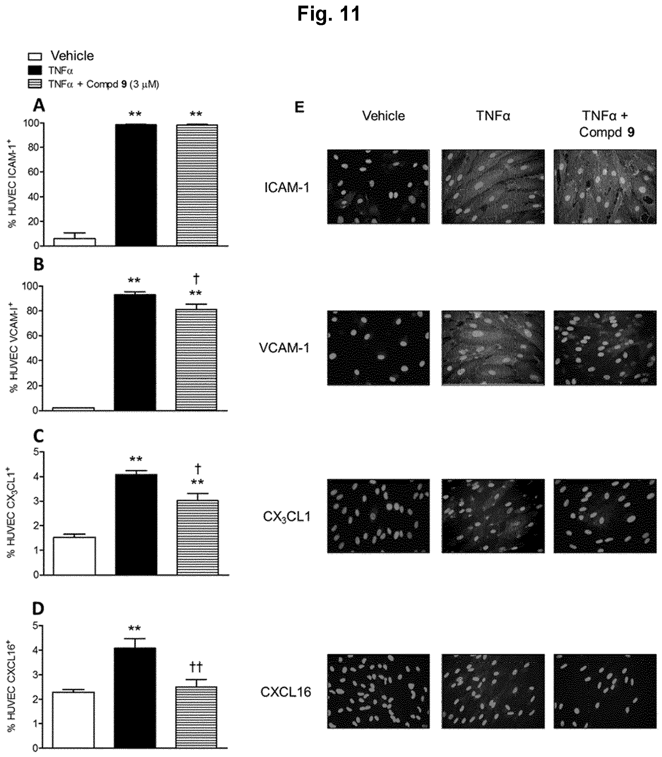

**Figure 11****.** Compound **9** of the present invention inhibits the expression in HUVEC of VCAM-1, CX₃CL1 and CXCL16 induced by TNFα, but not ICAM-1. Some cells were pretreated with compound **9** (3 µM) of the present invention 20 h, before stimulation with TNFα (20 ng/mL, 24 h). Flow cytometry was used to determine the expression of ICAM-1 (**Fig. 11A**), VCAM-1(**Fig. 11B**), CX₃CL1 (**Fig. 11C**) and CXCL16 (**Fig. 11D**). The results (mean ± SEM) were expressed as the mean fluorescence intensity for n=3-6 independent experiments. Expression of ICAM-1, VCAM-1, CX₃CL1 and CXCL16 was also seen in HUVEC not permeabilized by immunofluorescence, using alexa-fluor 488 (green) (**Fig. 11E**). The nuclei were counterstained with Hoechst dye (blue). **p <0.01 with respect to the vehicle group, ^{†}p <0.05 or ^{††}p <0.01 with respect to cells stimulated with TNFα.

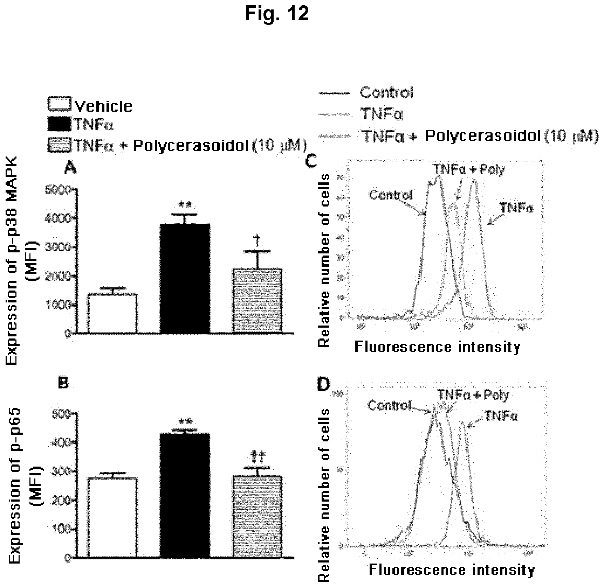

**Figure 12****.** The compound polycerasoidol (1) for use according to the present invention inhibits the activation of p38 MAPK and NF-κB (p65) induced by TNFα in HUVEC. The cells were stimulated for 1 h with TNFα (20 ng/mL). Some cells were pretreated with the compound polycerasoidol (10 µM) for use according to the present invention, 20 h, before stimulation with TNFα. The activation of p38 MAPK (**Fig. 12A**) and NF-κB (**Fig. 12B**) was determined by flow cytometry. The results (mean ± SEM) were expressed as the mean fluorescence intensity of n=3-6 independent experiments (**Fig. 12C and 12D**). The representative histograms are shown. **p <0.01 with respect to the vehicle group ^{†}p <0.05 and ^{††}p <0.01 with respect to cells stimulated with TNFα.

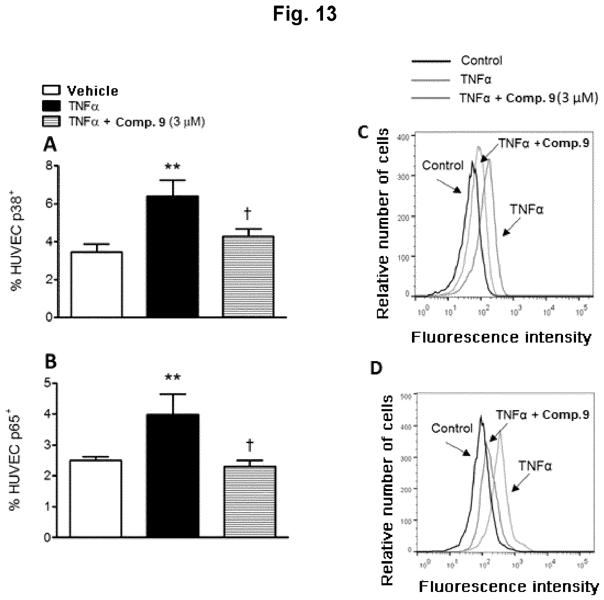

**Figure 13****.** Compound **9** of the present invention inhibits the activation of p38 MAPK and NF-κB (p65) induced by TNFα in HUVEC. The cells were stimulated for 1 h with TNFα (20 ng/mL). Some cells were pretreated with compound **9** (3 µM) of the present invention 20 h before stimulation with TNFα. The activation of p38 MAPK (**Fig. 13A**) and NF-κB (**Fig. 13B**) was determined by flow cytometry. The results (mean ± SEM) were expressed as the mean fluorescence intensity of n=3-6 independent experiments (Fig. 13C and 13D) The representative histograms are shown. **p <0.01 with respect to the vehicle group and ^{†}p <0.05 with respect to cells stimulated with TNFα.

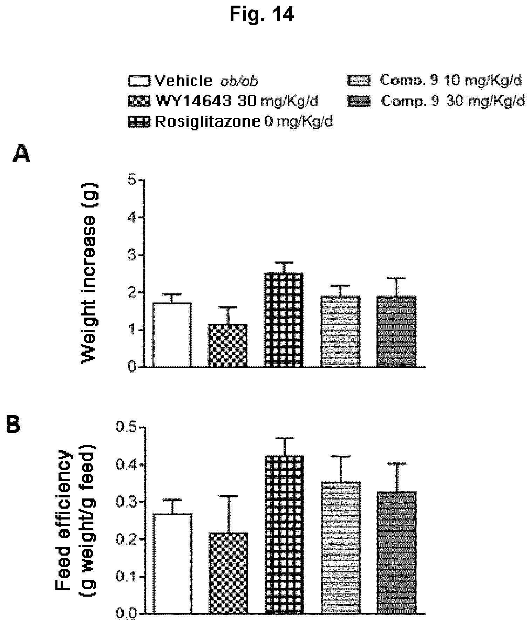

**Figure 14****.** Compound **9** of the present invention does not cause a significant weight gain (g) and feed efficiency in *ob*/*ob* mice after 4 days of treatment. The histograms show the weight gain (**Fig. 14A**) expressed as difference in weight between day 5 and day 0 (d₅-d₀), and feed efficiency (**Fig. 14B**) calculated as the ratio of weight gain and food consumed. No significant differences were observed either in weight gain or feed efficiency between mice treated with compound **9** of the present invention and the control groups. The columns represent the mean ± SEM of n=5-6 independent experiments.

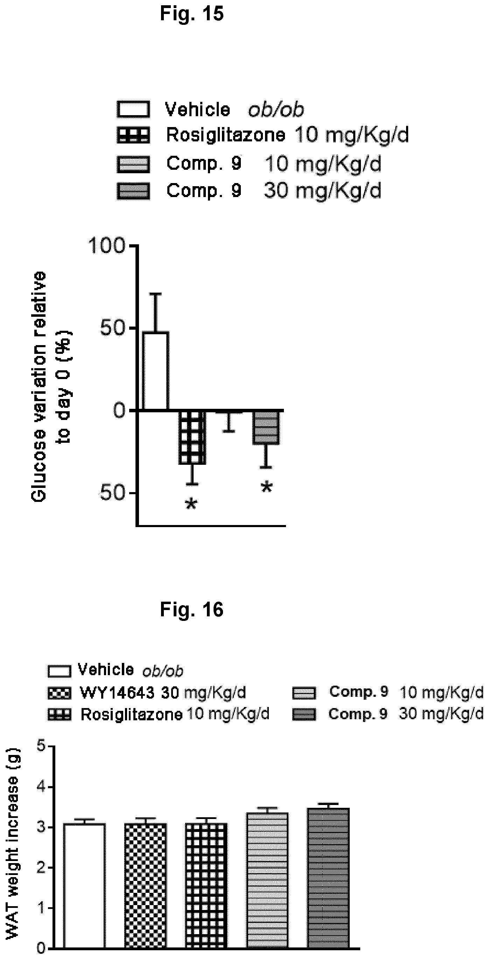

**Figure 15****.** Compound **9** of the present invention and rosiglitazone reduce plasma glucose values in *ob*/*ob* mice after 4 days of treatment. The glucose variation with respect to d₀ (day 0, before treatment) is shown, calculated according to the formula: ((ds-do)/do) × 100. The columns represent the mean ± SEM of n=5-6 independent experiments. *p <0.05 with respect to the group of ob/ob mice administered with the vehicle.
**Figure 16****.** Compound **9** of the present invention does not cause significant weight gain of white adipose tissue (WAT) in *ob*/*ob* mice after 4 days of treatment. The columns show the mean ± SEM for n=5-6 independent experiments

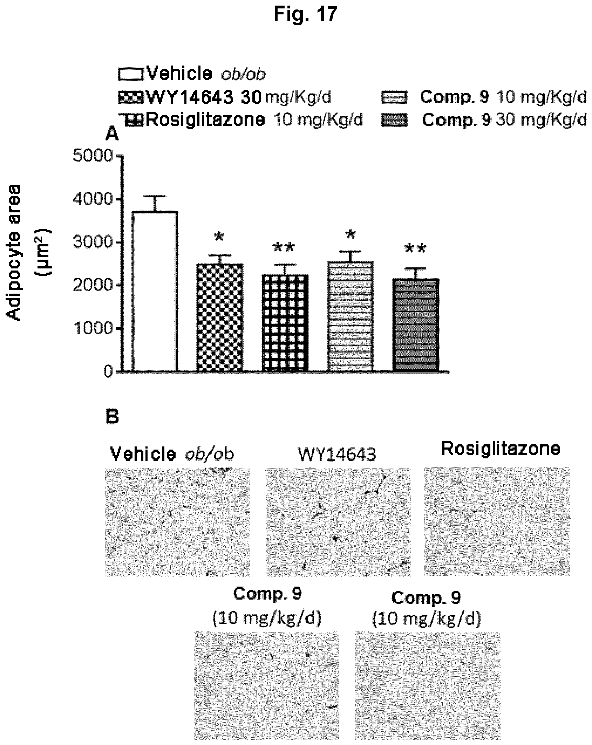

**Figure 17****.** Compound **9** of the present invention significantly reduces the mean surface area of WAT adipocytes in a dose-dependent manner in *ob*/*ob* mice after 4 days of treatment. Mean surface area of adipocytes in µm² (**Fig. 17A**), and the corresponding representative images (**Fig. 17B**). The columns represent the mean ± SEM of n=5-6 independent experiments. *p <0.05 or **p <0.01 with respect to the group of ob/ob mice administered with the vehicle.

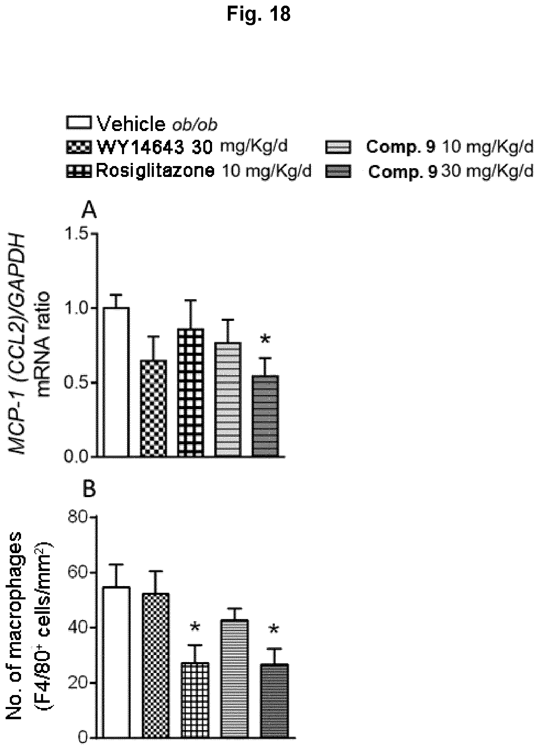

**Figure 18****.** Study of the anti-inflammatory effect on white adipose tissue (WAT) in *ob*/*ob* mice after 4 days of treatment. MCP-1 mRNA expression level in WAT (**Fig. 18A**) and F4/80⁺ macrophage quantification in WAT by immunohistochemistry (**Fig. 18B**). Compound **9** of the present invention reduces the expression of MCP-1 mRNA and the number of macrophages infiltrated in white adipose **tissue (WAT). The columns correspond to the mean ± SEM o**f **n= 5-6 independent** experiments. *p <0.05 with respect to the group of ob/ob mice administered with the vehicle.

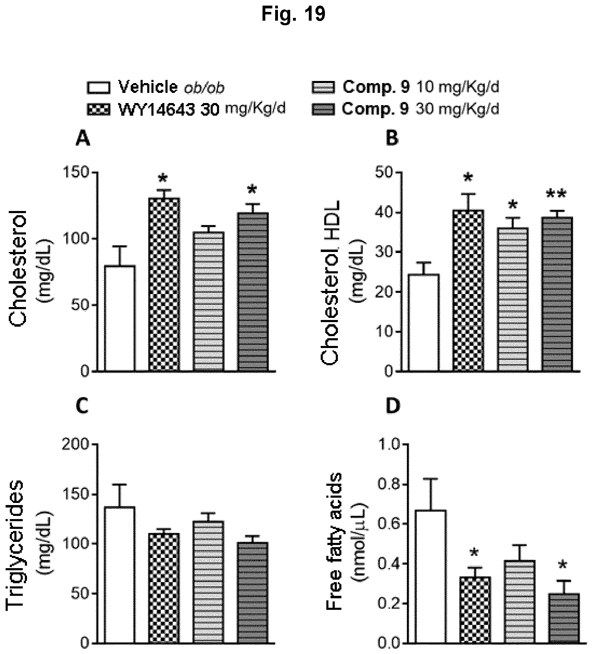

**Figure 19****.** Compound **9** of the present invention and WY-14643 increase total cholesterol and HDL-c levels, and reduce triglyceride and free fatty acid levels in plasma. Total cholesterol levels (**Fig. 19A**), HDL-c (**Fig. 19B**), triglycerides (**Fig. 19C**) and free fatty acids (**Fig.19D**). The columns represent the mean ± SEM of n=5-6 independent experiments. *p <0.05 or **p <0.01 with respect to the group of *ob*/*ob* mice administered with the vehicle.

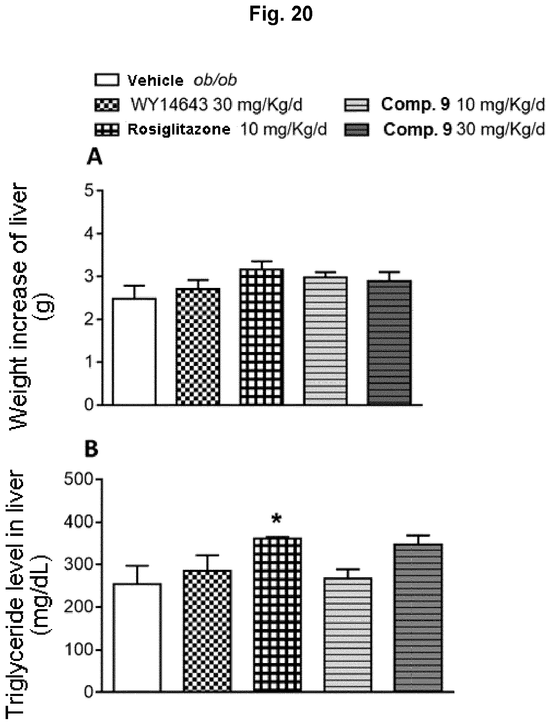

**Figure 20****.** Compound **9** of the present invention did not significantly affect the weight of the liver nor the accumulated triglycerides, compared to the control group (vehicle). Liver weight gain (**Fig. 20A**) and liver triglyceride levels (**Fig. 20B**). The columns represent the mean ± SEM of n=5-6 independent experiments. *p <0.05 with respect to the group of *ob*/*ob* mice administered with the vehicle.

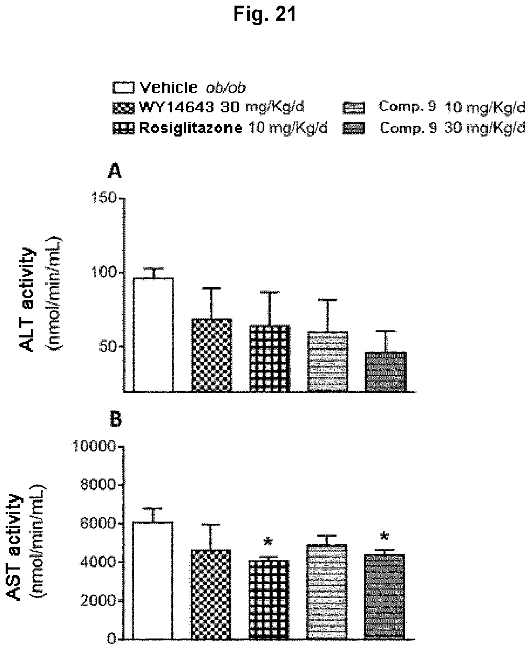

**Figure 21****.** Compound **9** of the present invention reduced the circulating (plasma) levels of alanine amino transferase (ALT) and aspartic transaminase (AST) in *ob*/*ob* mice after 4 days of treatment. The columns represent the mean ± SEM of n=5-6 independent experiments. *p <0.05 with respect to the group of ob/ob mice administered with the vehicle.

### EXAMPLES

The examples described below are for purposes of illustration only. All the research with human samples of this study has been performed in compliance with the principles of the Declaration of Helsinki and with the approval of the institutional ethics committee of the Hospital Clinico Universitario de Valencia (Valencia, Spain).

### Example 1: Production of compounds of formula I

All reagents and solvents were acquired from commercial sources and used directly. All reactions sensitive to humidity were performed in flasks dried in an oven in a nitrogen atmosphere and with dry solvents. All reactions were controlled by analytical TLC with silica gel 60 F254 (Merck 5554) and UV detection at 254 nm. Residues were purified through a silica gel column 60H (5-40 µm, Merck 7736) and by ultrafast chromatography (230-400 µm, Merck 9385). The preparatory TLC was performed using 0.5 mm silica gel plates (Merck). Hewlett-Packard (HP-1100) or TripleTOF5600 (ABSciex) was used for the LC-MS analysis, with the API (atmospheric pressure ionization) source configured as APCI (atmospheric pressure chemical ionization) or APIES (ionization by electrospray) in positive or negative mode. The NMR spectra (¹H, ¹³C, COSY 45, DEPT, HSQC and HMBC) were referenced for the signal of CDCl₃ solvent in a Bruker AC 300 or AC-500. The resonance multiplicities for ¹³C NMR were assigned by DEPT experiments. The NMR assignments were supported by COSY 45, DEPT, HSQC and HMBC experiments.

### 1.1. Production of the compounds 1 (polycerasoidol) and 2 (polycerasoidin) for use according to the present invention

The compounds polycerasoidol (**1**) and polycerasoidin (**2**) for use according to the present invention were isolated from a methanolic extract of bark of stems of *Polyalthia cerasoides* and *P. sclerophylla* and identified according to their chromatographic and spectral characteristics (Gonzalez *et al.,* 1995 and 1996).

### 1.2. Production of the compounds 3, 4, 4a, 4b, 4c, 6, 7 and 8 of the invention and compound 5 for use according to the present invention

**Compound 3 of the invention:** 1-bromopropane CH₃CH₂CH₂Br (20 µL 0.2 mmol) was added to a suspension of polycerasoidol (**1**) (80 mg, 0.2 mmol) and K₂CO₃ (80 mg) in acetone (2 mL), and the mixture was shaken at reflux for 4 h. The reaction mixture was concentrated to dryness and again dissolved in 10 mL of CH₂Cl₂. The organic phase was washed with brine (3 × 10 mL) and H₂O (3 × 10 mL), dehydrated with anhydrous Na₂SO₄ and evaporated to dryness in a rotary evaporator. The reaction crude was purified by silica gel column chromatography (hexane-AcOEt, 90:10) to obtain compound 3 (50 mg, 63 %) of the invention. NMR ¹H (400 MHz, CDCl₃) δ 6.49 (d, *J*= 2.6 Hz, H-7), 6.39 (d, *J*= 2.6 Hz, H-5), 5.91 (td, *J*= 1.3, 7.2 Hz, H-7', 5.15 (td, *J*= 0.9, 7.1 Hz, H-3'), 4.11 (t, *J*= 6.6 Hz, 2H, COOC*H*₂CH₂CH₃-9'), 2.68 (t, *J*= 6.6 Hz, 2H, CH₂-4), 2.57 (ddd, *J*= 1.2, 7.5, 15.1 Hz, 2H, CH₂-6'), 2.12 (s, 3H, CH₃-13'), 2.10-2.03 (m, 4H, CH₂-2' and CH₂-5'), 1.89 (d, 3H, *J*= 1.6 Hz, CH₃-10'), 1.75 (sex, *J*= 7.3 Hz, 2H, COOCH₂C*H*₂CH₃-9'), 1.86-1.60 (m, 4H, CH₂-3, CH₂-1'), 1.59 (s, 3H, CH₃-11'), 1.25 (s, 3H, CH₃-12'), 0.97 (t, *J*= 7.3 Hz, 3H, COOCH₂CH₂C*H*₃-9'); NMR ¹³C (125 MHz, CDCl₃) δ 168.4 (COOCH₂CH₂CH₃-9'), 147.9 (C-6), 145.8 (C-8a), 142.8 (CH-7'), 134.3 (C-4'), 127.2 (C-8'), 127.0 (C-8), 124.9 (CH-3'), 121.1 (C-4a), 115.6 (CH-7), 112.6 (CH-5), 75.2 (C-2), 65.8 (COOC*H*₂CH₂CH₃-9'), 39.6 (CH₂-1'), 39.0 (CH₂-5'), 31.3 (CH₂-3), 27.9 (CH₂-6'), 23.9 (CH₃-12'), 22.4 (CH₂-4), 22.1 (COOCH₂C*H*₂CH₃-9'), 22.0 (CH₂-2'), 20.6 (CH₃-10'), 16.0 (CH₃-13'), 15.7 (CHa-11'), 10.5 (COOCH₂CH₂C*H*₃-9'); LC-MS (APCI positive mode) *m*/*z* 401.1 (100) [MH]⁺; HREIMS *m*/*z* 400.263746 [M]⁺ (400.261360 calc for C₂₅H₃₆O₄).

**Compound 4 of the invention:** Benzyl chloride ClCH₂Ph (25 µL 0.2 mmol) was added to a suspension of polycerasoidol (**1**) (80 mg, 0.2 mmol) and K₂CO₃ (80 mg) in acetone (2 mL), and the mixture was shaken at reflux for 4 h. After the usual extraction, the reaction crude was purified by silica gel column chromatography (hexane-AcOEt, 90:10) to obtain compound **4** (60 mg, 67 %) of the invention. ¹H NMR (400 MHz, CDCl₃) δ 7.39-7.29 (m, 5H, COOCH₂*Ph-*9'), 6.50 (dd, *J*= 0.4, 2.7 Hz, H-7), 6.40 (dd, *J*= 0.4, 2.7 Hz, H-5), 5.95 (td, *J*= 1.3, 7.2 Hz, H-7'), 5.20 (s, 2H, COOC*H*₂Ph-9'), 5.12 (td, *J*= 1.1, 7.1 Hz, H-3'), 2.70 (td, *J*= 2.5, 6.7 Hz, 2H, CH₂-4), 2.57 (ddd, *J*= 1.3, 7.4, 15.2 Hz, 2H, CH₂-6'), 2.14 (s, 3H, CH₃-13'), 2.12-1.90 (m, 4H, CH₂-2' and CH₂-5'), 1.92 (d, 3H, *J*= 1.6 Hz, CH₃-10'), 1.82-1.60 (m, 4H, CH₂-3 and CH₂-1'), 1.56 (s, 3H, CH₃-11'), 1.26 (s, 3H, CH₃-12'); NMR ¹³C (125 MHz, CDCl₃) δ 167.9 (COOCH₂Ph-9'), 147.8 (C-6), 145.8 (C-8a), 143.6 (CH-7'), 134.1 (C-1" de COOCH₂*Ph*-9'), 134.3 (C-4'), 128.5 (2CH de COOCH₂*Ph*-9'), 128.0 (3CH de COOCH₂*Ph*-9'), 127.3 (C-8'), 126.7 (C-8), 124.8 (CH-3'), 121.2 (C-4a), 115.6 (CH-7), 112.6 (CH-5), 76.0 (C-2), 66.0 (COOC*H*₂Ph-9'), 39.6 (CH₂-1'), 39.0 (CH₂-5'), 31.3 (CH₂-3), 28.0 (CH₂-6'), 24.0 (CH₃-12'), 22.4 (CH₂-4), 22.1 (CH₂-2'), 20.6 (CH₃-10'), 16.0 (CH₃-13'), 15.7 (CH₃-11'); LC-MS (APCI positive mode) *m*/*z* 472.2 (100) [MH + Na]⁺; ElMS *m*/*z* 357 [M-CH₂Ph]⁺.

**Compound 4a of the invention:** 2-chloroacetamide ClCH₂CONH₂ (0.1 mmol) was added to a suspension of compound **4** (45 mg, 0.1 mmol) of the invention and K₂CO₃ (45 mg) in acetonitrile (5 mL), and the mixture was shaken at reflux for 9 h. After the usual extraction, the reaction crude was purified by silica gel column chromatography (hexane-AcOEt, 90:10) to obtain compound **4a** (25 mg, 50 %) of the invention. NMR ¹H (400 MHz, CDCl₃) δ 7.38-7.29 (m, 5H, COOCH₂Ph-9'), 6.60 (d, *J*= 3.0 Hz, H-7), 6.46 (dd, *J*= 3.0 Hz, H-5), 5.93 (td, *J*= 1.3, 7.3 Hz, H-7'), 5.18 (s, 2H, COO*CH*₂Ph-9'), 5.11 (td, *J*= 1.2, 7.3 Hz, H-3'), 4.41 (s, 2H, O*CH*₂CONH₂-6), 2.72 (td, *J*= 2.5, 6.5 Hz, 2H, CH₂-4), 2.56 (ddd, *J*= 1.2, 7.3, 14.8 Hz, 2H, CH₂-6'), 2.16 (s, 3H, CH₃-13'), 2.10-2.04 (m, 4H, CH₂-2' and CH₂-5'), 1.92 (d, 3H, *J*= 1.2 Hz, CH₃-10'), 1.81-1.58 (m, 4H, CH₂-3, CH₂-1'), 1.55 (s, 3H, CH₃-11'), 1.26 (s, 3H, CH₃-12'); NMR ¹³C (100 MHz, CDCl₃) δ 171.8 (OCH₂CONH₂-6), 167.7 (COOCH₂Ph-9'), 149.8 (C-6), 147.0 (C-8a), 143.5 (CH-7'), 136.2 (C-1" de COOCH₂*Ph*-9'), 134.4 (C-4'), 128.4 (2CH de COOCH₂*Ph*-9'), 128.0 (3CH de COOCH₂*Ph*-9'), 126.7 (C-8'), 126.7 (C-8), 124.7 (CH-3'), 121.2 (C-4a), 115.6 (CH-7), 112.0 (CH-5), 75.5 (C-2), 67.9 (O*CH*₂CONH₂-6), 65.9 (COO*CH*₂Ph-9'), 39.6 (CH₂-1'), 39.0 (CH₂-5'), 31.2 (CH₂-3), 28.0 (CH₂-6'), 24.0 (CH₃-12'), 22.6 (CH₂-4), 22.1 (CH₂-2'), 20.6 (CH₃-10'), 16.2 (CH₃-13'), 15.7 (CH₃-11'); HREIMS *m*/*z* 505.290785 [M]⁺ (505.282824 calc for C₃₁H₃₉NO₅).

**Compound 4b of the invention.** Benzyl chloride ClCH₂Ph (10 µL 0.1 mmol) was added to a suspension of compound **4** (35 mg, 0.1 mmol) and K₂CO₃ (35 mg) in acetone (5 mL), and the mixture was shaken at reflux for 15 h. After the usual extraction, the reaction crude was purified by silica gel column chromatography (hexane-AcOEt, 90:10) to obtain compound **4b** (20 mg, 40 %) of the invention. NMR ¹H (400 MHz, CDCl₃) δ 7.44-7.29 (m, 10H, OCH₂*Ph*-6, COOCH₂*Ph*-9'), 6.66 (d, *J*= 3.0 Hz, H-7), 6.54 (dd, *J*= 3.0 Hz, H-5), 5.94 (td, *J*= 1.2, 7.2 Hz, H-7'), 5.19 (s, 2H, COO*CH*₂Ph-9'), 5.12 (td, *J*= 1.2, 7.2 Hz, H-3'), 4.98 (s, 2H, O*CH*₂Ph-6), 2.73 (td, *J*= 2.8, 6.7 Hz, 2H, CH₂-4), 2.56 (ddd, *J*= 1.2, 7.2, 15.2 Hz, 2H, CH₂-6'), 2.16 (s, 3H, CH₃-13'), 2.11-2.04 (m, 4H, CH₂-2' and CH₂-5'), 1.92 (d, 3H, *J*= 1.2 Hz, CH₃-10'), 1.81-1.60 (m, 4H, CH₂-3, CH₂-1'), 1.56 (s, 3H, CH₃-11'), 1.27 (s, 3H, CH₃-12'); NMR ¹³C (100 MHz, CDCl₃) δ 167.0 (COOCH₂Ph-9'), 151.4 (C-6), 146.2 (C-8a), 143.5 (CH-7'), 137.6 and 136.2 (2C, C-1" de OCH₂*Ph*-6, C-1"' de COOCH₂*Ph*-9'), 134.3 (C-4'), 128.5-127.5 (10CH, OCH₂Ph-6, COOCH₂*Ph*-9'), 127.2 (C-8'), 126.7 (C-8), 124.9 (CH-3'), 120.9 (C-4a), 115.7 (CH-7), 112.2 (CH-5), 75.3 (C-2), 70.5 (O*CH*₂Ph-6), 65.9 (COO*CH*₂Ph-9'), 39.7 (CH₂-1'), 39.0 (CH₂-5'), 31.4 (CH₂-3), 28.0 (CH₂-6'), 24.0 (CH₃-12'), 22.6 (CH₂-4), 22.1 (CH₂-2'), 20.6 (CH₃-10'), 16.2 (CH₃-13'), 15.7 (CH₃-11'); ElMS *m*/*z* 538 [M]⁺ (70), 447 [M-CH₂Ph]⁺, 356 (10) [M-2 × CH₂Ph]⁺, 91 (100).

**Compound 4c of the invention.** 1-bromopropane CH₃CH₂CH₂Br (20 µL, 0.2 mmol) was added to a suspension of compound **4** (70 mg, 0.2 mmol) of the invention and K₂CO₃ (70 mg) in acetone (5 mL), and the mixture was shaken at reflux for 10 h. After the usual extraction, the reaction crude with the protected phenol group was dissolved in 10 mL of an EtOH-KOH 20% (v/v) mixture and kept at reflux for 10 h. Subsequently, the reaction mixture was acidified with HCl 1N, concentrated to dryness and redissolved in 10 mL of CH₂Cl₂. After the usual extraction, the reaction crude was purified by silica gel column chromatography (hexane-AcOEt, 90:10) to obtain **4c** (34 mg, 43 %) of the invention. NMR ¹H (300 MHz, CDCl₃) δ 6.58 (d, *J*= 2.9 Hz, H-7), 6.45 (d, *J*= 2.9 Hz, H-5), 6.06 (td, *J*= 1.3, 7.3 Hz, H-7'), 5.16 (td, *J*= 0.9, 6.6 Hz, H-3'), 3.83 (t, *J*= 6.6 Hz, 2H, O*CH*₂CH₂CH₃-6), 2.72 (t, *J*= 6.8 Hz, 2H, CH₂-4), 2.62 (dd, *J*= 7.5, 14.4 Hz, 2H, CH₂-6'), 2.15 (s, 3H, CH₃-13'), 2.11-2.05 (m, 4H, CH₂-2' and CH₂-5'), 1.90 (d, 3H, J= 1.5 Hz, CH₃-10'), 1.79-1.72 (m, 6H, OCH₂*CH*₂CH₃-6, CH₂-3 and CH₂-1'), 1.60 (s, 3H, CH₃-11'), 1.26 (s, 3H, CH₃-12'), 1.01 (t, *J*= 6.6 Hz, 3H, OCH₂CH₂*CH*₃-6); NMR ¹³C (75 MHz, CDCl₃) δ 173.0 (*C*OOH-9'), 151.6 (C-6), 146.3 (C-8a), 145.9 (CH-7'), 134.2 (C-4'), 127.1 (C-8'), 126.1 (C-8), 125.1 (CH-3'), 120.9 (C-4a), 115.4 (CH-7), 111.8 (CH-5), 75.2 (C-2), 70.0 (O*CH*₂CH₂CH₃-6), 39.7 (CH₂-1'), 39.0 (CH₂-5'), 31.4 (CH₂-3), 28.1 (CH₂-6'), 24.0 (CH₃-12'), 22.7 (CH₂-4), 22.6 (OCH₂*CH*₂CH₃-6), 22.1 (CH₂-2'), 20.5 (CH₃-10'), 16.2 (CH₃-13'), 15.7 (CH₃-11'), 22.6 (OCH₂CH₂*CH*₃-6); LC-MS (APCI positive mode) *m*/*z* 424.2 (100) [MH + Na]⁺.

**Compound 5 for use according to the present invention.** Concentrated HCl (1.5 mL) was added dropwise at 0°C to a solution of compound polycerasoidol (**1**) (10 mg, 0.03 mmol), for use according to the present invention, in methanol (5 mL). The mixture was shaken at reflux for 7 h. After the usual extraction, the reaction crude was purified by preparative silica gel TLC (hexane-AcOEt, 90:10) to obtain compound **5** (6 mg, 54 %) for use according to the present invention. NMR ¹H (300 MHz, CDCl₃) δ 6.48 (d, *J*= 2.9 Hz, H-7), 6.38 (d, *J*= 2.9 Hz, H-5), 5.94 (td, *J*= 1.2, 7.4 Hz, H-7'), 5.16 (td, *J*= 1.2, 7.4 Hz, H-3'), 3.73 (s, 3H, COO*CH*₃-9'), 2.80-2.57 (m, 4H, CH₂-4, CH₂-6'), 2.35-2.00 (m, 4H, CH₂-2' and CH₂-5'), 2.12 (s, 3H, CH₃-13'), 1.90-1.82 (m, 4H, CH₂-3, CH₂-1'), 1.90 (d, 3H, *J*= 1.3 Hz, CH₃-10'), 1.58 (s, 3H, CH₃-11'), 1.25 (s, 3H, CH₃-12'); NMR ¹³C (75 MHz, CDCl₃) δ 169.1 (*CO*OCH₃-9'), 147.3 (C-6), 146.0 (C-8a), 144.8 (CH-7'), 134.2 (C-4'), 127.2 (C-8'), 127.1 (C-8), 124.9 (CH-3'), 121.1 (C-4a), 115.5 (CH-7), 112.5 (CH-5), 75.1 (C-2), 51.1 (COO*CH*₃-9'), 39.5 (CH₂-1'), 39.0 (CH₂-5'), 31.3 (CH₂-3), 27.9 (CH₂-6'), 23.9 (CH₃-12'), 22.3 (CH₂-4), 22.0 (CH₂-2'), 20.5 (CH₃-10'), 16.0 (CH₃-13'), 15.7 (CH₃-11'); HREIMS *m*/*z* 372.236641 [M]⁺ (372.230060 calc for C₂₃H₃₂O₄).

**Compound 6 of the invention.** Benzoyl chloride PhCOCI (25 µL, 0.2 mmol) was added to a suspension of compound polycerasoidol (**1**) (100 mg, 0.3 mmol), for use according to the present invention, and K₂CO₃ (100 mg) in acetone (5 mL), and the mixture was shaken at reflux for 2 h. Then methyl bromoacetate BrCH₂COOCH₃ (20 µL, 0.2 mmol) was added and the mixture was shaken at reflux for 15 h. After the usual extraction, the reaction crude was purified by silica gel column chromatography (hexane-AcOEt, 90:10) to obtain compound **6** (55 mg, 35 %) of the invention. NMR ¹H (400 MHz, CDCl₃) δ 8.18 (dd, 2H, *J*= 1.5, 7.7 Hz, H-2" and H-6" de OCO*Ph*-6), 7.62 (td, *J*= 1.5, 7.7 Hz, H-4" de OCO*Ph*-6), 7.50 (td, 2H, *J*= 1.5, 7.7 Hz, H-3" and H-5" de OCO*Ph*-6), 6.80 (dd, *J*= 0.8, 2.8 Hz, H-7), 6.75 (dd, *J*= 0.8, 2.8 Hz, H-5), 6.02 (td, *J*= 1.2, 7.2 Hz, H-7'), 5.17 (td, *J*= 1.2, 7.2 Hz, H-3'), 4.69 (s, 2H, COO*CH*₂COOCH₃-9'), 3.77 (s, 3H, COOCH₂COO*CH*₃-9'), 2.77 (m, 2H, CH₂-4), 2.61 (m, 2H, CH₂-6'), 2.18 (s, 3H, CH₃-13'), 2.15-2.06 (m, 4H, CH₂-2', CH₂-5'), 1.94 (d, 3H, *J*= 1.6 Hz, CH₃-10'), 1.80-1.62 (m, 4H, CH₂-3, CH₂-1'), 1.57 (s, 3H, CH₃-11'), 1.30 (s, 3H, CH₃-12'); NMR ¹³C (100 MHz, CDCl₃) δ 170.0-165.0 (3C, O*CO*Ph-6 and *CO*OCH₂COOCH₃-9'), 149.5 (C-6), 145.0 (CH-7'), 142.7 (C-8a), 134.4 (C-1" de OCO*Ph*-6), 134.2 (C-4'), 130.1-128.5 (5CH, de OCO*Ph*-6), 127.4 (C-8'), 125.9 (C-8), 124.9 (CH-3'), 121. 2 (CH-7), 120.9 (C-4a), 119.2 (CH-5), 75.9 (C-2), 60.4 (COO*CH*₂COOCH₃-9'), 52.2 (COOCH₂COO*CH*₃-9'), 39.8 (CH₂-1'), 39.0 (CH₂-5'), 31.0 (CH₂-3), 28.0 (CH₂-6'), 24.1 (CH₃-12'), 22.4 (CH₂-4), 22.1 (CH₂-2'), 20.5 (CH₃-10'), 16.1 (CH₃-13'), 15.7 (CH₃-11'); HREIMS *m*/*z* 534.267390 [M]⁺ (534.261754 calc for C₃₂H₃₈O₇).

**Compound 7 of the invention**. Dimethyl bromomalonate BrCH(COOCH₃)₂ (5 µL, 0.05 mmol) was added to a suspension of compound polycerasoidin (**2**) (20 mg, 0.05 mmol), for use according to the present invention, and K₂CO₃ (20 mg) in acetone (5 mL), and the mixture was shaken at reflux for 6 h. Then methyl bromoacetate BrCH₂COOCH₃ (20 µL, 0.2 mmol) was added and the mixture was shaken at reflux for 15 h. After the usual extraction, the reaction crude was purified by preparative TLC (hexane-AcOEt, 80:20) to obtain compound **7** (17 mg, 67 %) of the invention. NMR ¹H (300 MHz, CDCl₃) δ 6.56 (d, *J*= 2.8 Hz, H-7), 6.44 (d, *J*= 2.8 Hz, H-5), 6.08 (td, *J*= 1.3, 7.2 Hz, H-7'), 5.62 (s, COO*CH*(COOCH₃)₂-9'), 5.16 (t, *J*= 6.6 Hz, H-3'), 3.83 (s, 6H, COOCH(COO*CH*₃)₂-9'), 3.73 (s, 3H, COO*CH*₃-6), 2.75-2.71 (m, 2H, CH₂-4), 2.66-2.59 (m, 2H, CH₂-6'), 2.18-2.05 (m, 4H, CH₂-2' and CH₂-5'), 2.15 (s, 3H, CH₃-13'), 1.96 (d, 3H, *J*= 1.3 Hz, CH₃-10'), 1.90-1.73 (m, 4H, CH₂-3, CH₂-1'), 1.59 (s, 3H, CH₃-11'), 1.26 (s, 3H, CH₃-12'); NMR ¹³C (75 MHz, CDCl₃) δ 169.0-167.2 (3C, *CO*OCH(COOCH₃)₂-9'), 151.5 (C-6), 145.4 (C-8a), 144.4 (CH-7'), 133.7 (C-4'), 126.6 (C-8'), 126.1 (COO*CH*(COOCH₃)₂-9'), 125.3 (C-8), 124.4 (CH-3'), 121.0 (C-4a), 115.0 (CH-7), 111.0 (CH-5), 75.4 (C-2), 56.1 (O*CH*₃-6), 52.1 (2C, COOCH(COO*CH*₃)₂-9'), 40.0 (CH₂-1'), 39.0 (CH₂-5'), 31.3 (CH₂-3), 28.3 (CH₂-6'), 24.1 (CH₃-12'), 23.3 (CH₂-4), 22.2 (CH₂-2'), 20.3 (CH₃-10'), 16.3 (CH₃-13'), 16.0 (CH₃-11'); HREIMS *m*/*z* 502.263390 [M]⁺ (502.256669 calc for C₂₈H₃₈O₈).

**Compound 8 of the invention.** Methyl bromoacetate BrCH₂COOCH₃ (5 µL, 0.05 mmol) was added to a suspension of compound polycerasoidin (**2**) (20 mg, 0.05 mmol), for use according to the present invention, and K₂CO₃ (20 mg) in acetone (5 mL), and the mixture was shaken at reflux for 6 h. After the usual extraction, the reaction crude was purified by preparative silica gel TLC (hexane-AcOEt, 80:20) to obtain compound **8** (15 mg, 67 %) of the invention. NMR ¹H (300 MHz, CDCl₃) δ 6.56 (d, *J*= 2.7 Hz, H-7), 6.44 (d, *J*= 2.7 Hz, H-5), 6.00 (td, *J*= 1.5, 7.2 Hz, H-7'), 5.15 (td, *J*= 1.5, 7.2 Hz, H-3'), 4.68 (s, 2H, COO*CH*₂COOCH₃-9'), 3.76 (s, 3H, COOCH₂COO*CH₃*-9'), 3.73 (s, 3H, COO*CH*₃-6), 2.73 (t, 2H, *J=* 7.0 Hz, CH₂-4), 2.59 (dd, 2H, *J*= 7.2, 14.8 Hz, CH₂-6'), 2.15 (s, 3H, CH₃-13'), 2.10-2.04 (m, 4H, CH₂-2' and CH₂-5'), 1.93 (d, 3H, *J*= 1.4 Hz, CH₃-10'), 1.79-1.56 (m, 4H, CH₂-3, CH₂-1'), 1.59 (s, 3H, CH₃-11'), 1.26 (s, 3H, CH₃-12'); NMR ¹³C (75 MHz, CDCl₃) δ 168.8 and 167.4 (2C, *CO*OCH₂COOCH₃-9'), 152.5 (C-6), 146.4 (C-8a), 145.4 (CH-7'), 134.7 (C-4'), 127.6 (C-8'), 126.3 (C-8), 125.4 (CH-3'), 121.3 (C-4a), 115.2 (CH-7), 111.4 (CH-5), 75.7 (C-2), 60.8 (COO*CH₂*COOCH₃-9'), 56.0 (O*CH₃*-6), 52.6 (COOCH₂COO*CH₃*-9'), 40.1 (CH₂-1'), 39.4 (CH₂-5'), 31.8 (CH₂-3), 28.4 (CH₂-6'), 24.4 (CH₃-12'), 23.1 (CH₂-4), 22.6 (CH₂-2'), 20.9 (CH₃-10'), 16.6 (CH₃-13'), 16.1 (CH₃-11'); HREIMS *m*/*z* 444.252007 [M]⁺ (444.251189 calc for C₂₆H₃₆O₆).

### 1.3. Synthesis of compound 9 of the invention:

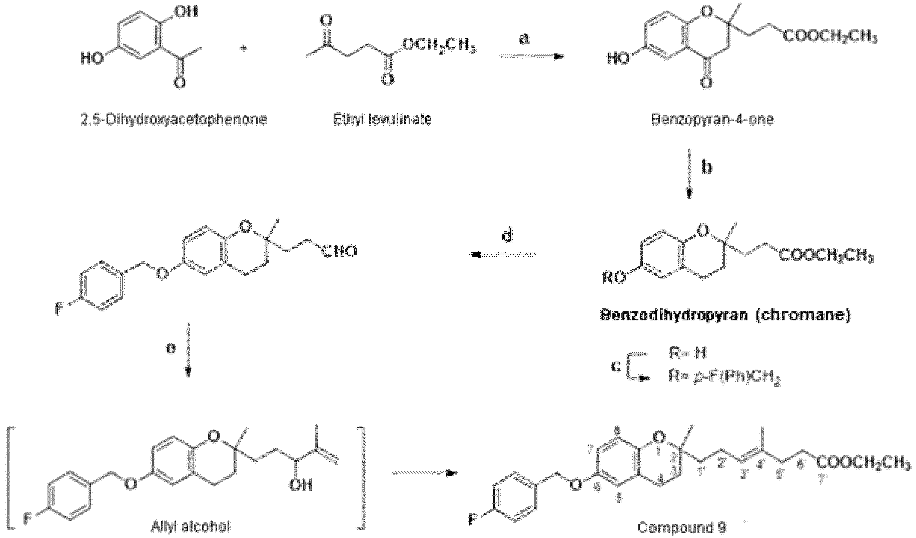

**(a) Synthesis of benzopyran-4-one:** 2,5-dihydroxyacetophenone (0.5 g, 3.28 mmol), ethyl levulinate (0.46 mL, 3.28 mmol) and pyrrolidine (0.87 mL, 9.86 mmol), were dissolved in absolute EtOH (10 mL) with a 3-Å (100 mg) molecular sieve.⁶ The mixture was shaken at 45°C for 24 h under a N₂ atmosphere. Then the reaction mixture was filtered and the filtrate was diluted with CH₂Cl₂ (100 mL) and washed with HCl 1N (3 × 50 mL), H₂O (3 × 50 mL) and brine (3 × 50 mL), dried on anhydrous Na₂SO₄ and evaporated in the rotary evaporator. The residue was purified by silica gel column chromatography (hexane-AcOEt, 70:30) to obtain the benzopyran-4-one (729 mg, 2.62 mmol, 80%) as a yellowish oil. NMR ¹H (400 MHz, CDCl₃) δ 7.34 (d, *J=* 3.0 Hz, H-5), 7.07 (dd, *J*= 8.9, 3.0 Hz, H-7), 6.80 (d, *J*= 8.9 Hz, H-8), 4.14 (q, *J*= 7.1 Hz, 2H, COOC*H₂*CH₃), 2.78 (d, *J*= 16.7 Hz, 1H, CH₂ₐ-3), 2.63 (d, *J*= 16.7 Hz, 1H, CH_{2b}-3), 2.53-2.46 (m, 2H, CH₂-2'), 2.1 (m, 2H, CH₂-1'), 1.38 (s, 3H, CH₃-4'), 1.25 (t, *J=* 7.1 Hz, 3H, COOCH₂C*H₃*); ¹³C NMR (100 MHz, CDCl₃) δ 193.0 (C-4), 173.3 (*CO*OCH₂CH₃-3'), 153.7 (C-8a), 150.2 (C-6), 125.2 (CH-7), 120.1 (C-4a), 119.5 (CH-8), 110.6 (CH-5), 79.8 (C-2), 60.8 (COO*CH₂*CH₃), 47.2 (CH₂-3), 34.1 (CH₂-1'), 28.7 (CH₂-2'), 23.3 (CH₃-4'), 14.1 (COOCH₂*CH₃*); ElMS *m*/*z* 278 [M: C₁₅H₁₈O₅]⁺ (35), 233 (25), 177 (100), 137 (75); HREIMS (%) *m*/*z* 278.11408 [M]⁺ (278.11542 calc for C₁₅H₁₈O₅).
**(b) Reduction of the ketone group:** The benzopyran-4-one (0.4 g, 1.44 mmol) was dissolved in a mixture of AcOH-H₂O (2:1, v/v) (14 mL). Then zinc powder (1.69 g, 25.92 mmol) and HClconc (9 mL) was added slowly for 30 min and the mixture was shaken for 1 h at ambient temperature. H₂O (15 mL) was added to the reaction mixture and filtered, and the filtrate was extracted with AcOEt (3 × 15 mL). The organic phases were combined, dried on anhydrous Na₂SO₄ and evaporated in the rotary evaporator. The residue was purified by silica gel column chromatography (hexane-AcOEt, 85:15) to obtain the benzopyran ester (220.3 mg, 0.834 mmol, 58%) as a colorless oil. NMR ¹H (300 MHz, CDCl₃) δ 6.55 (m, 3H, H-5, H-7, H-8), 4.12 (q, *J=* 7.1 Hz, 2H, COOC*H₂*CH₃), 2.70 (m, 2H, CH₂-4), 2.44 (t, *J*= 7.7 Hz, 2H, H-2'), 2.05-1.70 (m, 4H, CH₂-3, CH₂-1'), 1.23 (s, 3H, CH₃-2), 1.24 (t, *J=* 7.1 Hz, 3H, COOCH₂C*H₃*); ¹³C NMR (75 MHz, CDCl₃) δ 174.1 (*CO*OCH₂CH₃-3'), 148.8 (C-6), 147.4 (C-8a), 121.6 (CH-7), 117.8 (CH-8), 115.4 (C-4a), 114.6 (CH-5), 74.6 (C-2), 60.5 (COO*CH₂*CH₃) 34.3 (CH₂-3), 31.1 (CH₂-1'), 28.8 (CH₂-2'), 34.3 (CH₂-3), 23.7 (CH₃-2), 22.1 (CH₂-4), 14.1 (COOCH₂*CH₃*); ElMS m/z 264 [M: C₁₅H₂₀O₄]⁺ (100), 218 (50), 163 (35), 123 (55). HREIMS (%) *m*/*z* 264.13562 [M]⁺ (264.13616 calc for C₁₅H₂₀O₄) (100).
**(c) *O*-protection of the benzopyran ester:** A suspension of benzopyran ester (0.5 g, 1.89 mmol), *p*-fluorobenzyl chloride *p*F(Ph)CH₂Cl (0.3 mL, 2.46 mmol), anhydrous K₂CO₃ (0.4 g, 2.83 mmol) in absolute EtOH (20 mL) was stirred at 65°C under a N₂ atmosphere for 4 h. The reaction mixture was evaporated in the rotary evaporator, H₂O (3 × 20 mL) was added and it was extracted with CH₂Cl₂ (3 × 15 mL). The organic phases were combined, washed with HCl 1M (2 × 15 mL) and brine (2 × 15 mL), dehydrated with anhydrous Na₂SO₄ and evaporated at a reduced pressure. The residue was purified by silica gel column chromatography (hexane-AcOEt, 90:10) to obtain the O-protected benzopyran ester (556 mg, 1.49 mmol, 79%) as a colorless oil. NMR ¹H (300 MHz, CDCl₃) δ 7.39 (m, 2H, H-2", H-6"), 7.05 (m, 2H, H-3", H-5"), 6.70 (m, 3H, H-5, H-7, H-8), 4.94 (s, 2H, *p*-F-PhC*H₂*O), 4.13 (q, *J=* 7.1 Hz, 2H, CO₂*CH₂*CH₃), 2.76 (t, 2H, *J*= 6.7 Hz, CH₂-4), 2.48 (t, J= 7.7 Hz, 2H, CH₂-2'), 1.91 (m, 4H, CH₂-3, CH₂-1'), 1.24 (t, *J*= 7.2 Hz, 3H, CO₂CH₂*CH₃*-3'); NMR ¹³C (75 MHz, CDCl₃) δ 173.6 (*CO*OCH₂CH₃-3'), 162.3 (d, *J*_{CF}=243.7 Hz, C-4"), 152.0 (C-6), 147.9 (C-8a), 133.1 (d, *J*_{CF}= 3 Hz, C-1"), 129.1 (d, 2C, *J*_{CF}= 8.3 Hz, C-2", C-6"), 121.4 (C-4a), 117.7 (CH-5), 115.2 (d, 2C, *J*_{CF}= 24.8 Hz, CH-3", CH-5"), 115.1 and 114.4 (CH-7 and CH-8), 74.6 (C-2), 69.9 (*p*-F-PhC*H₂*O), 60.3 (CO₂*CH₂*CH₃), 34.3 (CH₂-1'), 31.0 (CH₂-3), 28.7 (CH₂-2'), 23.6 (CH₃-2), 22.2 (CH₂-4), 14.1 (CO₂CH₂*CH₃*); HREIMS *m*/*z* (%) 372.1730 [M]⁺ (372.173688 calc for C₂₂H₂₅O₄F) (83).
**(d) Reduction of *O*-protected benzopyran ester.** A solution of O-protected benzopyran ester (0.2 g, 0.54 mmol) in anhydrous CH₂Cl₂ (6 mL) at -78°C under a N₂ atmosphere was shaken for 15 min. A solution of diisobutyl aluminium hydride (DIBAL-H) 1M in THF (2.47 mL, 2.47 mmol) was added dropwise to this solution. After 20 min the reaction was stopped by adding MeOH (1 mL) and shaken for 15 min, and then a saturated aqueous solution of NH₄Cl (1 mL) was added. The mixture was shaken for 1 h at ambient temperature, filtered and the filtrate extracted with AcOEt (3 × 15 mL). The organic phases were combined, dried on anhydrous Na₂SO₄ and evaporated at reduced pressure. The residue was purified by column chromatography (hexane-AcOEt, 90:10) to obtain the aldehyde (128 mg, 0.34 mmol, 63%) as a colorless oil. NMR ¹H (300 MHz, CDCl₃) δ 9.78 (t, *J=* 1.6 Hz, 1H, CHO), 7.39 (m, 2H, H-2" and H-6"), 7.06 (m, 2H, H-3" and H-5"), 6.73 (m, 3H, H-5, H-7, H-8), 4.94 (s, 2H, OC*H₂*Ph-p-F), 2.75 (m, 2H, CH₂-4), 2.60 (m, 2H, CH₂-2'), 1.90 (m, 4H, CH₂-3, CH₂-1'), 1.30 (s, 3H, CH₃-2); NMR ¹³C (75 MHz, CDCl₃) δ 202 (CHO), 162.4 (d, *J*_{CF}=244.4 Hz, C-4"), 152.1 (C-6), 147.7 (C-8a), 133.1 (d, *J*_{CF}= 3.3 Hz, C-1"), 129.3 and 129.2 (d, 2C, *J*_{CF}= 8.3 Hz, C-2", C-6"), 121.4 (C-4a), 117.7 (CH-5), 115.2 (d, 2C, *J*_{CF}= 24.8 Hz, CH-3", CH-5"), 115.2 and 114.5 (CH-7 and CH-8), 74.6 (C-2), 69.9 (O*CH₂*Ph-p-F), 38.4 (CH₂-2'), 31.8 (CH₂-1'), 31.2 (CH₂-3), 23.7 (CH₃-2), 22.3(CH₂-4); HREIMS *m*/*z* (%) 329.1552 [M]⁺ (329.1547 calc for C₂₀H₂₁O₃F), 343.1710 (343.1704 calc for C₂₁H₂₃O₃F).
**(e) Grignard reaction and Johnson-Claisen transposition:** A solution of the aldehyde intermediate (0.2 g, 0.61 mmol) in anhydrous THF (5 mL) was shaken at -78°C under a N₂ atmosphere for 15 min, and then treated with a solution of isopropenyl magnesium bromide 0.5 M (7.32 mL, 3.66 mmol) (Sen *et al.,* 1990).⁷ The mixture was shaken at -78°C for 3 h. The reaction mixture was treated with a saturated solution of NH₄Cl and shaken for 15 min at ambient temperature. H₂O was added and extracted with AcOEt (3 × 15 mL). The organic phases were combined, and the resulting organic phase was washed with H₂O (3 × 15 mL) and brine (3 × 15 mL), dehydrated with anhydrous Na₂SO₄ and concentrated in the rotary evaporator.

The residue obtained (250 mg) was used in the following reaction unpurified. The residue was treated with 10 mL triethyl orthoacetate and catalytic amounts of isobutyric acid (3 drops) (Sen *et al.,* 1990).⁷ The mixture was shaken at 140°C for 2 h. When cooled, the mixture was concentrated in the rotary evaporator to eliminate excess triethyl orthoacetate. The residue was diluted with CH₂Cl₂ (20 mL) and washed with H₂O (3 × 15 mL), dehydrated with anhydrous Na₂SO₄ and concentrated in the rotary evaporator. The residue was purified by silica gel column chromatography (hexane/AcOEt, 98:2) to obtain 126 mg of compound **9** (0.29 mmol, 47 %) of the invention. NMR ¹H (500 MHz, CDCl₃) δ 7.39 (m, 2H, H-2" and H-6"), 7.06 (m, 2H, H-3" and H-5"), 6.73 (m, 3H, H-5, H-7, H-8), 5.14 (t, *J=* 7.0 Hz, 1H, CH-3'), 4.94 (s, 2H, O*CH₂*Ph-p-F), 4.11 (q, *J=* 7.4 Hz, 2H, COOC*H₂*CH₃), 2.72 (t, *J*= 6.8, 2H, CH₂-4), 2.39 (m, 2H, CH₂-6'), 2.28 (m, 2H, CH₂-5'), 2.08 (m, 2H, CH₂-2'), 1.82 (m, 2H, CH₂-3), 1.67 (m, 6H, CH₂-1'), 1.60 (s, 3H, CH₃-4'), 1.27 (s, 3H, CH₃-2), 1.23 (t, *J*= 7.3 Hz, 3H, COOCH₂C*H₃*); NMR ¹³C (125 MHz, CDCl₃) δ 173.4 (CO), 162.4 (d, *J*_{CF}=245 Hz, C-4"), 151.9 (C-6), 148.2 (C-8a), 133.5 (C-4'), 133.3 (d, *J*_{CF}= 3 Hz, C-1"), 129.2 (d, 2C, *J*_{CF}= 8.3 Hz, C-2", C-6"), 124.9 (C-3'), 121.7 (C-4a), 117.8 (CH-5), 115.3 (d, 2C, *J*_{CF}= 24.8 Hz, CH-3", CH-5"), 115.2 and 114.4 (CH-7 and CH-8), 75.6 (C-2), 70.1 (O*CH₂*Ph-p-F), 60.2 (COO*CH₂*CH₃), 39.2 (CH₂-1'), 34.6 (CH₂-6'), 33.2 (CH₂-5'), 30.9 (CH₂-3), 24.1 (CH₃-2), 22.4 (CH₂-4), 22.2 (CH₂-2'), 15.8 (CH₃-4'), 14.2 (COOCH₂*CH₃*); HREIMS *m*/*z* (*%*) 441.2441 [M]⁺ (441.2436 calc for C₂₇H₃₃FO₄).

### 1.4. Synthesis of the compounds 10 to 12 of the invention:

Steps (a), (b), (c), (d) and (e) were performed as described in section 1.3 for compound 9 of the invention, although the O-protection of the benzopyran ester was performed with benzyl chloride (R₁= benzyl) in the case of compounds 10 and 11 of the invention and, with *p-*fluorobenzyl chloride (R₁= *p*-FluoroBn) in the case of compound 12 of the invention.

After performing step (e), another reduction of the ester group obtained is performed by Johnson-Claisen transposition, to again obtain an aldehyde, in the conditions described in step (d) of section 1.3.

The compounds 10 and 12 of the invention were obtained by olefination of the aldehyde obtained by Wittig reaction with a phosphorus ylide (Ph)₃P=C(CH₂CH₃)(COOCH₂CH₃) or Horner-Wadsworth-Emmons reaction with a phosphonate (EtO)₂P(O)-CH(CH₂CH₃)(COOCH₂CH₃). Compound 11 of the invention was obtained by hydrolysis of the ester group, by treatment with KOH at reflux of compound 10 of the invention.

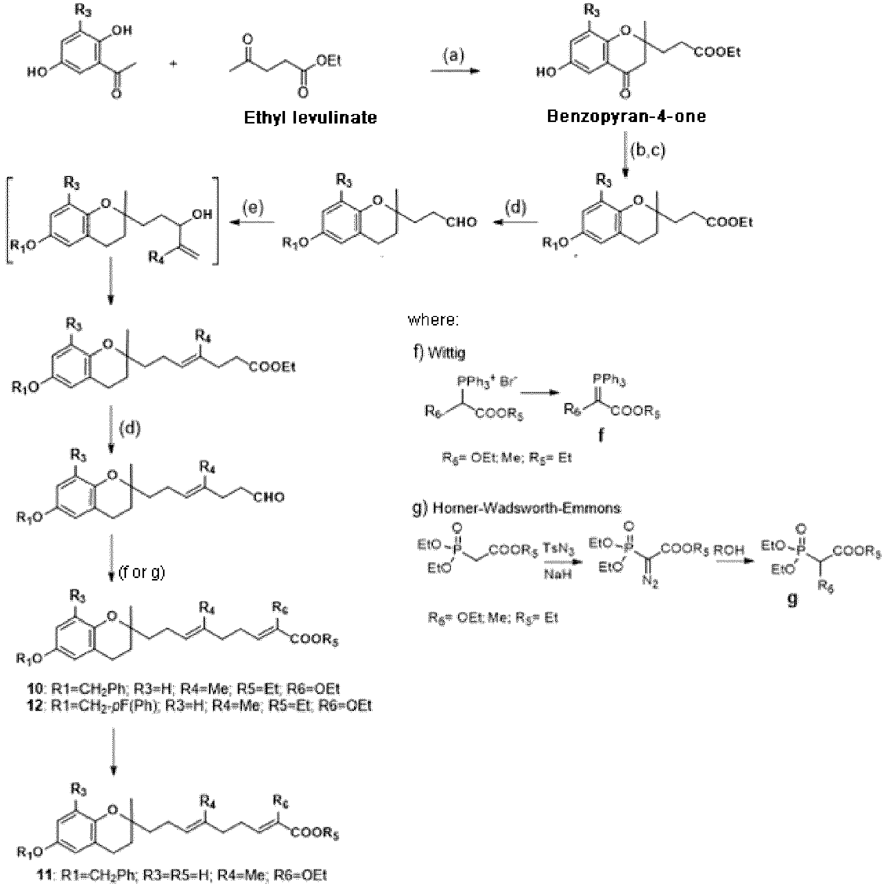

### 1.5. Synthesis of compound 13 of the invention:

Steps (a), (b), (c) and (d) were performed as described in section 1.3, wherein the O-protection of the benzopyrane ester was performed with benzyl chloride. The aldehyde obtained after step (d) was subjected to olefination by Wittig reaction with a phosphorus ylide (Ph)₃P=C(CH₂CF₃)(COOCH₂CH₃) or Horner-Wadsworth-Emmons reaction with a phosphonate (EtO)₂P(O)-CH(CH₂CF₃)(COOCH₂CH₃).

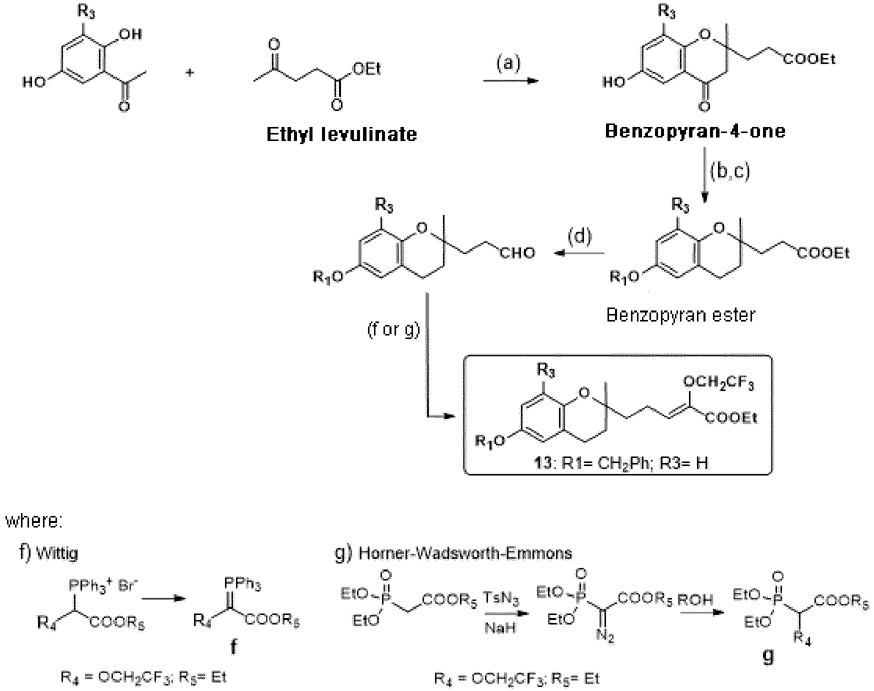

### In vitro studies

### Example 2: Evaluation of hPPARα and hPPAR transactivationγ

The natural compounds 1 and 2 for use according to the present invention, the compounds **3, 4, 4a, 4b** and **4c** of the invention, compound 5 for use according to the present invention, the compounds **6-8** of the invention and the compound **9** of the invention were studied *in vitro* by PPARα/γ transactivation assays. A transcription assay was performed in Cos-7 cells transiently transfected with a luciferase reporter plasmid in the presence of expression vectors pGAL4hPPARα and pGAL4hPPARy (Carmona *et al.,* 2007). The Cos-7 cells were obtained from ATCC (CRL-1651). The cells were maintained under standard culture conditions (Dulbecco modified minimum essential Eagle medium, DMEM) supplemented with 10% fetal calf serum (FCS) at 37°C in 5% humidity CO₂ atmosphere. The medium was changed every 2 days. The Cos-7 cells were seeded in 60 mm discs at a density of 5.5 × 10⁵ cells/disc in DMEM supplemented with 10% FCS and incubated at 37°C for 16 h before transfection. The cells were transfected in DMEM using jetPEI, with reporter (pG5-TK-pGL3) and expression (pGal4-h, hPPARα or hPPARγ) plasmids. The expression plasmid pCMV-β-galactosidase was cotransfected as a control for transfection efficacy. After 16 h the transfection was stopped adding DMEM supplemented with 10% FCS, and the cells were trypsinised and seeded in 96-well plates and incubated for 6 h in DMEM containing 10% FCS. Subsequently, the cells were incubated for 24 h in DMEM containing 0.2% FCS and increasing concentrations of the compounds or the vehicle (DMSO, maximum concentration 0.1%) were tested. Finally, the cells were washed once with PBS cooled on ice, lysed and the luciferase and β-galactosidase assays were performed.

To evaluate the compounds for PPAR transcriptional activity, the tests were conducted in a PPAR/Gal4 chimera of a human luciferase reporter gene to determine the maximum transactivation response of each compound. The different compounds were tested in a concentration range from 0.001 to 10 µM. The results obtained for all the compounds were compared with the data of the reference compounds WY-14643 and rosiglitazone in hPPARα and hPPARγ, respectively.

The results are summarized in **Table 1.**

| **Table 1** | **hPPARα** | | **hPPARγ** | |
|---|---|---|---|---|
| **Compound** | **EC₅₀ (nM)** | **Efficacy (%)** | **EC₅₀ (nM)** | **Efficacy (%)** |
| Rosiglitazone | 10000 | 15 | 4 | 100 |
| WY-14643 | 10000 | 100 | NA | NA |
| Polycerasoidol, 1 | 184 | 107 | 84 | 95 |
| Polycerasoidin, 2 | 1000 | 82 | 1000 | 55 |
| 3 | 10000 | 100 | 991 | 86 |
| 4a | 10000 | 18 | 10000 | 0 |
| 4b | 10000 | 28 | 10000 | 0 |
| 4c salt | 10000 | 191 | 1692 | 88 |
| 5 | 10000 | 27 | 574 | 35 |
| 6 | 10000 | 14 | 10000 | 0 |
| 7 | 1616 | 76 | 10000 | 37 |
| 8 | 10000 | 39 | 12 | 24 |
| 9 | 300 | 251 | 400 | 57 |
| 10 | 10000 | 60 | 2000 | 183 |
| 11 | 1500 | 96 | 300 | 62 |
| 12 | 10000 | 113 | 2000 | 183 |
| 13 | 10000 | 166 | 400 | 39 |

| | | | | |
|---|---|---|---|---|
| (^{a}) Maximum activity obtained with each compound expressed as a percentage of the maximum activity of WY-14643 for PPARα (EC₅₀ 12 µM) and rosiglitazone for PPARγ (EC₅₀ 4 nM) respectively. (NA: not active on the tested hPPAR). | | | | |

Among all the compounds tested, the natural product polycerasoidol **(1)** for use according to the present invention, showed the greatest PPARα/γ dual agonist activity, and its maximum response was comparable to that obtained by the reference compounds (WY-14643 and rosiglitazone). The data of EC₅₀ showed that natural compound **1** for use according to the present invention, and its semisynthetic derivative **3** of the invention, with the free phenol group, show total agonist activity for hPPARα and hPPARγ, and high transactivation percentages (107-100% and 95-86% for hPPARα and hPPARγ, respectively). The compound **4c** of the invention, the O-propylated derivative with a free carboxylic function in C-9', showed the greatest transactivation percentage (191% and 88% for hPPARα and hPPARγ, respectively). The remaining derivatives of polycerasoidol **(4a** and **4b** of the invention and **5** for use according to the present invention) showed lower transactivation percentages than the compound **1** for use according to the present invention, and the compounds **3** and **4c** of the invention at the tested concentrations. The modifications on the carboxylic group in C-9' of the side chain in position 2, introducing the group COOCH(COOCH₃)₂ result in compound **7** of the invention with selectivity for PPARα activation (76% for hPPARα and 37% for hPPARγ). However, the derivative **8** of the invention, with a group COOCH₂COOCH₃ in C-9', showed a lower PPARα activation (39% for hPPARα and 24% for hPPARγ). The compound **9** of the invention also showed dual agonist activity and a high transactivation percentage (251% for hPPARα and 57% for hPPARγ, respectively).

The compounds **10, 11, 12** and **13** of the invention also showed PPARα and PPARγ dual agonist activity, standing out the potency of compound 11 of the invention, with transactivation percentages of 96% and 62% for hPPARα and hPPARγ respectively.

It should be noted that compounds **1** (natural) for use according to the present invention and **9** (synthetic) of the invention showed powerful dual agonist action for receptors PPARα and PPARγ. Compound **2** of the invention showed moderate PPARα/γ dual activity and compound **8** of the invention displaced its selectivity to the activation of PPARγ **(Table 1).**

### Example 3: Cytotoxicity studies on human neutrophils and human umbilical vein endothelial cells (HUVEC)

It was previously mentioned that there are significant limitations in the use of PPAR agonists in humans, due to adverse effects such as hepatotoxicity, cancer, cardiotoxicity and other cardiovascular effects. Glitazars are the most widely studied compounds with PPARγ agonist activity. However, as indicated in the introduction, only the use of the dual agonists saroglitazar (Lipaglyn^{™}) in India since 2013 and lobeglitazone in Korea (Duvie^{™}), with antidiabetic and hypolipemiant effects, is approved. Nowadays, there is a greater interest in developing PPARα and PPARγ dual agonists with fewer adverse effects than PPARα or PPARγ selective ligands (Tan *et al.,* 2017).

To evaluate potential adverse effects (cytotoxicity) of the compounds of the invention, the compounds **1** and **2** for use according to the present invention, compound **8** of the invention, and the reference compounds were evaluated at concentrations of 30 and 100 µM in human neutrophils **(****Fig. 1A****)** and primary cultures of human umbilical vein endothelial cells (HUVEC) **(****Fig. 1B****)** using two different techniques: MTT colorimetry assay and flow cytometry analysis for cellular apoptosis and survival. In addition, the synthetic compound 9 of the invention was evaluated at 10, 30 and 100 µM **(****Fig. 2****).**

MTT assay. The viability of neutrophils and HUVEC was determined by the MTT colorimetric assay MTT (3(4,5-dimethylthiazol-2-yl)-2,5-diphenyltetrazoyl bromide).

Neutrophils were obtained from the leukocyte-platelet fraction (buffy coats) of healthy donors by centrifuging with Ficoll-Hypaque density gradient.

Human umbilical vein endothelial cells (HUVEC) were isolated with the collagenase treatment and maintained in a human specific endothelial base medium (EBM-2) supplemented with endothelial growth medium (EGM-2) and 10% FCS. Cells of the first passage were cultured until their confluence in 24-well plates. Before starting each experiment, the cells were incubated for 24 h in a medium containing 1% FBS.

100 µL of neutrophils and HUVEC (2 × 10⁵ cells/mL) suspension in supplemented RPMI medium were added to each well of a 96-well plate. The cells were incubated in absence and/or presence of the compounds at 37°C for24h. An MTT solution was prepared (2 mg/mL in PBS). 100 µL of the MTT solution were added to each well and incubated at 37°C for an additional 3 h. The supernatants were discarded and 200 µL of DMSO were added to each well to dissolve the precipitated formazan. The optical densities at two wavelengths (560 and 630 nm) were measured in a spectrophotometer (Infinite M200, Tecan, Mannedorf, Switzerland).

All the compounds of formula (I) tested showed significant cytotoxicity at 100 µM on both types of cells **(****Figures 1** **and** **2****).** The compound polycerasoidin (2) for use according to the present invention and the derivative **8** of the invention also showed toxicity at 30 µM on neutrophils and HUVEC, and WY-14643 produced cytotoxicity at 30 µM (9%) but only on human neutrophils **(****Fig. 1****).** The synthetic compound **9** of the invention showed cytotoxicity only in HUVEC at 30 µM **(****Fig. 2****)** (12% with respect to the vehicle).

In addition, the apoptosis and survival rate for human neutrophils and HUVEC was determined by double staining with annexin V and propidium iodide (PI) by flow cytometry.

Cytofluorometric analysis of apoptosis and survival. The kit FITC Annexin V Apoptosis Detection kit (BD Bioscience, San Jose, CA) was used. The staining protocol and all the reagents were supplied with the commercial kit. HUVEC were separated from the culture flasks by accutase (StemPro^{®} Accutase^{®} Cell dissociation reagent, Thermofischer Scientific Waltham, MA). Both cell types were incubated at 37°C for 24h in the presence or absence of the test compounds. Confluent HUVEC and freshly isolated neutrophils were washed, resuspended in binding buffer 1x (1 × 10⁶ cells/mL) and stained with annexin V conjugated with FITC and propidium iodide (PI) as described in the manufacturer's instructions. The cells were analyzed in a BD FACSVerse Flow Cytometer (BD Biosciences, San Jose, CA) and differentiated as early apoptosis cells (annexin V⁺ and PI⁻), late apoptosis cells and/or necrotic cells (annexin V⁺ and PI⁺), and live cells (annexin V⁻ and PI⁻).

In neutrophils only compound **8** of the invention and rosiglitazone at a concentration of 100 µM caused a minimal effect on cell survival (5.3% and 8.5% respectively) **(****Figure 3B****).** The synthetic compound **9** of the invention also caused a minimal effect on neutrophil survival at 30 and 100 µM concentration (1.07% and 1.55%, respectively), compared to the vehicle **(****Figure 4B****).** Instead, all the compounds increased the apoptosis significantly and reduced the survival at 30 µM concentration in HUVEC, with the exception of the compound polycerasoidol **(1)** for use according to the present invention **(**Figures 3C and D) and compound **9** of the invention **(**Figures 4C and D)\. In HUVEC, the most toxic compounds were the compound polycerasoidin **(2)** for use according to the present invention, its analogue **8** of the invention and rosiglitazone, which reduced cell survival by 38,9, 61,3 and 79,2%, respectively, at 100 µM concentration and exerted some significant effects at 30 µM concentration **(**Figures 3C and D). It is worth noting the absence of toxicity of compound 9 of the invention in HUVEC at the tested concentrations, including at 100 µM, compared with the vehicle **(****Figures 4** **C** and **D).**

The introduction mentioned the limited use on humans of numerous PPARα and PPARγ agonists due to toxicity problems which is responsible of significant adverse effects. In fact, although rosiglitazone has numerous beneficial effects in patients with type 2 diabetes, the evidence of its adverse cardiovascular effects, that include the risk of myocardial infarction and cardiac insufficiency, has forced the withdrawal of the drug in numerous countries of the European Community (Palee *et al.,* 2011). Thus, toxicity studies are important in this type of compounds, and the MTT and Flow cytometry assays we have conducted show that the compound **1** for use according to the present invention and compound **9** of the invention are less cytotoxic than the specific reference agonists for PPARα and PPARγ.

### Example 4: Effect of the compounds 1 and 2 for use according to the present invention, and compounds 8 and 9 of the invention on the leukocyte-endothelium interaction induced by TNFα under flow conditions

The endothelium that covers the blood vessel lumen has several functions, the alterations of which ("endothelial dysfunction") have been related to cardiovascular diseases, thromboembolism and atherosclerosis. In fact, endothelial dysfunction is one of the first manifestations of the start of atherosclerosis that leads to a proinflammatory and prothrombotic phenotype of the endothelium, causing the release and migration of leukocytes into the subendothelial space.

PPARs, in particular PPAR*α* and PPARγ, are expressed in most vascular cells, including endothelial cells and smooth muscle cells, where they participate in specific anti-inflammatory effects and lipid control (Rosenson *et al.,* 2012). ¹² PPARγ agonists (rosiglitazone) can inhibit the inflammatory process, reducing the accumulation of neutrophils and mononuclear cells (Palee *et al.,* 2011).

The parallel flow camera technique was used, which allows viewing cell adhesion under dynamic physiological flow conditions.

In the execution of this study, neutrophils and mononuclear cells were perfused through monolayers of HUVEC previously stimulated, or not, with TNFα (20 ng/mL) for 24 h. For this purpose, the neutrophils and human mononuclear cells were obtained in suspension from healthy volunteers by the Ficoll-Hypaque method, centrifuging by density gradient. Freshly isolated cells (1 × 10⁶/mL) were perfused through the monolayer of endothelial cells (HUVEC) stimulated, or not, with TNFα for 24 h. In all the experiments the interactions of the leukocytes were determined after being perfused for 5 min at 0.5 dyne/cm². The HUVEC plates were placed in the flow chamber (GlycoTech, Gaithersburg, MD) and a camera was coupled to a phase-contrast inverted microscope (Axio Observer A1 Carl Zeiss microscope, Thornwood, NY) to view cell adhesion (20x lens and 10x eyepiece). In addition, the images were recorded on video for subsequent analysis.

The HUVEC were pretreated with the compounds polycerasoidol **(1)** and polycerasoidin **(2)** for use according to the present invention, and compound **(8)** or compound **9** of the invention at doses of 1, 3, 10, 30 and 100 µM, as well as WY-14643 or rosiglitazone at 1 µM, 20 hours before stimulation with TNFα (Sanz *et al.,* 2012).

TNFα caused significantly increased adhesion to endothelial cells of mononuclear leukocytes and neutrophils **(****Figures 5** **and** **6****).** None of the compounds significantly inhibited HUVECneutrophil interactions at a concentration of 1 µM; however, it should be noted that pretreatment with the compound polycerasoidol **(1)** for use according to the present invention, the synthetic analogue **9** of the invention and rosiglitazone significantly reduced the adhesion of HUVEC-mononuclear cells **(****Figures 5** **and** **6****)** in a concentration-dependent manner **(****Figures 5C** **and** **6B****).** In this assay the compound polycerasoidol **(1)** for use according to the present invention was 5 times less potent than rosiglitazone, while compound **9** of the invention showed a potency approximately 3.6 and 16 times greater than rosiglitazone and polycerasoidol, respectively (values IC₅₀ of 0.30 µM vs. 1.1 µM and 4.9 µM, respectively). Based on these results and the cytotoxicity studies on apoptosis and survival in neutrophils, it can be concluded that the compound polycerasoidol **(1)** for use according to the present invention and the synthetic compound **9** of the invention were able to inhibit the recruitment of mononuclear cells without compromising the immune response of neutrophils, necessary for the host defenses. Therefore, it seems that both compounds are interesting candidates for additional immuno-pharmacological assays given their capability of reducing mononuclear cell recruitment, their potency as dual PPARα/γ agonists and their low cytotoxicity.

### Example 5: The compound polycerasoidol (1) for use according to the present invention and compound 9 of the invention inhibited mononuclear cell adhesion to the endothelium, induced by TNFα via RXRα/PPARγ interaction

The compound polycerasoidol (1), for use according to the present invention, and the synthetic compound 9 of the invention are dual PPARα/γ agonists and rosiglitazone is a PPARγ selective agonist. To suppress PPARα or PPARγ expression in endothelial cells, we have used a gene silencing approach via interfering RNA (siRNA).

Confluent HUVEC were transfected with specific siRNA for RXRα, PPARα and PPARγ (Dharmacon, Lafayette, CO) using RNAiMAX of lipofectamin (Invitrogen, Carslbad, CA) for 48 h (Sanz *et al.,* 2012). ¹³ Then the cells were treated for 20 h with the compound polycerasoidol (1), for use according to the present invention, at 10 µM or rosiglitazone at 1 µM and subsequently stimulated for 4 h with TNFα (10 ng/mL). To confirm, the silencing of PPARα, PPARγ and RXRα via siRNA, a Western blot analysis was performed (Sanz *et al.,* 2012). ¹³ After silencing, the cells were washed, scraped, collected and centrifuged at 15.000 g at 4°C for 30 min to obtain the total protein extract. The protein concentration was determined by the Bradford method. The samples were denatured, subjected to 10% SDS-polyacrylamide gel electrophoresis (SDS-PAGE) and transferred to a nitrocellulose membrane. The membranes were blocked with 5% BSA-PBS/0.05% Tween 20 and incubated with the corresponding antibody. Rabbit polyclonal antibodies against RXRα and PPARγ and the mouse monoclonal antibody against PPARα were acquired from Santa Cruz Biotechnology (dilution: 1:400, Santa Cruz, CA). An anti-rabbit or anti-mouse IgG secondary antibodies linked to radish peroxidase were supplied by DakoCytomation (dilution 1:2000, Denmark).

48 hours after transfection with PPARα or PPARγ specific siRNA, the HUVEC showed > 68% and 66% reduction in protein, respectively, compared to the levels detected in siRNA control cells **(****Figure 7****).** In control siRNA transfected cells, stimulation with TNFα induced significant increases in the adhesion of mononuclear cells that were inhibited when the endothelial cells were pretreated with the compound polycerasoidol **(1),** for use according to the present invention, or rosiglitazone **(**Figure 8A and B). It should be noted that while these responses were not affected by PPARα silencing **(****Figure 8A****),** the responses induced by the compound polycerasoidol **(1),** for use according to the present invention, were annulled in HUVEC deficient in PPARγ **(****Figure 8B****).** PPAR form heterodimers with RXR which in some cases respond in a synergic manner to both agonists. Since PPARγ can interact with RXRα to generate their anti-inflammatory activity (Sanz *et al.,* 2012), the directed deletion of the expression of RXRα in HUVEC with RXRα specific siRNA (>72% reduction in protein, **Figure 7****)** also completely suppressed the inhibitory effect of the compound polycerasoidol **(1),** for use according to the present invention, on mononuclear cell adhesion induced by TNFα **(****Figure 8C****).**

In addition, to confirm the interaction between PPARγ and RXRα, HUVEC were immunoprecipitated with an anti-PPARγ antibody and were subjected to electrophoresis and western-blot with an anti-RXRα antibody, and increased RXRα/PPARγ interaction was detected in cells pretreated with the compound polycerasoidol **(1),** for use according to the present invention, or rosiglitazone **(****Figure 8D****).**

For the immunoprecipitation, the protein extracts were obtained in 50 mM Tris-HCl (pH 8), 150 mM NaCl, 1% Nonidet P-40 and protease (1 mM PMSF, 40 µg/mL aprotinin and 40 µg/mL leupeptin) and phosphatase inhibitors. Then the protein extracts (~200 µg) were incubated with 5 µg of an antibody against PPARγ. Immunocomplexes were precipitated using an anti-rabbit IgG beads (cat# 8800, eBioscience, San Diego, CA) and suspended in a sample buffer with 50 mM dithiotreitol (DTT). Western blot was performed with an antibody against RXRα and the membranes were incubated with the secondary HRP-conjugate Rabbit TrueBlot (eBioscience, San Diego, CA) as secondary antibody. Chemoluminescence signals were developed with ECL (GE Healthcare, Madrid, Spain).

The same immunoprecipitation experiment was conducted with the synthetic compound **9** of the invention **(****Figure 9****).** In view of the results, it can be concluded that the compound polycerasoidol **(1),** for use according to the present invention, and the compound **9** of the invention, do not require the activation of PPARα to inhibit the recruitment of mononuclear cells, but they require the activation of RXRα and PPARγ to exert their anti-inflammatory effect, as with other PPARγ or RXRα selective agonists (Sanz *et al.,* 2012).

### Example 6: The compound polycerasoidol (1), for use according to the present invention, and the synthetic compound 9 of the invention reduced the expression of the intercellular adhesion molecule-1 (ICAM-1), the vascular cell adhesion molecule-1 (VCAM-1) and fractalquine (CX₃CL1), induced by TNFα

In cardiovascular and cardiometabolic diseases (hypertension, obesity, diabetes, metabolic syndrome) high levels have been detected of circulating mediators, among other cytokines and chemokines that can represent endothelial function disorders. Proinflammatory cytokines stimulate the endothelium to express cellular adhesion molecules (CAM), such as vascular cell adhesion molecule-1 (VCAM-1) and intercellular adhesion molecule-1 (ICAM-1), increasing leukocyte adhesion to vascular endothelial cells, which is an important process in the initial phase of the inflammatory and immunological response (Zernecke *et al.,* 2010). PPARγ is expressed in the vasculature and leukocytes, and its activation can reduce the expression of CAMs, such as ICAM-1 or VCAM-1, as well as the expression of CX₃CL1 (fractalkine). CX₃CL1 is a chemokine expressed on the surface of endothelial cells and it can bind to its receptor CX₃CR1, expressed in monocytes, T lymphocytes and NK cells, promoting cell adhesion. CX₃CL1 can also be detached from the cell surface under the action of ADAM 10 and ADAM 17, releasing a soluble form that can act as a chemoattractant of the leukocytes expressing its receptor. In addition to CX₃CL1, there is another transmembrane chemokine, CXCL16, which as CX₃CL1 is also expressed in endothelial cells and can be detached by the action of ADAM 10. It exerts its action by interacting with its CXCR6 receptor, present in certain leukocyte subtypes, promoting the adhesion thereof to the endothelial surface. Thus, these chemokines and adhesion molecules regulate the adhesion and migration of leukocytes to the vascular wall, and there are indications that an increase in their endothelial expression is associated with an increase in atherosclerosis (Zernecke *et al.,* 2008).

To investigate the expression ofVCAM-1, ICAM-1, fractalkine (CXaCL1) and CXCL16, HUVEC were pretreated with the compound polycerasoidol **(1)** (10 µM), for use according to the present invention, or with compound **9** (3 µM) of the invention for 24 h, and then stimulated with TNFα (20 ng/mL) for another 24 h. The cells were collected from the flasks using accutase (StemPro^{®} Accutase^{®} Cell Dissociation Reagent, ThermoFischer Scientific, Waltham, MA).

Then they were washed and incubated for 1 h with a APC-conjugated mAb antibody against human VCAM-1 (100 µg/mL; clone STA, BioLegend, San Diego, CA), a FITC- conjugated mAb against human ICAM-1 (400 µg/mL; clone HA58, BioLegend, San Diego, CA), a PE-conjugated mAb against human CX₃CL1 (1.25 µg/mL; clone 51637, R&D Systems, Minneapolis, MN) or with a APC-conjugated mAb antibody against human CXCL16 (clone 256213, R&D Systems, Abingdon, UK), all at a dilution of 1:25 in 3% BSA/PBS. The samples were analyzed using a BD FACSVerse Flow Cytometer (BD Biosciences, San Jose, CA).

This study showed that pretreating HUVEC with the compound polycerasoidol **(1),** for use according to the present invention, for 20 h at 10 µM significantly reduced the expression of ICAM-1 and VCAM-1, induced by TNFα **(****Fig. 10****),** the effect being stronger for VCAM-1 (87.8% inhibition) than for ICAM-1 (23.8% inhibition). To investigate the effect of polycerasoidol **(1)** on the expression of fractalkine (CXaCL1), the cells were stimulated with TNFα and the responses were evaluated by flow cytometry and immunofluorescence after 24 h.

The results showed that the compound polycerasoidol **(1),** for use according to the present invention, at 10 µM reduced the expression of this chemokine by 77.6% **(****Figure 10C****).** The expression of adhesion molecules such as VCAM-1, ICAM-1 or chemokines as CX₃CL1 or CXCL16, was also determined for compound **9** of the invention at 3 µM, which showed a significant decrease in the expression of VCAM-1, CX₃CL1 and CXCL16 **(****Figure 11****).**

To perform the immunofluorescence assays, confluent HUVEC were cultured on glass and some cells were incubated for 24 h with the compound polycerasoidol **(1)** (10 µM), for use according to the present invention, or compound **9** (3 µM) of the invention, before being stimulated with TNFα (20 ng/mL) for an additional 24 h. The cells were then washed twice with PBS (1x), fixed with 4% paraformaldehyde and blocked in a PBS (1x) solution containing 1% BSA. HUVEC were incubated at 4°C overnight with a mouse primary monoclonal antibody against human VCAM-1 (dilution 1:200; clone 1.G11B1, Serotec, Kidlington, UK), ICAM-1 (dilution 1:200; clone 6.5B5, Serotec, Kidlington, UK), CX₃CL1 (dilution 1:200; clone 81506, R&D Systems, Minneapolis, MN) or CXCL16 (1:200 dilution; Immunostep, Salamanca, Spain) in a 0.1% BSA/PBS solution, followed by incubation with a goat alexa-fluor 488-conjugated anti-rabbit secondary antibody (dilution 1:1000; A11034, Life Technologies, Carlsbad, CA) at room temperature for 1 h. The cell nuclei were counterstained with Hoechst (dilution 1:4000 in PBS, Sigma-Aldrich, Madrid, Spain). Images were obtained with a fluorescence microscope (Axio Observer A1, Carl Zeiss, Thornwood, NY) equipped with a 40x lens and 10x eyepiece.

### Example 7: The compound polycerasoidol (1), for use according to the present invention, and compound 9 of the invention, inhibit activation of p38 MAPK and NF-κB induced by TNFα in HUVEC

There is substantial evidence that PPAR ligands can modulate MAPK signaling pathways (Park *et al.,* 2011). According to our investigation the intracellular signaling pathways are underlying the inhibitory responses exerted by the compound polycerasoidol **(1),** for use according to the present invention, and the synthetic compound **9** of the invention.

The effect of the compound polycerasoidol **(1),** for use according to the present invention, or compound **9** of the invention, on the phosphorylation of p38 MAPK and p65 (NF-κB) induced by TNFα was determined in HUVEC by flow cytometry. HUVEC were previously incubated for 24 h with compound **1** (10 µM), for use according to the present invention, or with compound **9** (3 µM), of the invention, and stimulated for 1 h with TNFα (20 ng/mL). The endothelial cells were fixed with BD Cytofix Fixation Buffer (BD Biosciences, San Jose, CA) and permeabilized with BD Sol Perm III solution (BD Biosciences, San Jose, CA) and sequentially stained with a 1:10 dilution of a PE-conjugated mAb antibody against human p65 (pS529, clone K10-895.12.50; BD Biosciences, San Jose, CA) and a 1:10 dilution of Alexa Fluor-conjugated mAb antibody against human p38 MAPK (pT80/pY182, clone 36/p38; BD Biosciences, San Jose, CA). The cells were analyzed with a BD FACSVerse flow cytometer (BD Biosciences, San Jose, CA).

The results show that compound polycerasoidol **(1),** for use according to the present invention, was able to decrease substantially (63.4%) the activation of p38 MAPK, as also could compound **9** (71.9%) of the invention **(****Figures 12A** and **13A****).** In addition, the activation of various compounds in the MAPK family is associated with the transactivation of NF-κB (p65). The mobilization of NF-κB to the nucleus activates the transcription of genes that encode the expression of numerous inflammatory mediators, such as endothelial adhesion molecules or the synthesis of cytokines and chemokines (Monaco *et al.,* 2004). We could determine that compound polycerasoidol **(1),** for use according to the present invention, and compound 9 of the invention, were able to inhibit significantly the activation of NF-κB induced by TNFα **(****Figures 12B** and **13B****).**

### In vivo studies

### Example 8: Treatment of oblob mice with compound 9 of the invention.

Protocols conforming to European Union guidelines for the care and protection of animals were approved by the Ethics Committee of the University of Valencia. All efforts were made to minimize the number of animals used and their suffering. *ob*/*ob* mice have a leptin deficiency, and are associated with hyperphagia and obesity, as well as hyperglycemia and hyperinsulinemia. This study used male *ob*/*ob* mice (C57BU6J) (Charles River laboratories, Chatillon-sur-Chalaronne, France).

The mice were housed in individual cages in a room with controlled temperature, humidity and light. They were kept and bred (UCIM animal house, University of Valencia) in pathogen-free conditions with free access to food and autoclaved water, at a humidity of 60-65% and a constant temperature of 22±2°C with a dark/light cycle of 12 h (light from 06:00-18:00 h, dark from 18:00-06:00 h). The mice were allowed to acclimate for ten days prior to the study.

Male *ob*/*ob* mice aged 7 to 8 weeks were treated daily orally using an orogastric tube with compound **9** (10 and 30 mg/Kg/d) of the invention, rosiglitazone (10 mg/ Kg/ d) and/or WY-14643 (30 mg/Kg/d) between 9:00 and 11:00 a.m. by oral gavage for 4 days. The control *ob*/*ob* mice were just administered with vehicle (physiological saline solution with HEC 1%). The doses were selected in view of prior results obtained from an experiment with *ob*/*ob* mice (n=3) in which compound **9** of the invention and the compounds of reference, rosiglitazone and WY-14643, were administered intraperitoneally (data not shown). 6 animals were used in each group in independent experiments (n=6). The compounds were prepared freshly each day in a 1% suspension of HEC for oral administration. Randomization was performed based on the glycaemia values on day 0. For the duration of the experiment the amount of food ingested and the body weight were determined daily for each mouse. Every day between 9:00 and 11:00 a.m. (prior to treatment) blood glucose levels were measured using (Contour next USB Blood glucose meter, BAYER, Basel, Switzerland). On the fifth day of the experiment the mice were anesthesized with isofluorane and blood was extracted by cardiac puncture. The blood was collected in tubes containing EDTA or heparin. Plasma samples were obtained by centrifugation and stored at -80°C. After obtaining the samples, the animals were sacrificed by cervical dislocation and subsequently different organs such as white adipose tissue (WAT) and liver were extracted for additional studies. The organs were weighed and frozen for subsequent RNA analysis and to conduct histological studies

### Histological analysis

The samples of epididymal white adipose tissue (WAT) and liver were fixed in 4% paraformaldehyde, embedded in paraffin, sectioned with a microtome (Leica RM2245, Leica Biosystems, Wetzlar, Germany) and mounted in microscope slides Superfrost^{®} plus (Thermo Fisher Scientific, Waltham, MA). Cross sections of WAT and liver were obtained. At least 10 slides were examined containing ≈ 4 cross sections of tissue (5 µm thickness) of each animal. Hematoxilin and eosin (H&E) staining was performed of the WAT and liver. Inflammatory infiltration in WAT was measured as previously described (Toyoda *et al.,* 2008). After inactivation of the peroxidase (3% H₂O₂) and blocking with goat serum (Abcam, Cambridge, UK), cross sections of WAT were incubated overnight (4°C) with the following primary (mAb) monoclonal antibody: a rabbit anti-mouse F4/80 mAb (dilution 1:100, Abcam, Cambridge, UK). A specific marking was detected with a secondary goat HRP-conjugated anti-rabbit antibody (dilution 1:500, Dako, Copenhagen, Denmark). To confirm the specificity of the antibodies, control antibodies with the same isotope (Abcam, Cambridge, UK) or secondary antibody were used only as negative controls. The samples were revealed with a solution containing 3,3'-diaminobenzidine (DAB, Vector Laboratories Inc, Burlingame, CA), and subsequently stained with Harris hematoxilin (Sigma-Aldrich, Madrid, Spain) and dehydrated. Five fields of each WAT tissue section were photographed (Axio Observer A1, Carl Zeiss microscope, Thornwood, NY) digitalized and analyzed *(Imaged* software 1.48V, Bethesda, MA). Quantification was performed double blind with codes.

### Extraction of mRNA and real-time PCR

The total RNA was extracted from mouse WAT by homogenization. Reverse transcription was performed with 1000 ng of total RNA using the inverse transcription kit TaqMan (Applied Biosystems, Thermo Fisher Scientific, Waltham, MA) and converted to cDNA by standard methods. The cDNA was amplified with specific feeders for the mouse monocytic chemoattractant protein 1 (MCP-1 or CCL2, Mm00441242_m1) in a Real Time PCR 7900HT apparatus (Applied Biosystems, Thermo Fisher Scientific, Waltham, MA) using Universal Master Mix (Applied Biosystems, Thermo Fisher Scientific, Waltham, MA). All feeders were designed by Applied Biosystems. The relative quantification of the various transcriptions was determined by the 2^{-ΔΔCt} method using GAPDH (Applied Biosystems, Thermo Fisher Scientific, Waltham, MA) as endogenous and normalized control for the control group.

### Measurement of metabolites

Free fatty acids (FFA) in plasma, and the enzymes aspartate aminotransferase (AST) and alanine aminotransferase (ALT) were determined with a colorimetric assay kit (Sigma-Aldrich, Madrid, Spain). In addition the plasma lipid levels for total cholesterol and triglycerides (TG) were measured by enzymatic methods (WAKO, Cape Charles, VA). The concentration of HDLcholesterol (HDL-c) was also determined with the same method used to measure total cholesterol, after precipitating the apolipoprotein-B (apoB) with dextran sulphate/MgCl₂ (Sigma-Aldrich, Madrid, Spain) as previously described (Zieske *et al.,* 2005).

### 8.1 Compound 9 of the invention does not cause variations in body weight and feeding efficiency

An adverse effect during treatment with TZD in individuals with T2D is weight gain (Ahmadian *et al.,* 2013). To determine the effects of the treatment with compound **9** of the invention, mice and food ingested were weighed daily. The weight gain at the end of treatment was calculated (d₅-d₀) **(****Figure 14A****).** Treatment with compound 9 of the invention at the doses of 10 and 30 mg/Kg/d did not cause significant differences in weight gain compared to the control group. Weight gain was 1.70 ±0.25, 2.5±0.32 and 1.88±0.52 g for control mice treated with rosiglitazone and compound 9 (30 mg/Kg/d) of the invention, respectively. Feed efficiency showed the same tendency, and no observable differences were found in mice treated with compound 9 of the invention **(****Figure 14B****).**

### 8.2 Compound 9 of the invention reduces glycaemia values

While after 4 days a 47% increase in glucose blood levels (248 vs. 300 mg/dL) was detected in control *ob*/*ob* mice administered with vehicle, administration of compound **9** of the invention decreased by 20% in glucose blood levels (261 *vs*. 194 mg/dL) at the dose of 30 mg/Kg/d compared to day 0 (d₀). Finally, treatment with rosiglitazone reduced glucose blood levels by 32% (217 vs. 135 mg/dL) **(****Figure 15****).**

### 8.3 Effect of compound 9 of the invention on white adipose tissue (WAT)

Treatment of *ob*/*ob* mice with compound **9** of the invention did not significantly increase the weight of epididymal white adipose tissue (WAT) compared to the control group **(****Figure 16****).** In addition, a histological examination of WAT revealed changes in cell morphology, with a significant decrease in the mean area of the adipocytes in mice treated with compound 9 of the invention in a dose-dependent manner, similar to the effect caused by WY-14643 **(****Figure 17****).** Thus suggesting the capability of compound **9** of the invention to prevent adipocyte hypertrophy. In contrast, treatment with rosiglitazone increased the number of small size adipocytes in WAT deposits, reducing the mean area of the adipocytes and confirming its adipogenic effect.

### 8.4 Anti-inflammatory effect of compound 9 of the invention on white adipose tissue (WAT)

There is evidence that, in obese individuals with T2D, a chronic inflammatory process that is initiated in the adipose tissue is triggered. In the WAT of obese mice, gene expression levels in inflammatory pathways are increased, resulting in an increase in inflammatory chemokines in the adipose tissue of obese individuals, such as monocyte chemoattractant protein (MCP-1), which participates in monocyte recruitment to the inflammation area (Yoshimura *et al.,* 1989), as well as an increased infiltration of macrophages in the adipose tissue (MacKnight *et al*., 1996).

Studies were conducted on the WAT inflammatory process and it was observed that the expression of MCP-1 was significantly reduced **(****Figure 18A****)** in *ob*/*ob* mice treated with compound 9 of the invention at 30 mg/Kg/d. In addition, the quantification of macrophages in WAT by immunohistochemistry using an antibody against F4/80, showed that compound **9** of the invention, at a dose of 30 mg/Kg/d, was able to significantly reduce the number of macrophages, indicating the capability of compound **9** of the invention to reduce macrophage infiltration in WAT, similar to the effect caused by rosiglitazone **(****Figure 18B****).**

### 8.5 Compound 9 of the invention improves blood lipid parameters

The effects on the dyslipidemia of *ob*/*ob* mice was studied, determining various plasma parameters such as total cholesterol, HDL-c, triglycerides and free fatty acids. Treatment with compound **9** of the invention at a dose of 30 mg/Kg/d and WY-14643 significantly increased total cholesterol levels with respect to the control group of *ob*/*ob* mice (50% and 64%, respectively) **(****Figure 19A****).** Compound **9** of the invention at the doses of 10 and 30 mg/Kg/d and WY-14643 also significantly increased HDL-c with respect to the control group of *ob*/*ob* mice **(****Figure 19B****).** Moreover, compound 9 of the invention and WY-14643 showed a decrease of triglyceride levels in plasma, although not in a significant manner, which may be due to the short treatment time (122 mg/dL or 101 mg/dL at a dose of 10 or 30 mg/Kg/d, respectively, vs. 137 mg/dL for the control *ob*/*ob*) **(****Figure 19C****).** Compound **9** of the invention at 30 mg/Kg/d and WY-14643 were able to significantly decrease blood levels of free fatty acids (0.25 and 0.33 mol/µL, respectively, *vs*. 0.67 mol/µL in the control *ob*/*ob*) **(****Figure 19D****).**

### 8.6 Effect of compound 9 of the invention in the liver

The effect of compound **9** of the invention in a primordial organ such as the liver was studied, showing a non-significant weight increase with respect to the *ob*/*ob* control group **(****Figure 20A****).** Quantification of the triglyceride content showed that in the short time of treatment with compound **9** of the invention at 30 mg/Kg/d, triglycerides did not accumulate significantly in the liver compared to the *ob*/*ob* control group **(****Figure 20B****).**

### 8.7. Compound 9 of the invention reduced alanine aminotransferase (ALT) and aspartate aminotransferase (AST) levels

Compound **9** of the invention reduced ALT and AST levels, the latter one experimenting a statistically significant decrease at a concentration of 30 mg/Kg/d (28%) compared to the *ob*/*ob* control group after 4 days of treatment **(****Figure 21****).** This suggests that compound **9** of the invention could reduce hepatic damage, showing a hepatoprotective effect.

### REFERENCES

1. Cheang, W. S. et al. The peroxisome proliferator-activated receptors in cardiovascular diseases: experimental benefits and clinical challenges. Br. J. Pharmacol. 2015, 172, 5512-5522.
2. Ahmadian, M. et al. PPARγ signaling and metabolism: the good, the bad and the future. Nat. Med. 2013, 19, 557-566.
3. Tan, C. K. et al. Synthetic and natural Peroxisome Proliferator-Activated Receptor (PPAR) agonists as candidates for the therapy of the metabolic syndrome. Expert Opin. Ther. Targets 2017, 21, 333-348.
4. González, M. C. et al. Polycerasoidin and polycerasoidol, two new prenylated benzopyran derivatives from Polyalthia cerasoides. J. Nat. Prod. 1995, 58, 1278-1284.
5. González, M. C. et al. Prenylated benzopyran derivatives from two Polyalthia species. Phytochem. 1996, 43, 1361-1364.
6. Kabbe, H.-J., Widdig, A. Synthesis and reactions of 4-chromanones. Angew. Chem. Int. Ed. Engl. 1982, 21, 247-256.
7. Sen, S. E. et al. Inhibition of vertebrate squalene epoxidase by extended and truncated analogues of trisnorsqualene alcohol. J. Med. Chem. 1990, 33, 1698-1701.
8. Bisceglia, J. A.; Orelli, L. R. Recent Applications of the Horner-Wadsworth-Emmons Reaction to the Synthesis of Natural Products. Curr. Org. Chem. 2012, 16, 2206-2230.
9. Toshima, K. et al. The First Total Synthesis of Concanamycin F (Concanolide A). J. Org. Chem. 2001, 66, 1708-1715.
10. Carmona, M. C. et al. S 26948: a new specific peroxisome proliferator-activated receptor-modulator with potent antidiabetes and antiatherogenic effects. Diabetes 2007, 56, 2797-2808.
11. Palee, S. et al. PPARgamma activator, rosiglitazone: Is it beneficial or harmful to the cardiovascular system? World J. Cardiol. 2011, 3, 144-152.
12. Rosenson, R. S. et al. Modulating peroxisome proliferator-activated receptors for therapeutic benefit? Biology, clinical experience, and future prospects. Am. Heart J. 2012, 164, 672-680.
13. Sanz, M. J. et al. Retinoid X receptor agonists impair arterial mononuclear cell recruitment through peroxisome proliferator-activated receptor-gamma activation. J. Immunol. 2012, 189, 411-424.
14. Zernecke, A.; Weber, C., Chemokines in the vascular inflammatory response of atherosclerosis. Cardiovasc. Res. 2010, 86, 192-201.
15. Zernecke, A. et al. Chemokines in atherosclerosis an update. Artherioscler. Thromb. Vasc. Biol. 2008, 28, 1897-1908.
16. Park, J. B. et al. Peroxisome proliferator-activated receptor-gamma agonists suppress tissue factor overexpression in rat balloon injury model with paclitaxel infusion. PLoS One 2011, 6, e2832.
17. Monaco, C.; Paleolog, E., Nuclear factor kappaB: a potential therapeutic target in atherosclerosis and thrombosis. Cardiovasc. Res. 2004, 61, 671-678.
18. Toyoda, T. et al. Effect of peroxisoma proliferator-activated receptor-α ligands in the interaction between adipocytes and macrophages in obese adipose tissue. Obesity 2008, 16, 1199-1207.
19. Zieske A. W. et al. Elevated serum c-reactive protein levels and advanced atherosclerosis in youth. Arterioscler. Thromb. Vasc. Biol. 2005, 25, 1237-1243.
20. Yoshimura, T. et al. Human monocyte chemoattractant protein-1 (MCP-1). Full-length cDNA cloning, expression in mitogen-stimulated blood mononuclear leukocytes, and sequence similarity to mouse competence gene JE. FEBS Lett. 1989, 244, 487-49321.
21. McKnight, A. J. et al. Molecular cloning of F4/80, a murine macrophage-restricted cell surface glycoprotein with homology to the G-protein-linked transmembrane 7 hormone receptor family. J. Biol. Chem. 1996, 271, 486-489.
22. Taha H et al., (2015) PLoS ONE 10(5) e0126126.doi: 10.1371 /journal.pone.0126126.
23. Zhao Yang et al., Journal of Agricultural and Food Chemistry (2010), 58(8), 4844-4852.
24. Karimian et al., (2015) PLoS ONE 10(5) e0127434.doi:10.1371/journal.pone.0127434.
25. Zafra-Polo et. Al., J. Nat. Prod., 1996, 59(10), 913-916.
26. WO02/26729 A2
27. Pearce B C et al.: "Hypercholesterolemic activity of synthetic and natural tocotrienols". Journal of Medicinal Chemistry 1992, vol. 35, no. 20, 30 November 1991 (1991-11-30), pages 3595-3606, XP002393508
28. EP0421419 A2
29. Sanz M-J et al.: "A novel dual PPARa and PPRγ agonist inhibit TNFa-induced mononuclear cell recruitment", Basic and Clinical Pharmacology, and Toxicology, vol. 123, no. 2, 1 August 2018 (2018-08-01), page 44.

## Claims

1. Compound selected from the group consisting of 3, 4, 4a, 4b, 4c, 6, 7, 8, 9, 10, 11, 12 and 13:

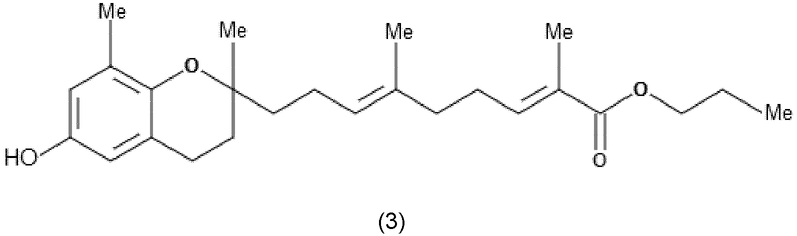

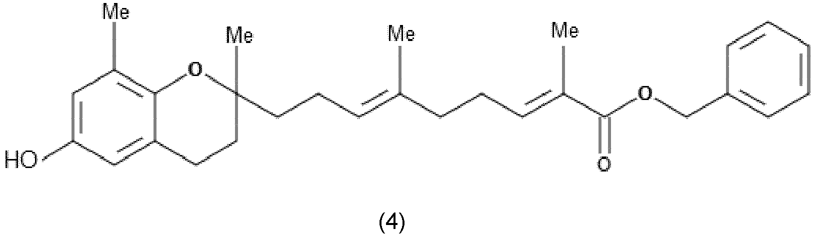

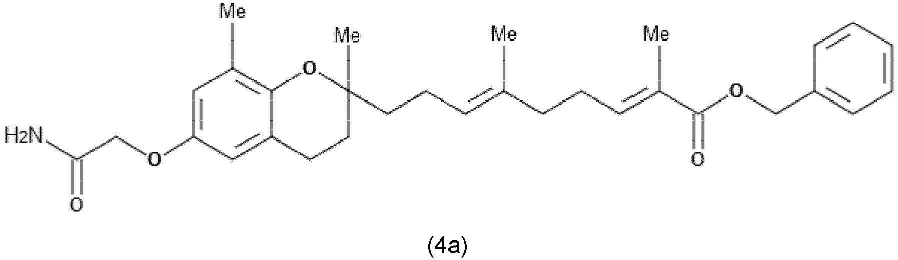

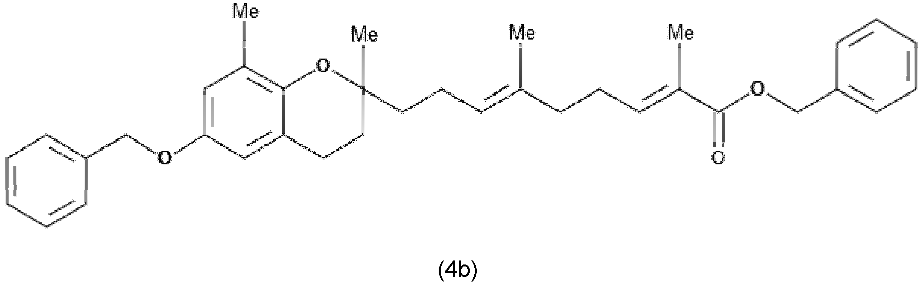

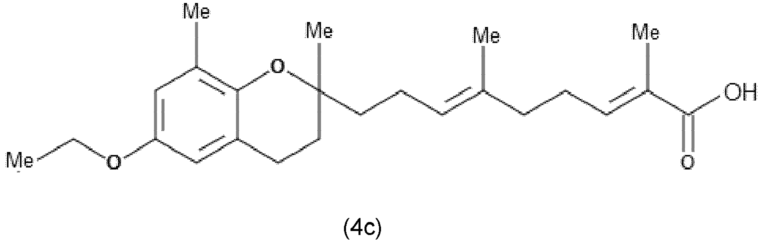

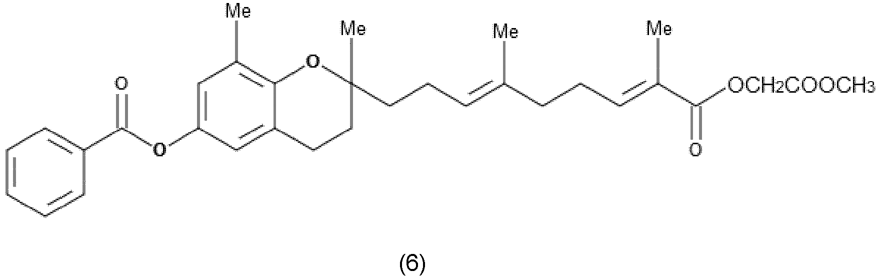

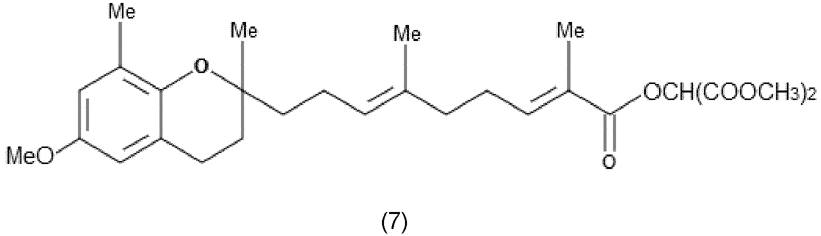

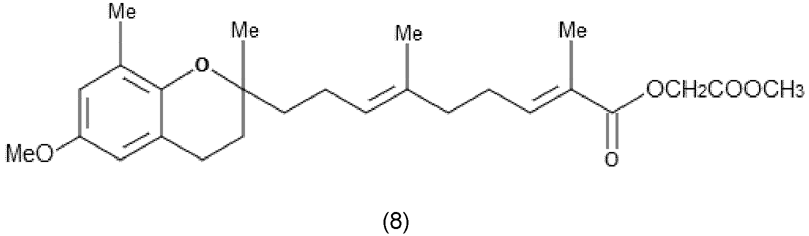

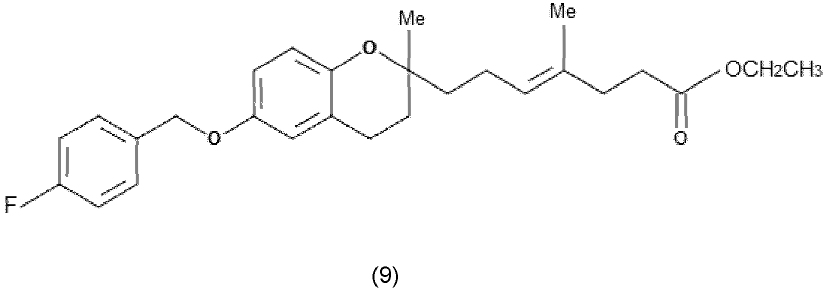

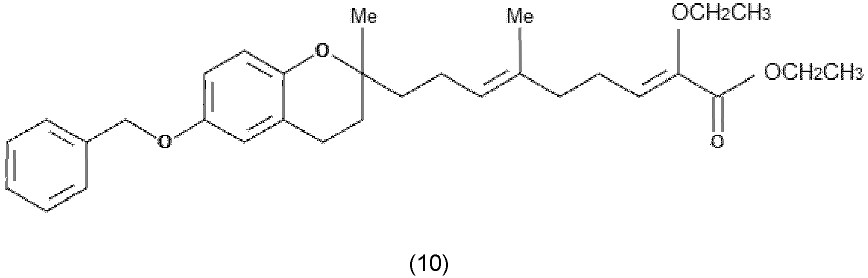

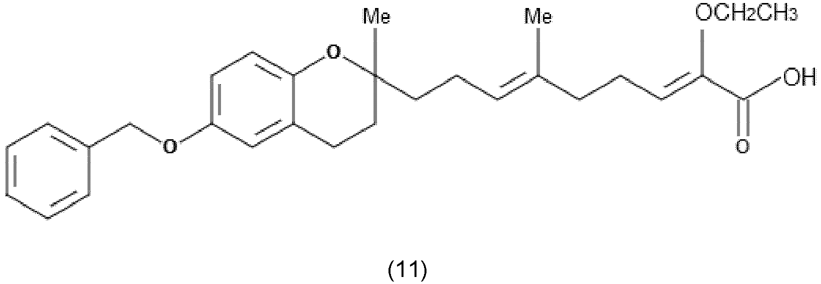

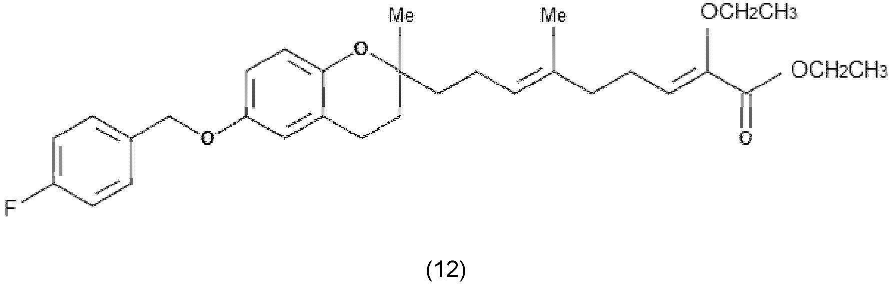

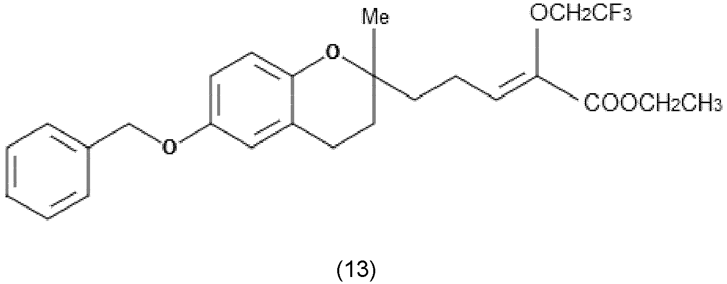

2. Compound according to claim 1, for use as a medicament.

3. Compound according to claim 1 for use in the treatment of a disease that responds to the administration of PPARα and/or PPARγ agonists, wherein the disease that responds to the administration of PPARα and/or PPARγ agonists is independently selected from the group consisting of cardiometabolic disorders, type 2 diabetes (T2D), obesity, metabolic syndrome, hypercholesterolemia, hypertriglyceridemia, primary chylomicronemia, hyperlipoproteinemia, familial dysbetalipoproteinemia, and neurodegenerative diseases.

4. Pharmaceutical composition comprising an effective amount of at least one compound of formula I, according to claim 1, and at least one pharmaceutically acceptable excipient.

5. Pharmaceutical composition according to claim 4, for use as a medicament.

6. Pharmaceutical composition according to claim 4 for use in the treatment of a disease that responds to the administration of PPARα and/or PPARγ agonists, wherein the disease that responds to the administration of PPARα and/or PPARγ agonists is independently selected from the group consisting of cardiometabolic disorders, type 2 diabetes (T2D), obesity, metabolic syndrome, hypercholesterolemia, hypertriglyceridemia, primary chylomicronemia, hyperlipoproteinemia, familial dysbetalipoproteinemia, and neurodegenerative diseases.

7. Compound independently selected from the group consisting of 1, 2, 3, 4, 4a, 4b, 4c, 5, 6, 7, 8, 9, 10, 11, 12 and 13: 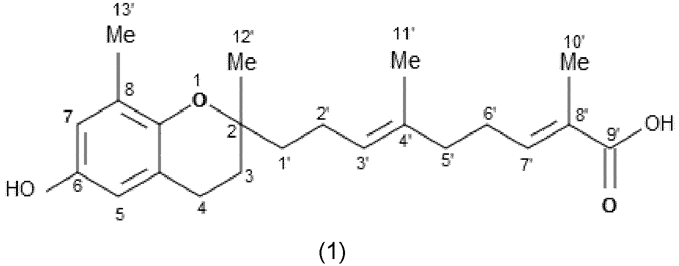 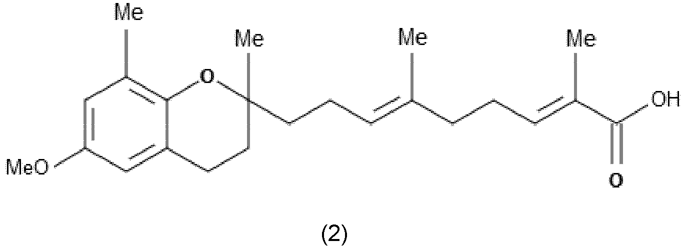 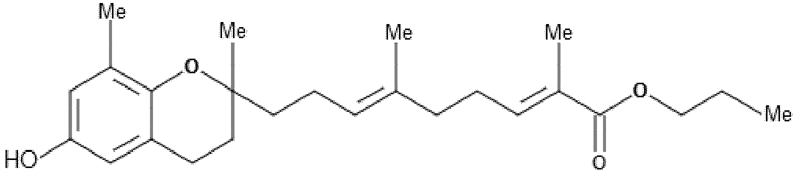 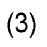 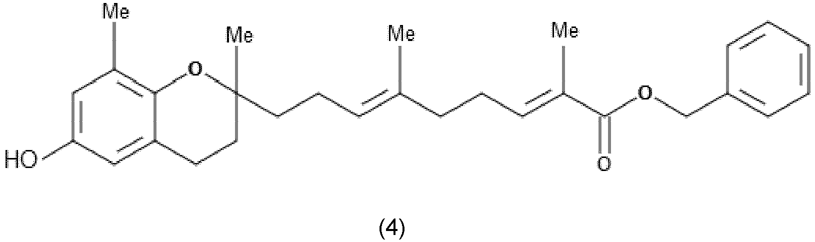 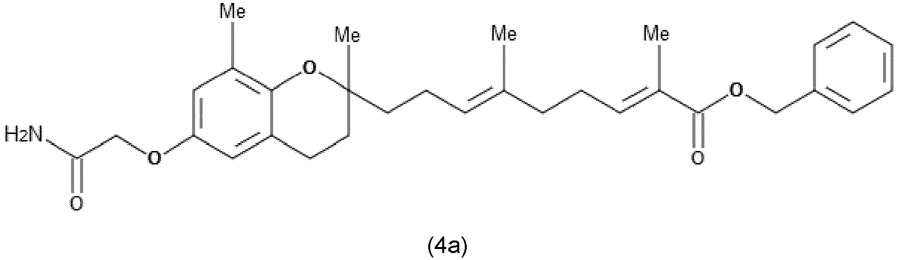 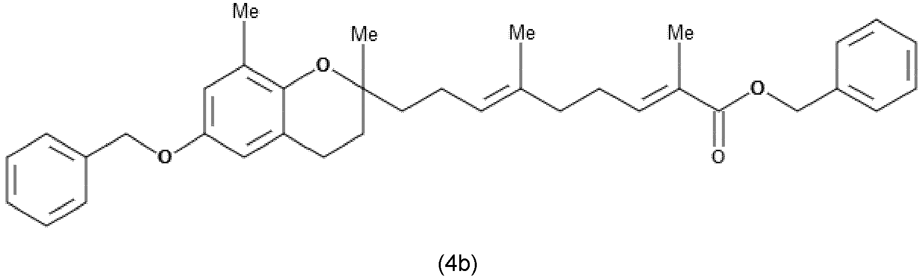 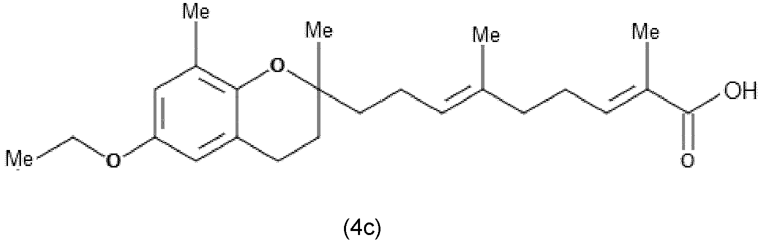 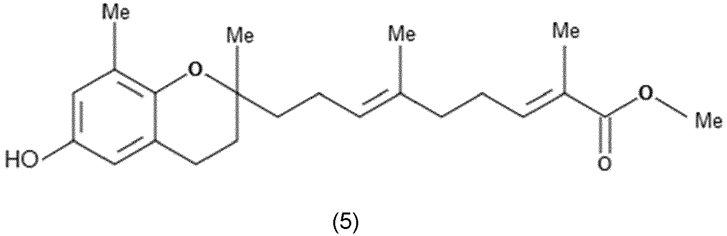 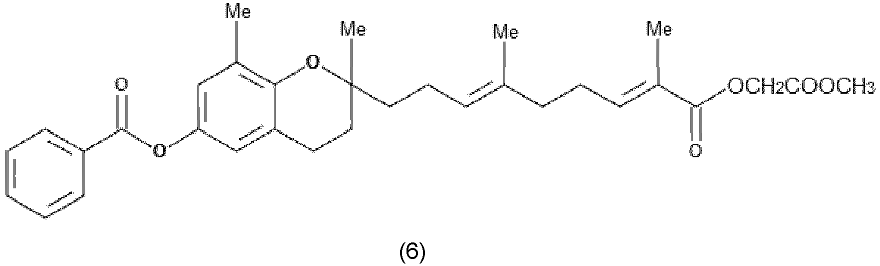 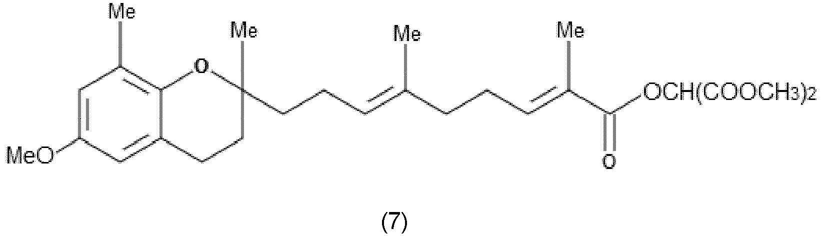 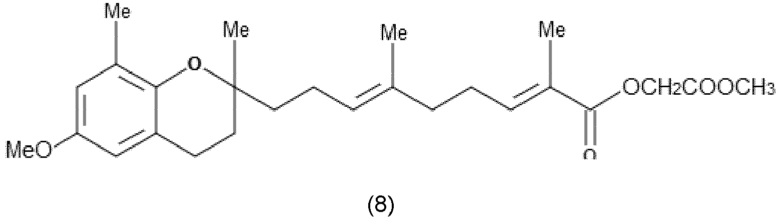 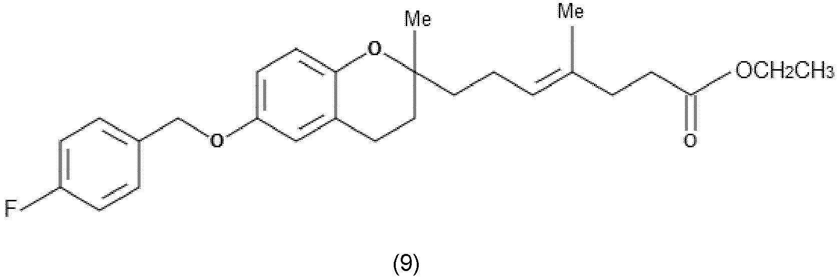 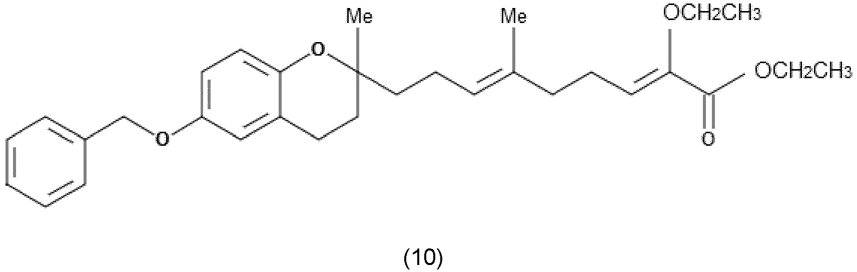 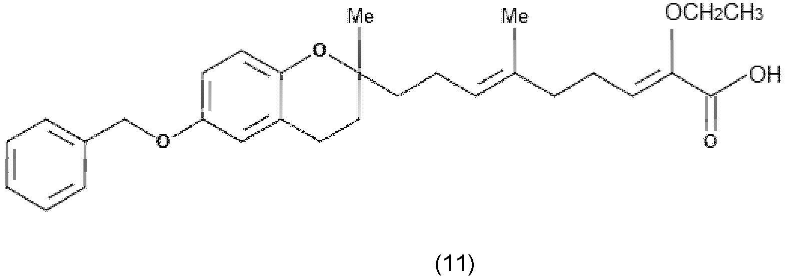 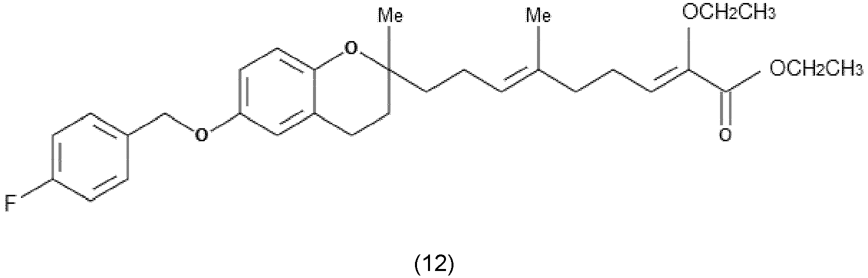 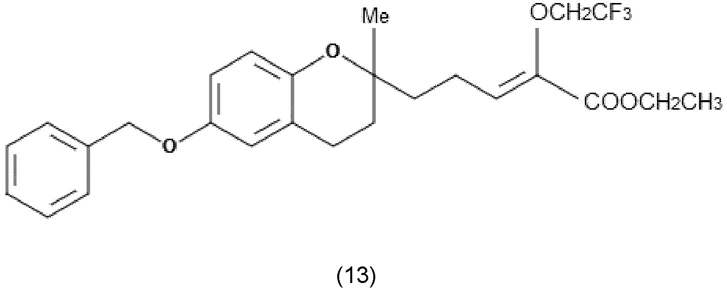 for use in the treatment of a disease that responds to the administration of PPARα and/or PPARγ agonists, wherein the disease that responds to the administration of PPARα and/or PPARγ agonists is independently selected from the group consisting of cardiovascular disorders, type 2 diabetes (DT2), obesity, metabolic syndrome, hypercholesterolemia, hypertriglyceridemia, primary chylomicronemia, hyperlipoproteinemia, familial dysbetalipoproteinemia, inflammation, and neurodegenerative diseases.

8. Pharmaceutical composition comprising an effective amount of at least one compound independently selected from the group consisting of 1, 2, 3, 4, 4a, 4b, 4c, 5, 6, 7, 8, 9, 10, 11, 12 and 13: 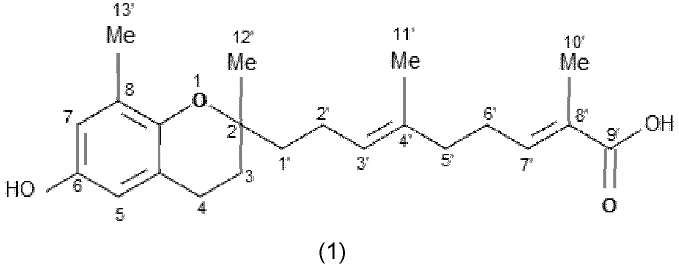 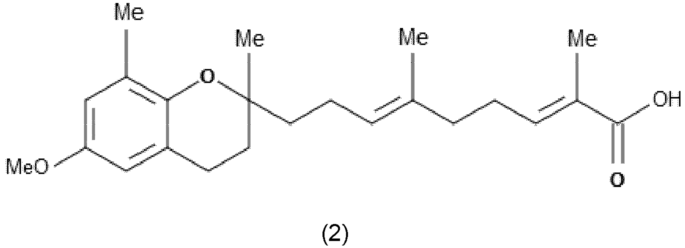 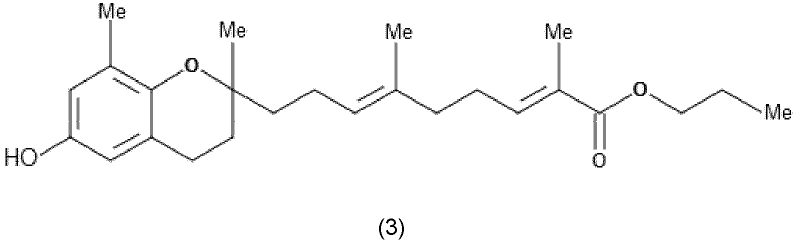 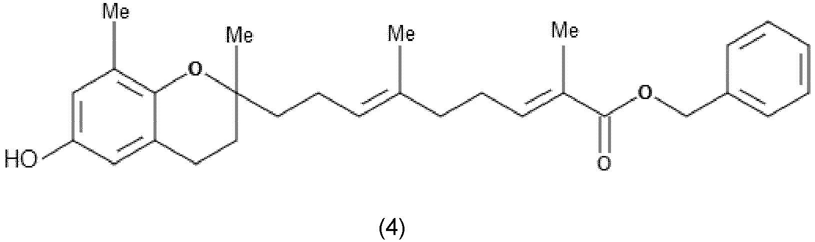 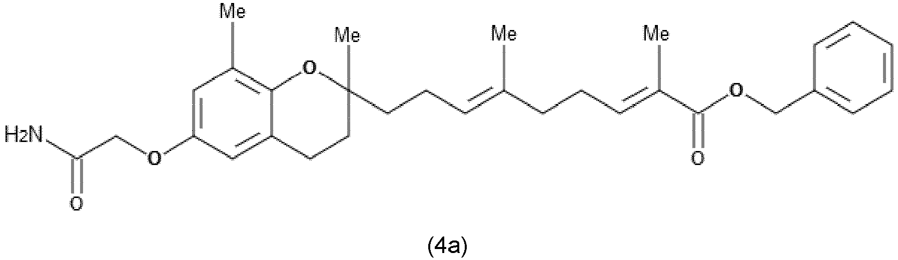 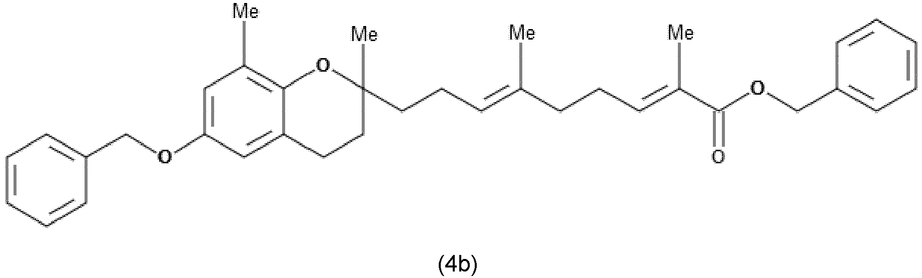 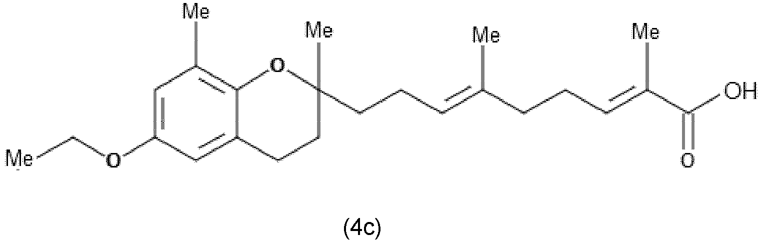 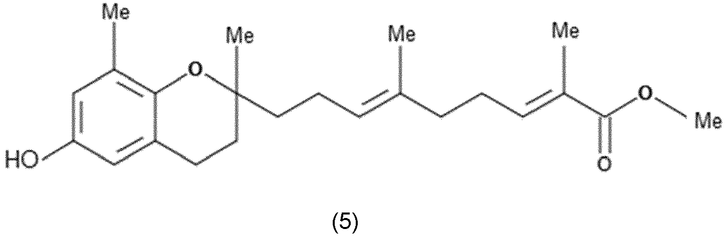 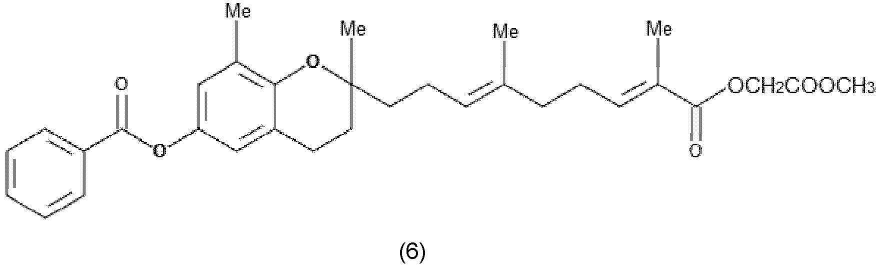 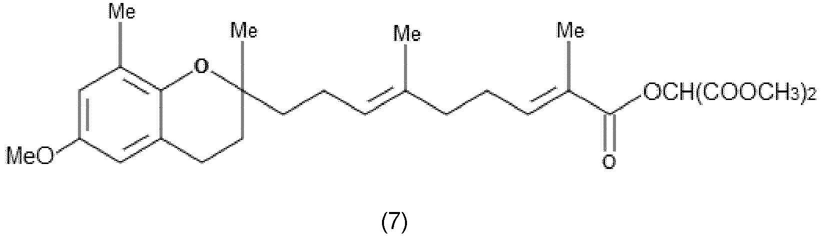 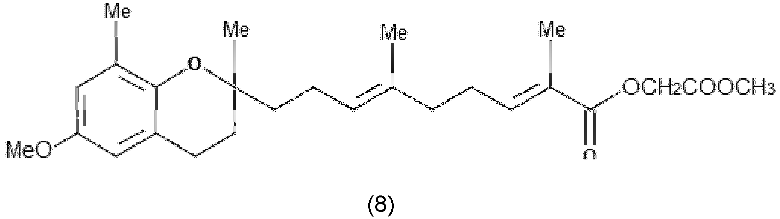 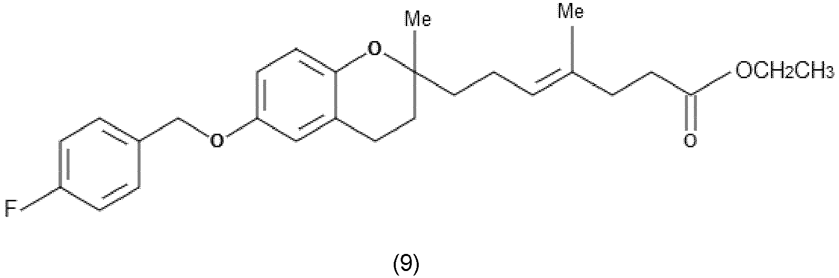 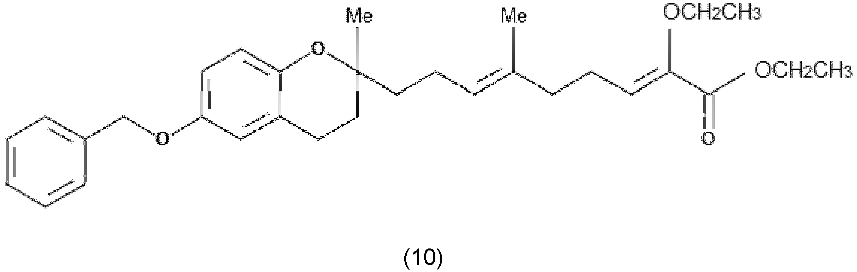 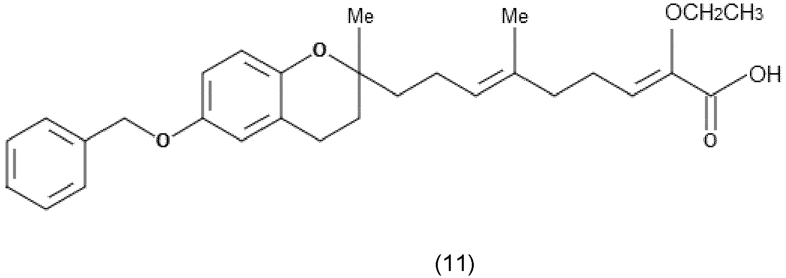 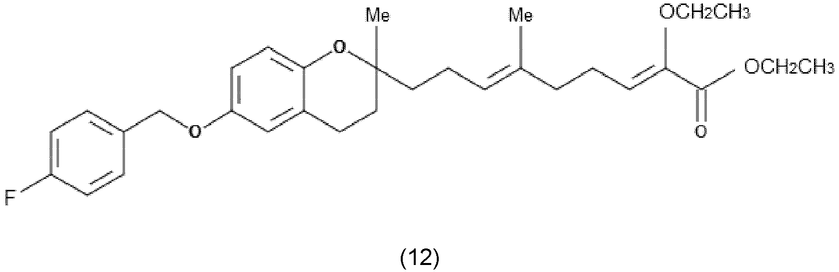 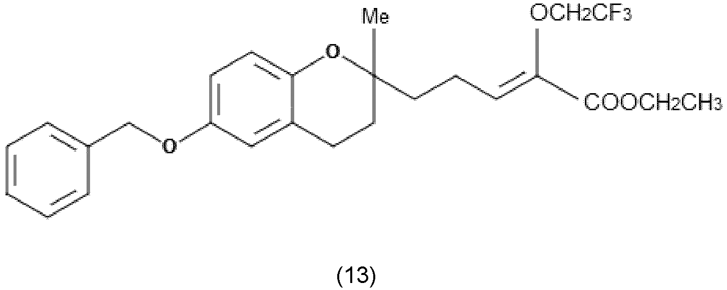 and at least one pharmaceutically acceptable excipient, for use in the treatment of a disease that responds to the administration of PPARα and/or PPARγ agonists, wherein the disease that responds to the administration of PPARα and/or PPARγ agonists is independently selected from the group consisting of cardiovascular disorders, type 2 diabetes (DT2), obesity, metabolic syndrome, hypercholesterolemia, hypertriglyceridemia, primary chylomicronemia, hyperlipoproteinemia, familial dysbetalipoproteinemia, inflammation, and neurodegenerative diseases.

## Patentansprüche

1. Verbindung ausgewählt aus der Gruppe bestehend aus 3, 4, 4a, 4b, 4c, 6, 7, 8, 9, 10, 11, 12 und 13:

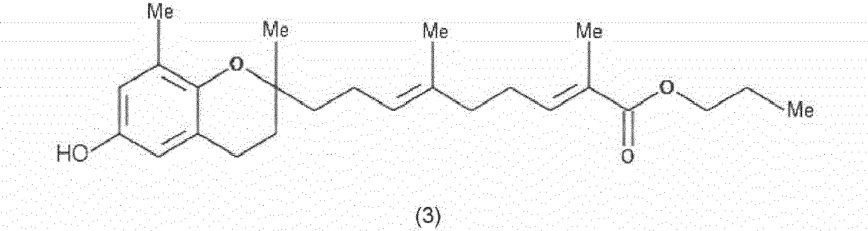

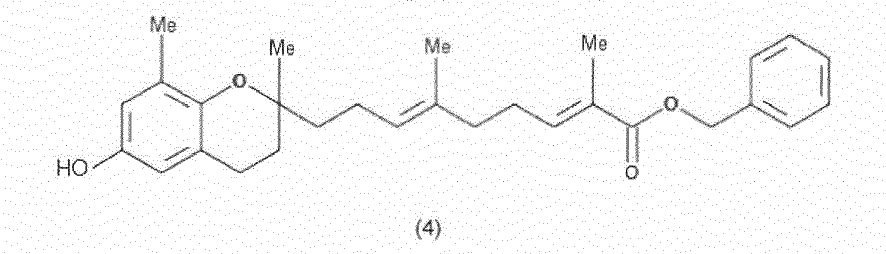

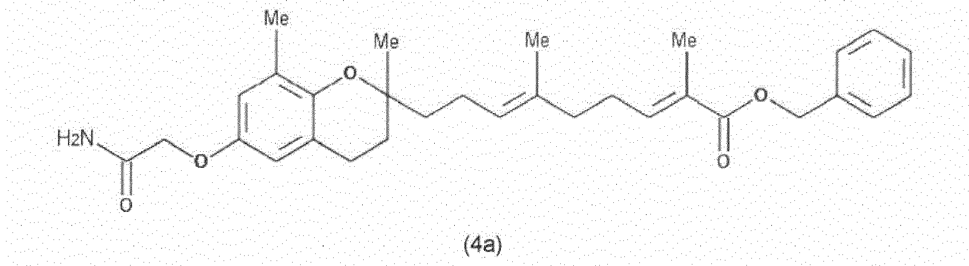

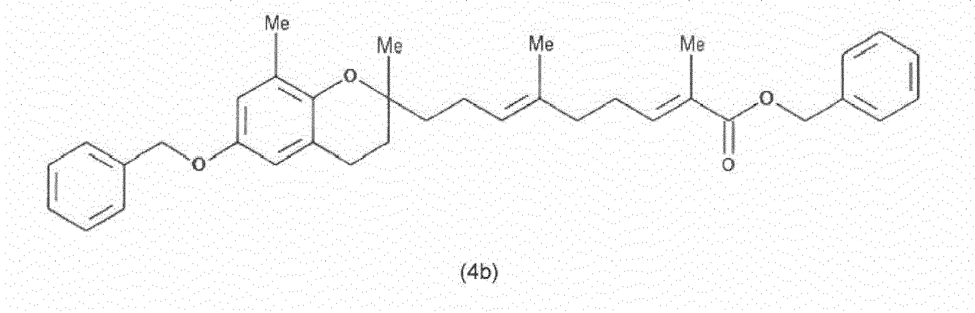

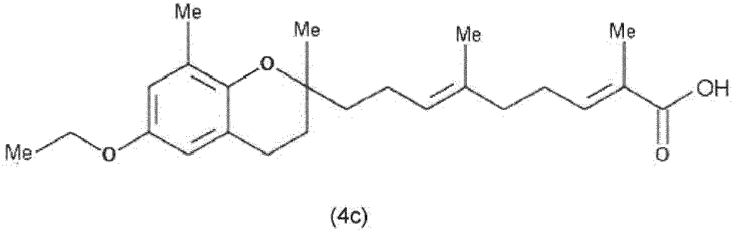

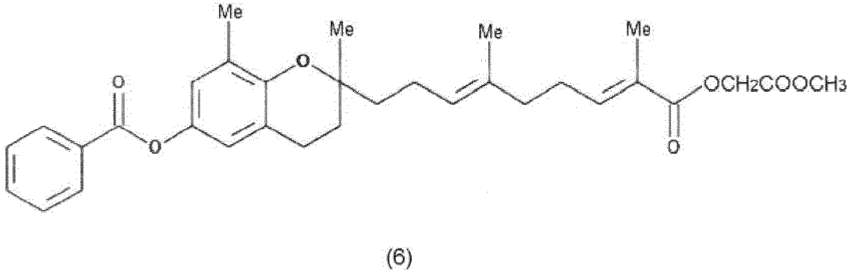

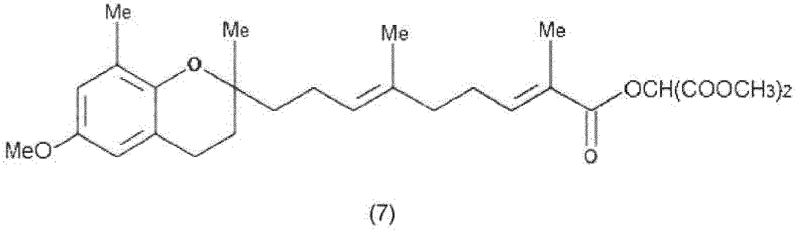

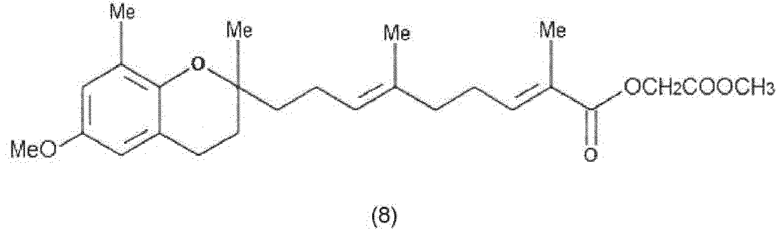

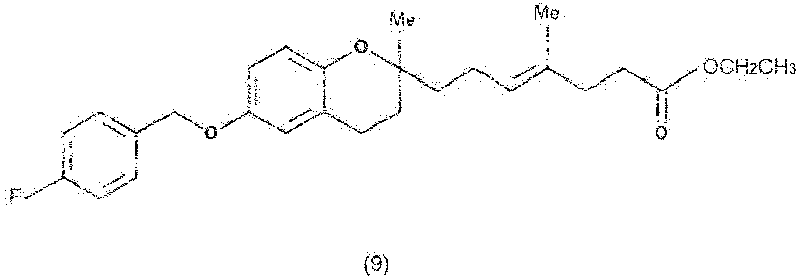

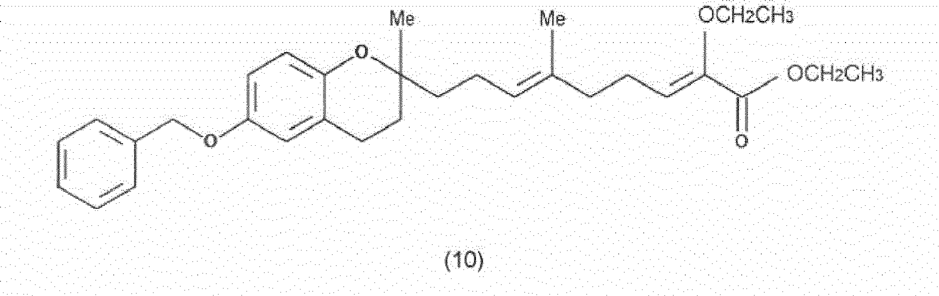

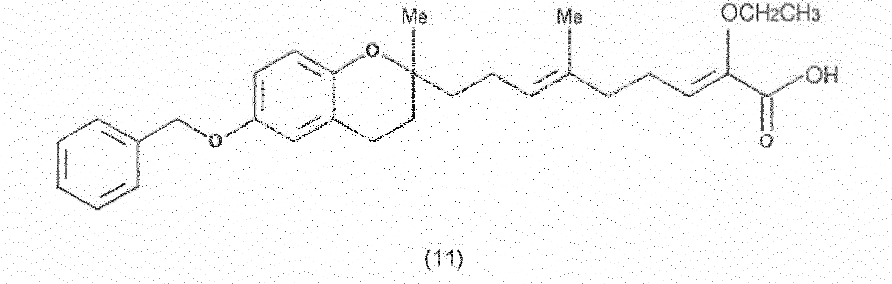

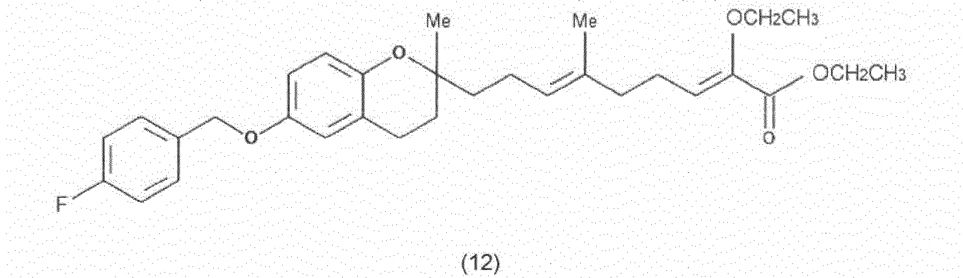

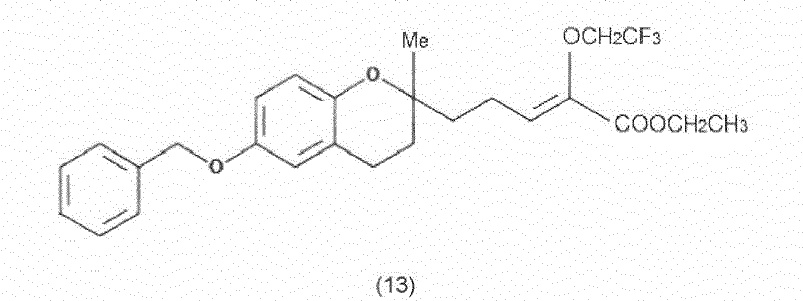

2. Zusammensetzung nach Anspruch 1 zur Verwendung als Medikament.

3. Zusammensetzung nach Anspruch 1 zur Verwendung bei der Behandlung einer Erkrankung, die auf die Verabreichung von PPARα- und/oder PPARγ-Agonisten anspricht, wobei die Erkrankung, die auf die Verabreichung von PPARα- und/oder PPARγ-Agonisten anspricht, unabhängig ausgewählt ist aus der Gruppe bestehend aus kardiometabolischen Störungen, Typ-2-Diabetes (T2D), Fettleibigkeit, metabolischem Syndrom, Hypercholesterinämie, Hypertriglyceridämie, primärer Chylomikronämie, Hyperlipoproteinämie, familiärer Dysbetalipoproteinämie und neurodegenerativen Erkrankungen.

4. Pharmazeutische Zusammensetzung, umfassend eine wirksame Menge mindestens einer Verbindung der Formel I nach Anspruch 1 und mindestens einen pharmazeutisch verträglichen Itilfsstoff.

5. Pharmazeutische Zusammensetzung nach Anspruch 4 zur Verwendung als Medikament.

6. Pharmazeutische Zusammensetzung nach Anspruch 4 zur Verwendung bei der Behandlung einer Erkrankung, die auf die Verabreichung von PPARα- und/oder PPARγ-Agonisten anspricht, wobei die Erkrankung, die auf die Verabreichung von PPARα- und/oder PPARγ-Agonisten anspricht, unabhängig ausgewählt ist aus der Gruppe bestehend aus kardiometabolischen Störungen, Typ-2-Diabetes (T2D), Fettleibigkeit, metabolischem Syndrom, Hypercholesterinämie, Hypertriglyceridämie, primärer Chylomikronämie, Hyperlipoproteinämie, familiärer Dysbetalipoproteinämie und neurodegenerativen Erkrankungen.

7. Verbindung unabhängig ausgewählt aus der Gruppe bestehend aus 1, 2, 3, 4, 4a, 4b, 4c, 5, 6, 7, 8, 9, 10, 11, 12 und 13: 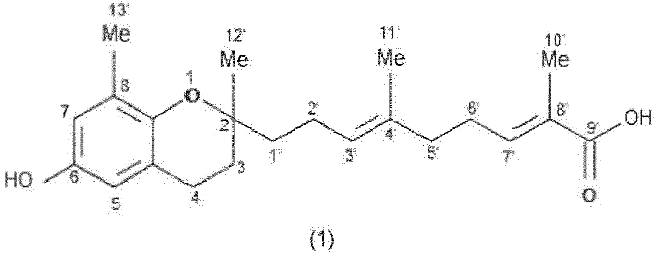 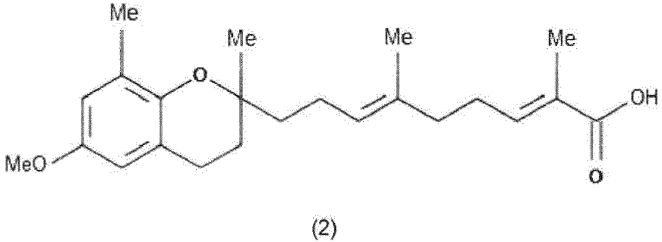 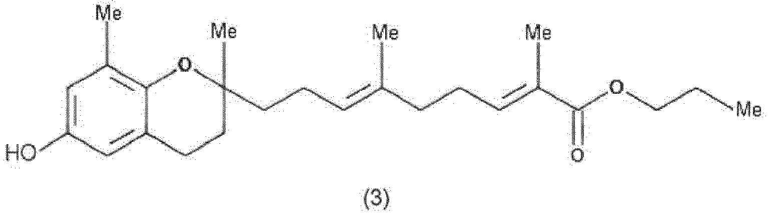 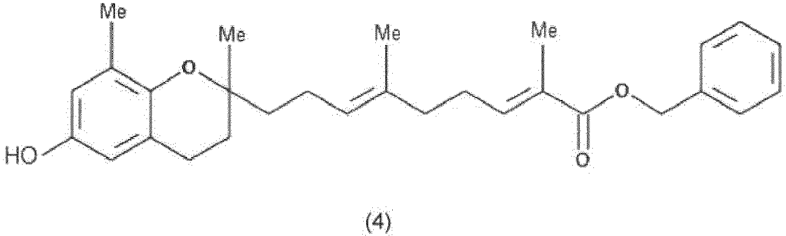 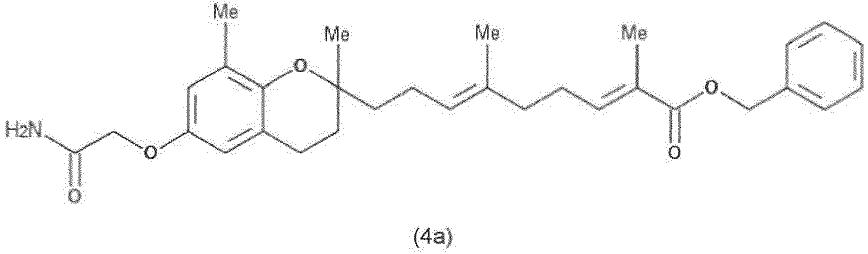 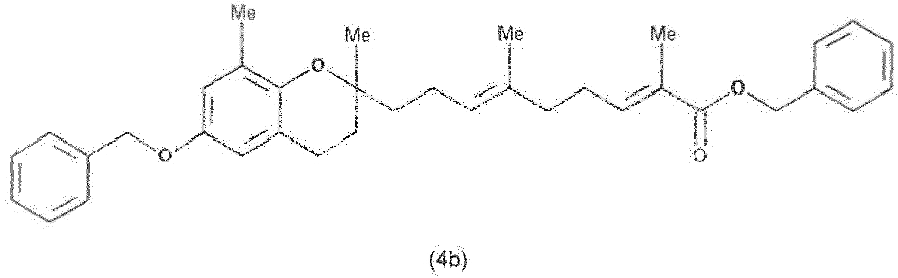 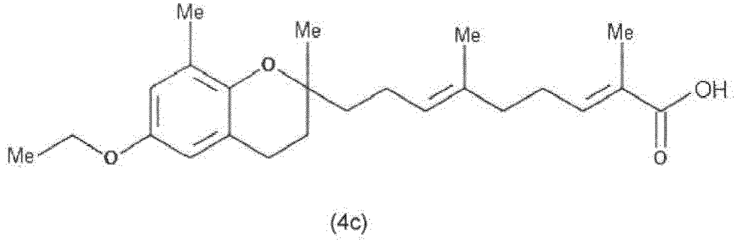 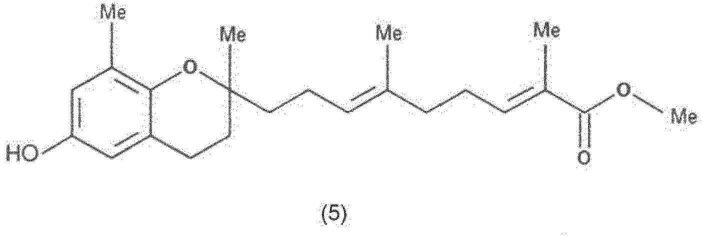 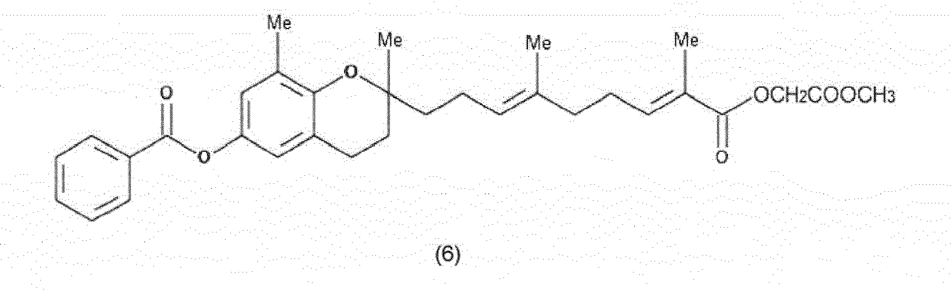 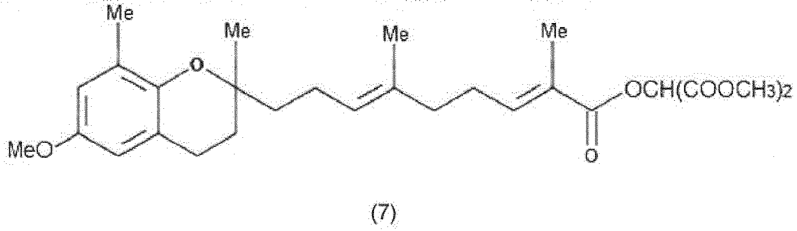 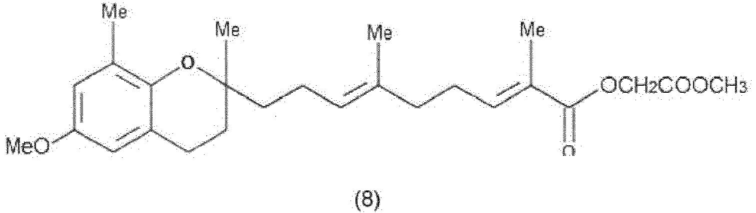 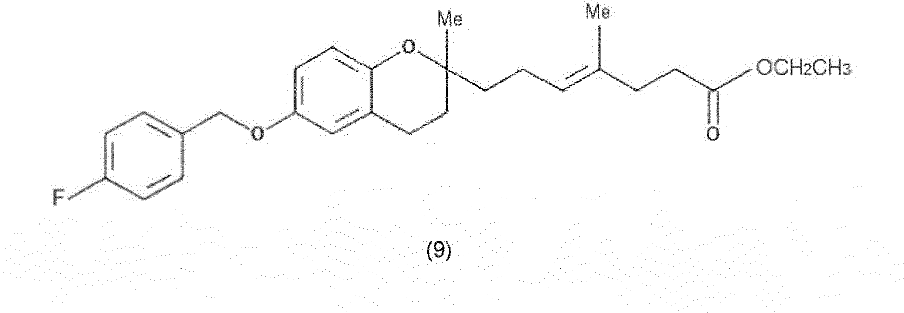 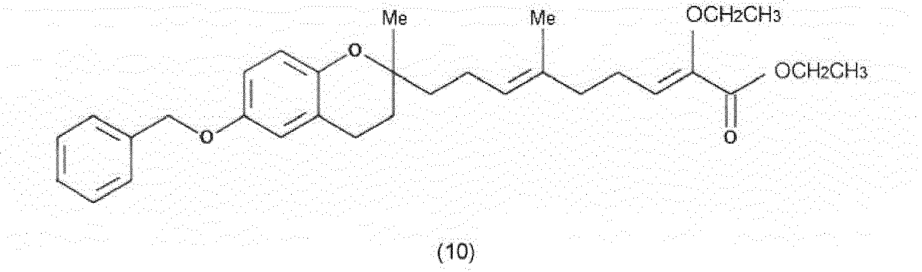 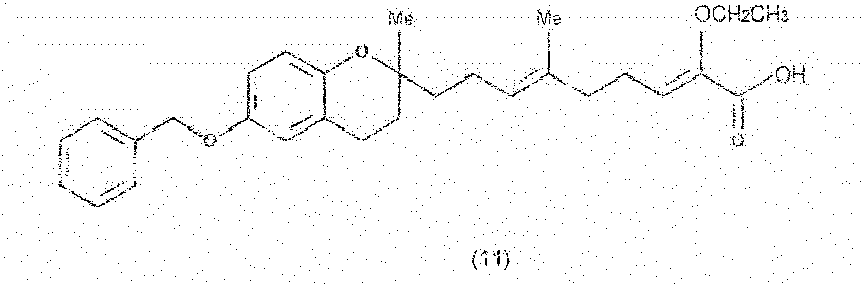 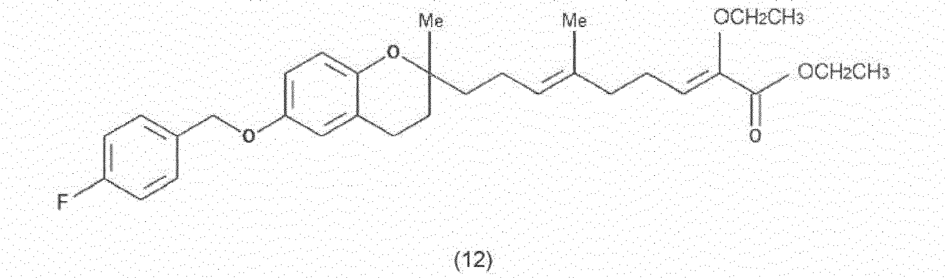 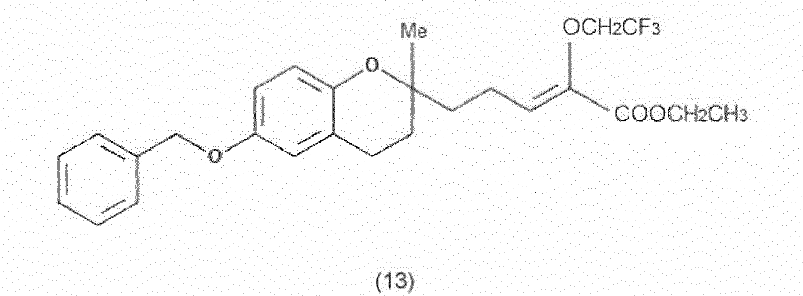 zur Verwendung bei der Behandlung einer Erkrankung, die auf die Verabreichung von PPARα- und/oder PPARγ-Agonisten anspricht, wobei die Erkrankung, die auf die Verabreichung von PPARα- und/oder PPARγ-Agonisten anspricht, unabhängig ausgewählt ist aus der Gruppe bestehend aus kardiovaskulären Erkrankungen, Typ-2-Diabetes (DT2), Fettleibigkeit, metabolischem Syndrom, Hypercholesterinämie, Hypertriglyceridämie, primärer Chylomikronämie, Hyperlipoproteinämie, familiärer Dysbetalipoproteinämie, Entzündung und neurodegenerativen Erkrankungen.

8. Pharmazeutische Zusammensetzung, umfassend eine wirksame Menge mindestens eines Verbindung, des unabhängig ausgewählt ist aus der Gruppe bestehend aus 1, 2, 3, 4, 4a, 4b, 4c, 5, 6, 7, 8, 9, 10, 11, 12 und 13: 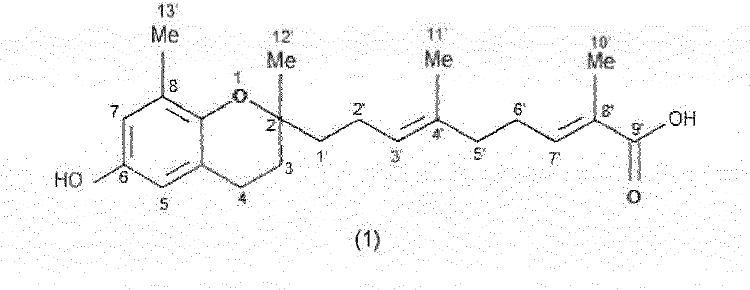 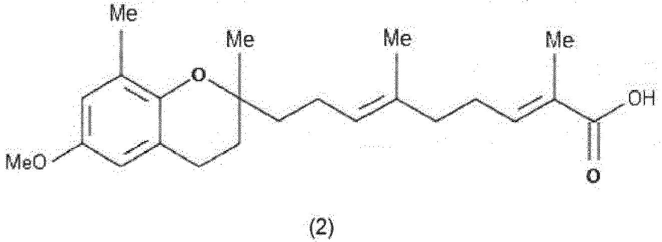 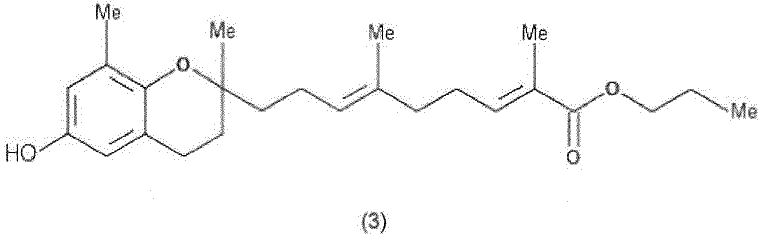 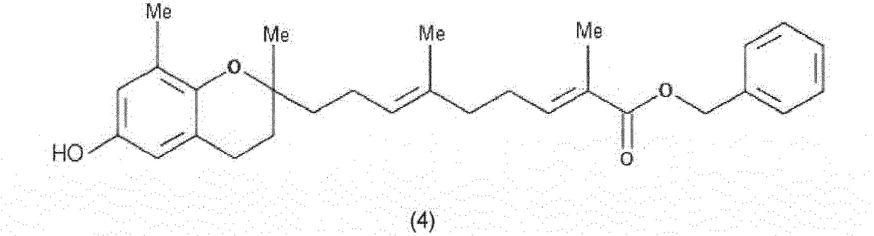 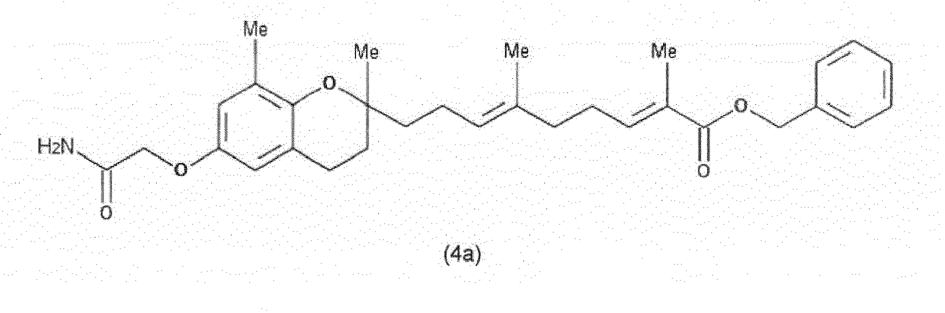 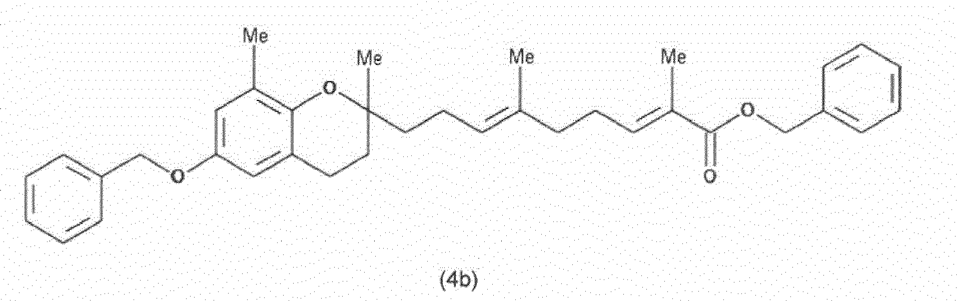 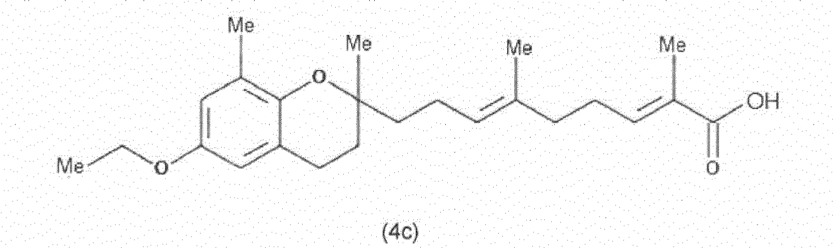 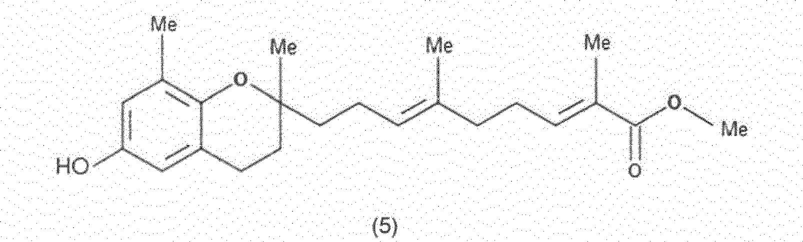 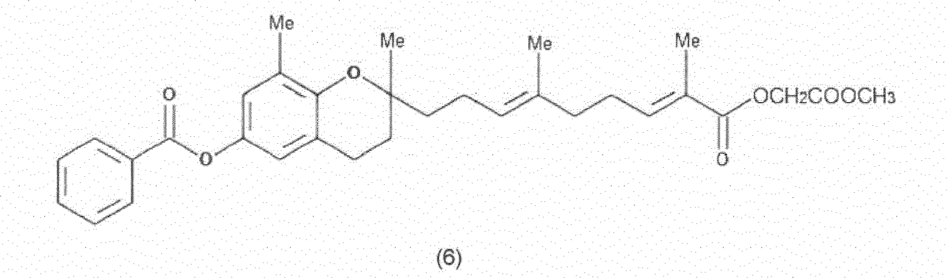 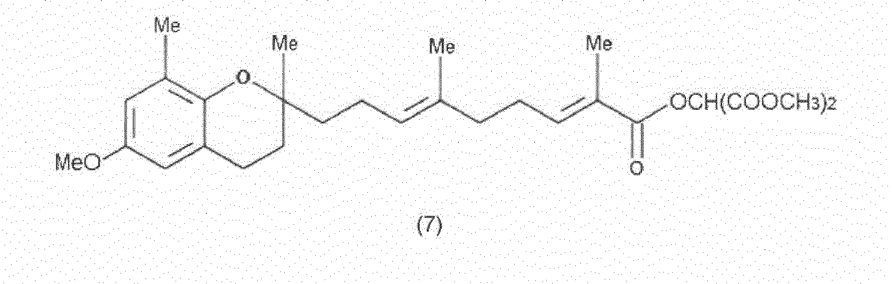 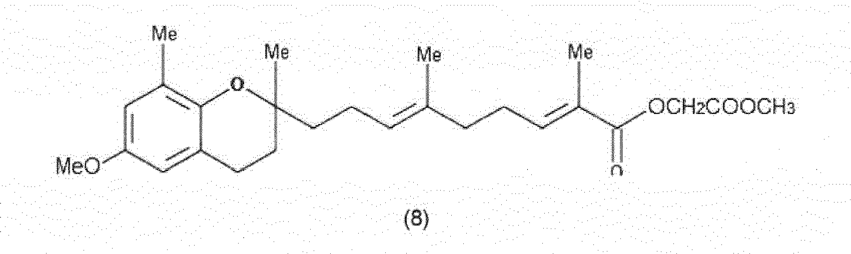 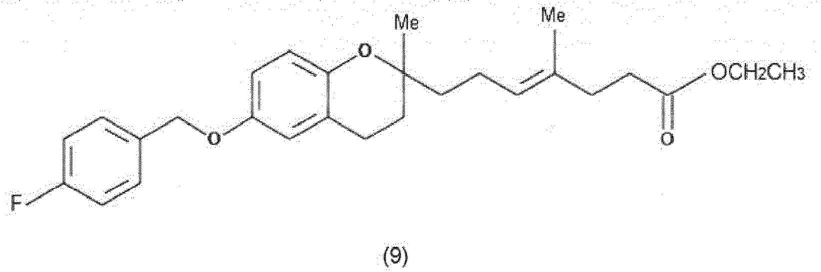 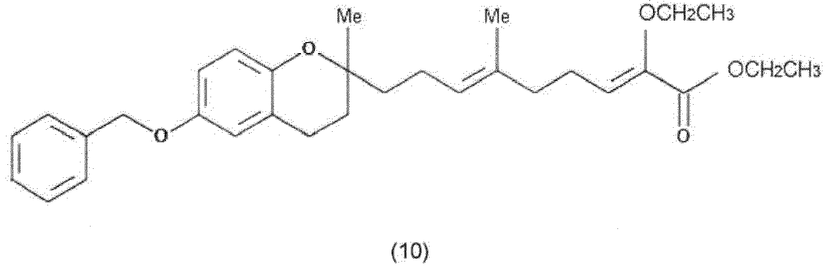 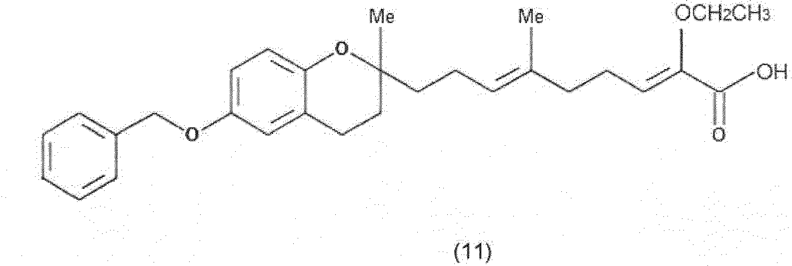 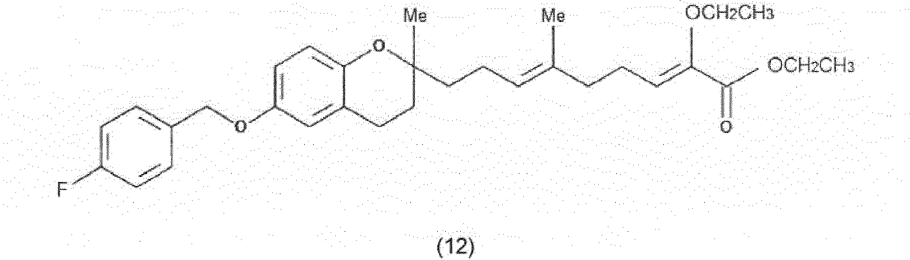 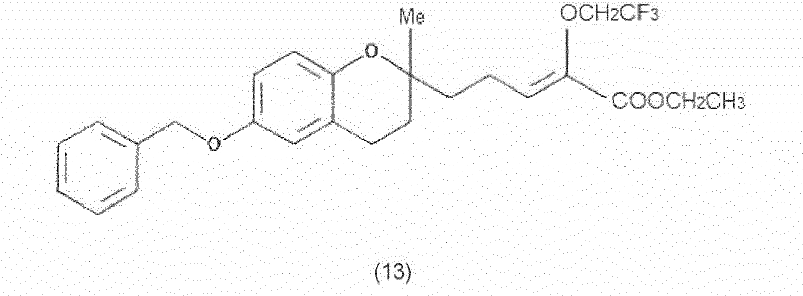 und mindestens einen pharmazeutisch verträglichen Itilfsstoff zur Verwendung bei der Behandlung einer Erkrankung, die auf die Verabreichung von PPARα- und/oder PPARγ-Agonisten anspricht, wobei die Erkrankung, die auf die Verabreichung von PPARα- und/oder PPARγ-Agonisten anspricht, unabhängig ausgewählt ist aus der Gruppe bestehend aus kardiovaskulären Störungen, Typ-2-Diabetes (DT2), Fettleibigkeit, metabolischem Syndrom, Hypercholesterinämie, Hypertriglyceridämie, primärer Chylomikronämie, Hyperlipoproteinämie, familiärer Dysbetalipoproteinämie, Entzündung und neurodegenerativen Erkrankungen.

## Revendications

1. Composé choisi dans le groupe constitué par 3, 4, 4a, 4b, 4c, 6, 7, 8, 9, 10, 11,12 et 13 :

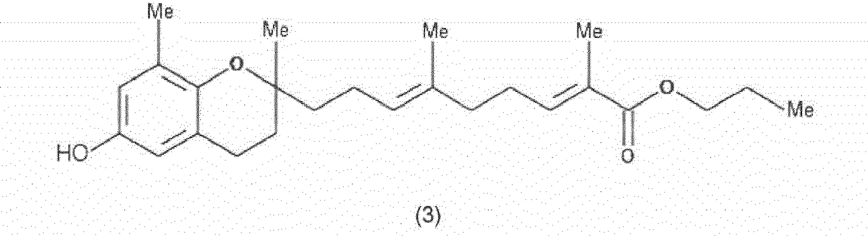

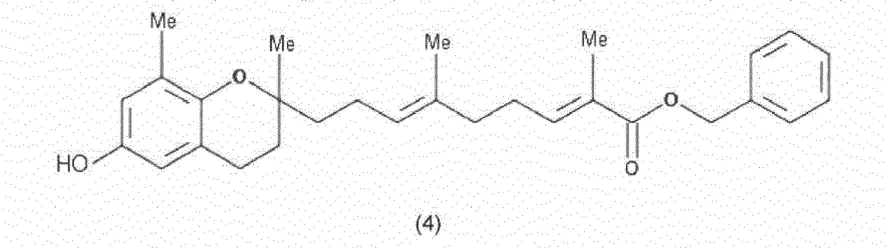

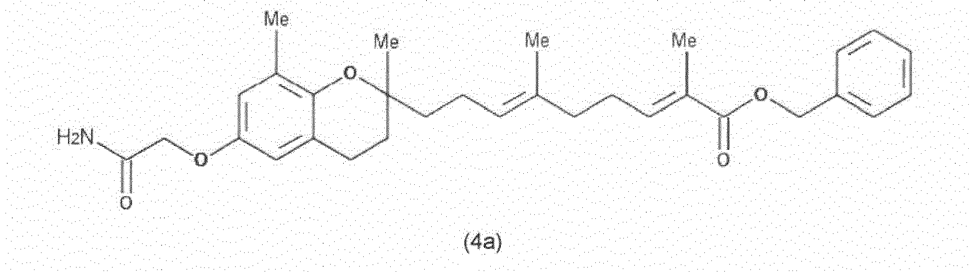

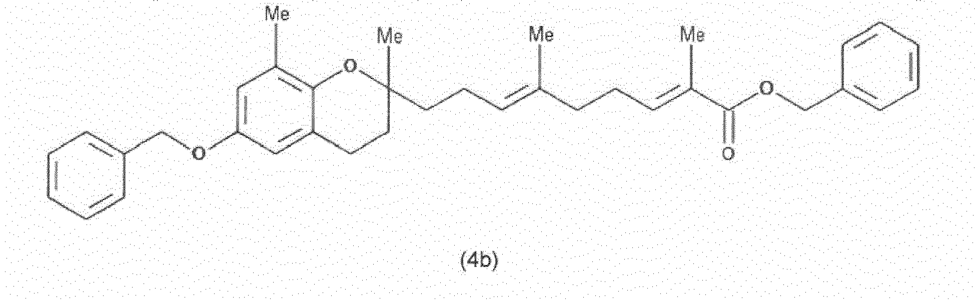

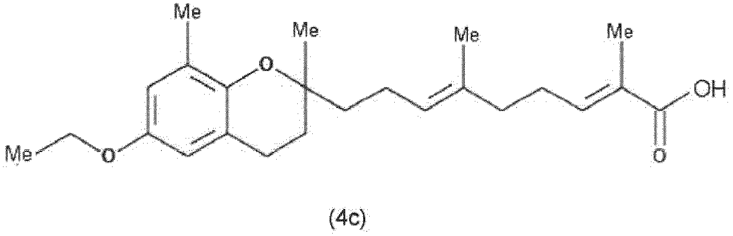

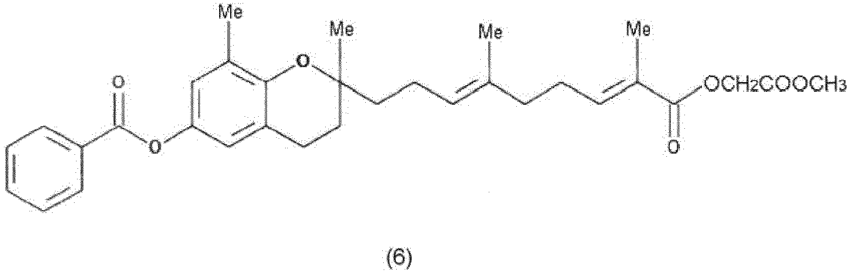

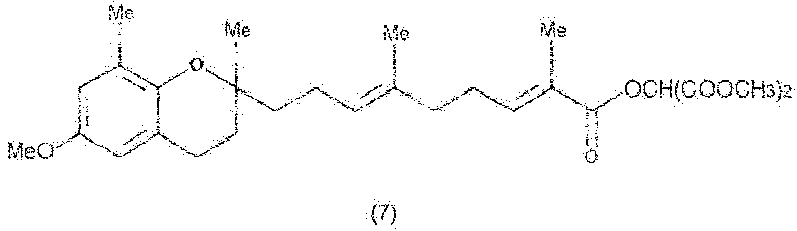

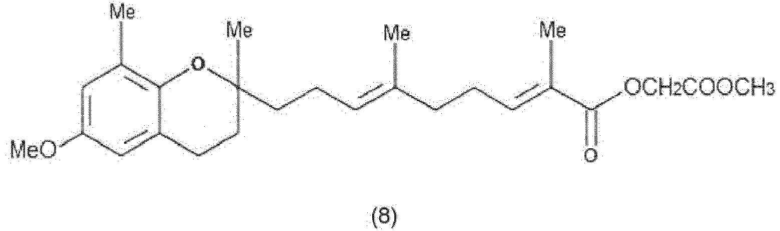

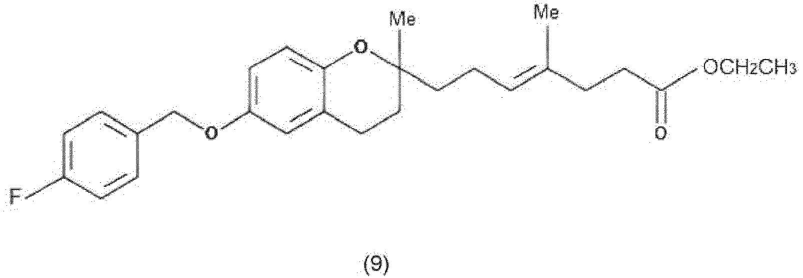

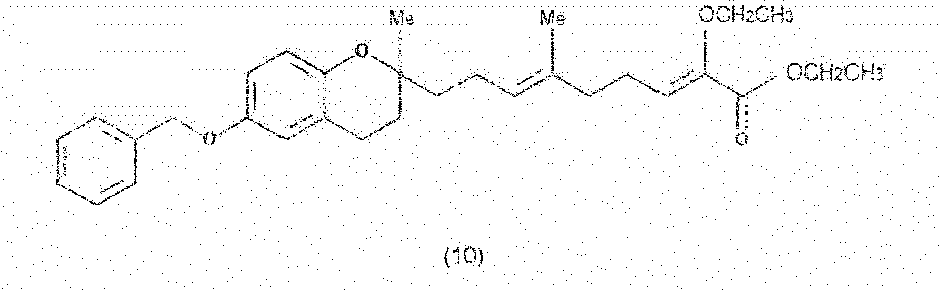

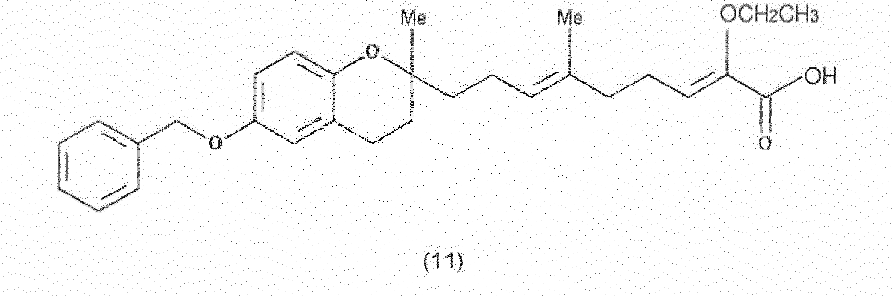

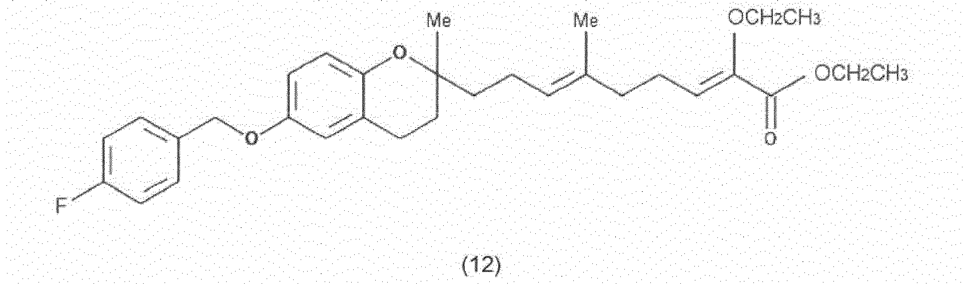

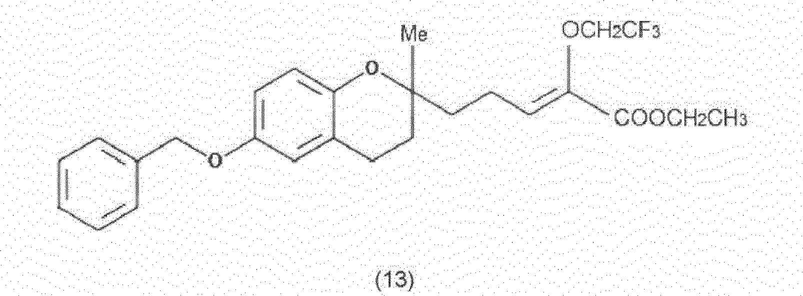

2. Composé selon la revendication 1, destinée à être utilisée comme médicament.

3. Composé selon la revendication 1, destiné à être utilisé dans le traitement d'une maladie qui répond à l'administration d'agonistes PPARα et/ou PPARγ, dans lequel la maladie qui répond à l'administration d'agonistes PPARα et/ou PPARγ est indépendamment choisie dans le groupe constitué par les agonistes PPARα et/ou PPARγ comprenant les troubles cardiométaboliques, le diabète de type 2 (T2D), l'obésité, le syndrome métabolique, l'hypercholestérolémie, l'hypertriglycéridémie, la chylomicronémie primaire, l'hyperlipoprotéinémie, la dysbétalipoprotéinémie familiale et les maladies neurodégénératives.

4. Composition pharmaceutique comprenant une quantité efficace d'au moins un composé de formule I, selon la revendication 1, et d'au moins un excipient pharmaceutiquement acceptable.

5. Composition pharmaceutique selon la revendication 4, destinée à être utilisée comme médicament.

6. Composition pharmaceutique selon la revendication 4, destinée à être utilisée dans le traitement d'une maladie qui répond à l'administration d'agonistes PPARα et/ou PPARγ, dans laquelle la maladie qui répond à l'administration d'agonistes PPARα et/ou PPARγ est indépendamment choisie dans le groupe constitué par les troubles cardiométaboliques, le diabète de type 2 (T2D), l'obésité, le syndrome métabolique, l'hypercholestérolémie, l'hypertriglycéridémie, la chylomicronémie primaire, l'hyperlipoprotéinémie, la dysbétalipoprotéinémie familiale et les maladies neurodégénératives.

7. Composé choisi indépendamment dans le groupe constitué par 1, 2, 3, 4, 4a, 4b, 4c, 5, 6, 7, 8, 9, 10, 11, 12 et 13 : 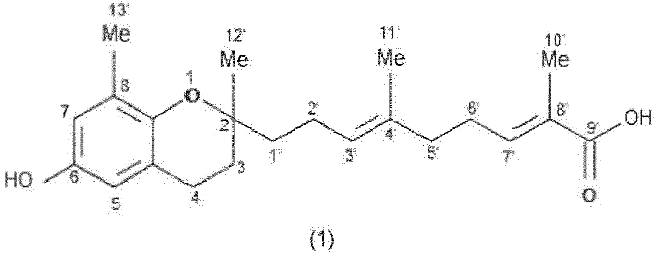 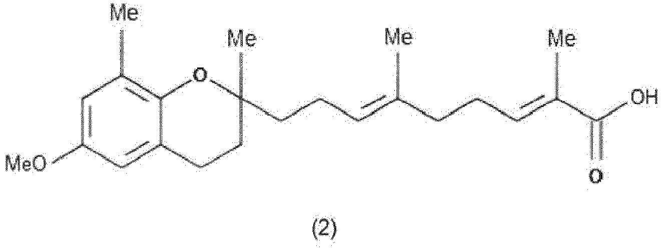 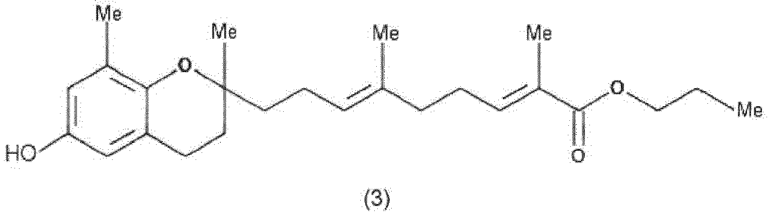 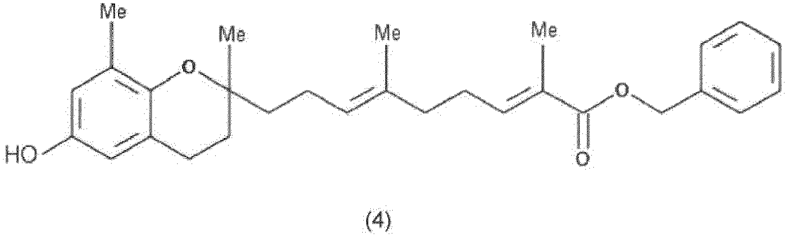 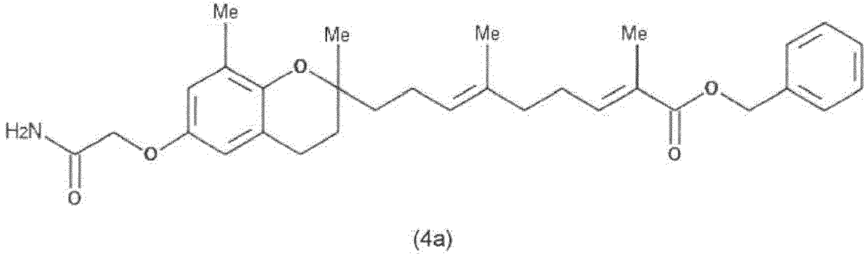 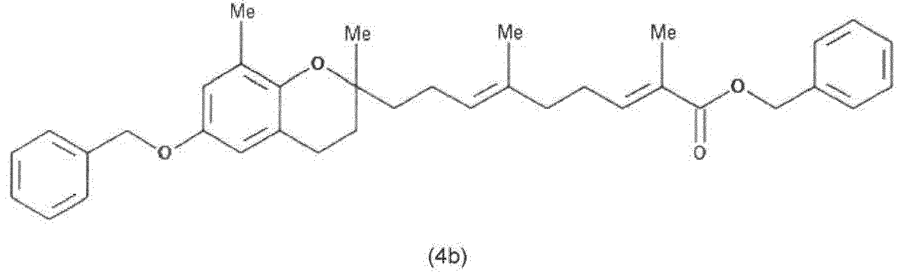 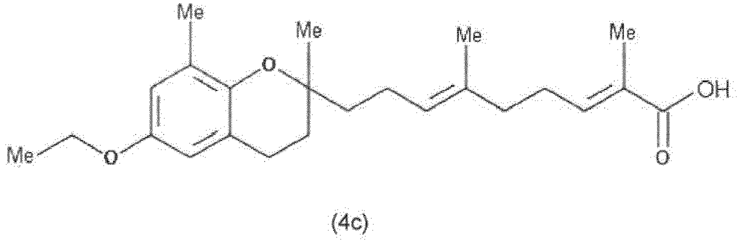 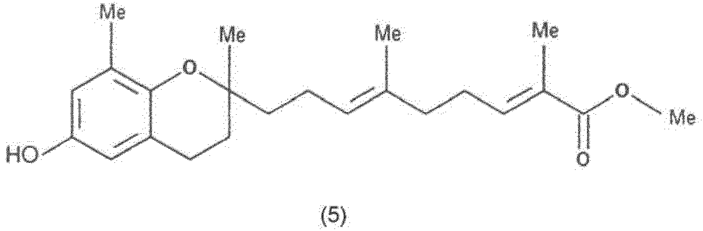 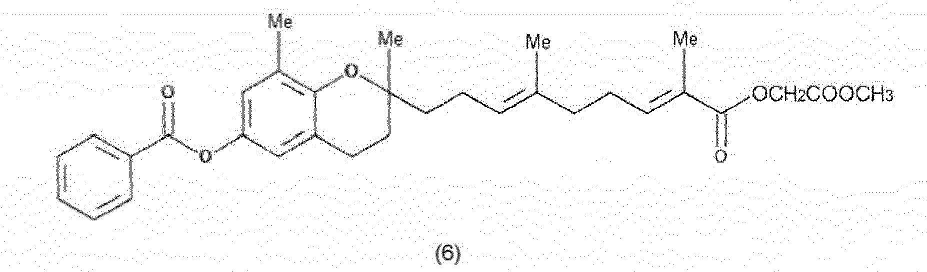 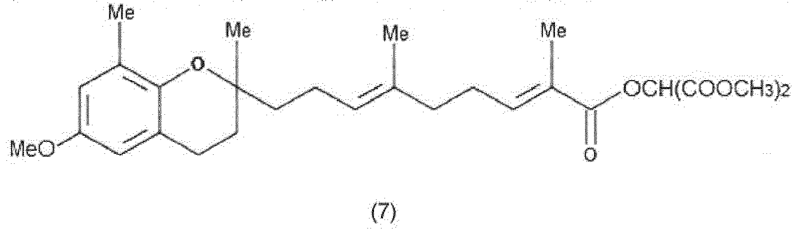 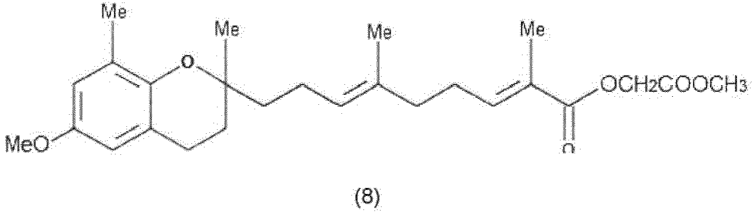 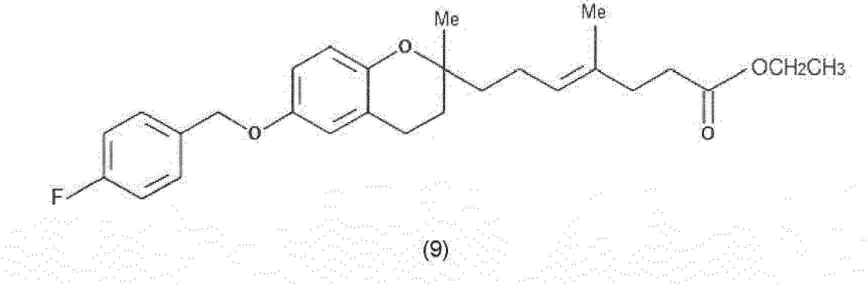 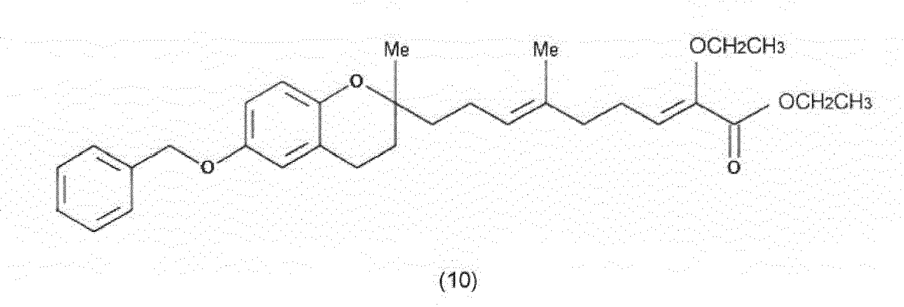 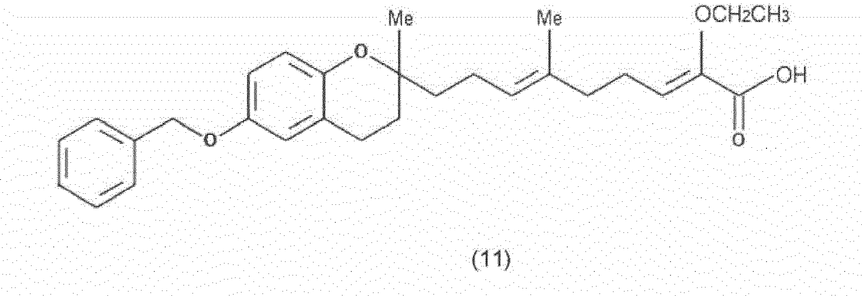 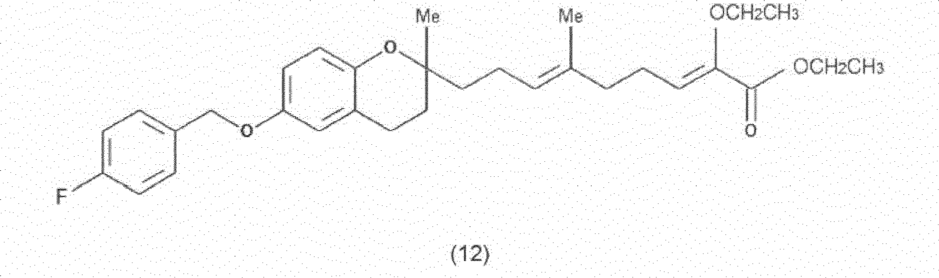 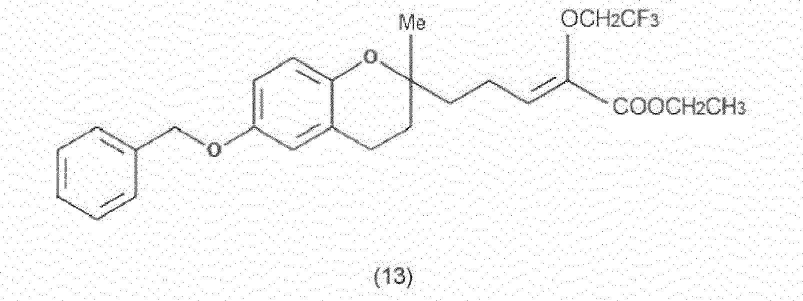 destinée à être utilisée dans le traitement d'une maladie qui répond à l'administration d'agonistes PPARα et/ou PPARγ, dans laquelle la maladie qui répond à l'administration d'agonistes PPARα et/ou PPARγ est indépendamment sélectionnée dans le groupe constitué de troubles cardio-vasculaires, diabète de type 2 (DT2), obésité, syndrome métabolique, hypercholestérolémie, hypertriglycéridémie, chylomicronémie primaire, hyperlipoprotéinémie, dysbétalipoprotéinémie familiale, inflammation, et maladies neurodégénératives.

8. Composition pharmaceutique comprenant une quantité efficace d'au moins un composé choisi indépendamment dans le groupe constitué par 1, 2, 3, 4, 4a, 4b, 4c, 5, 6, 7, 8, 9, 10, 11, 12 et 13 : 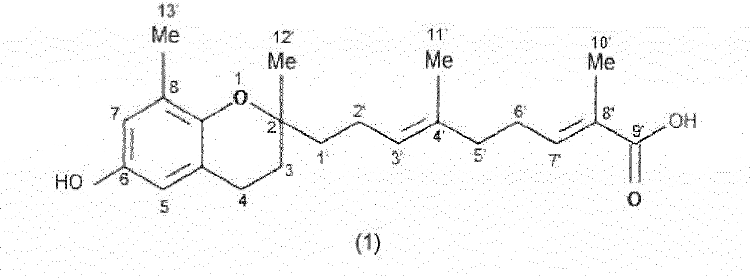 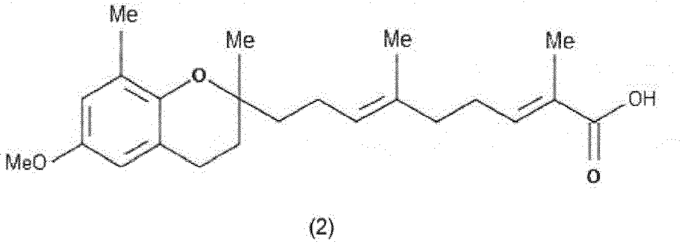 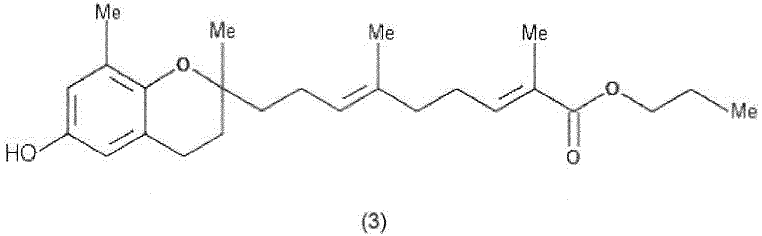 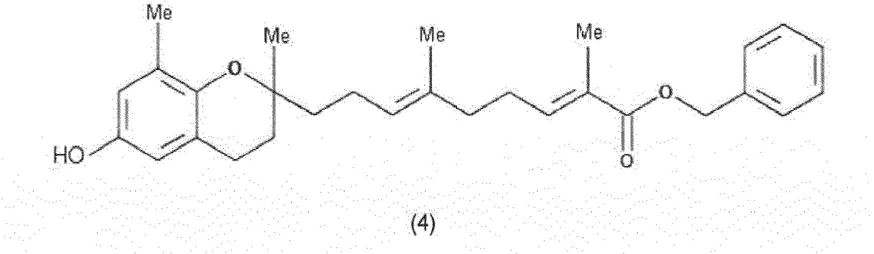 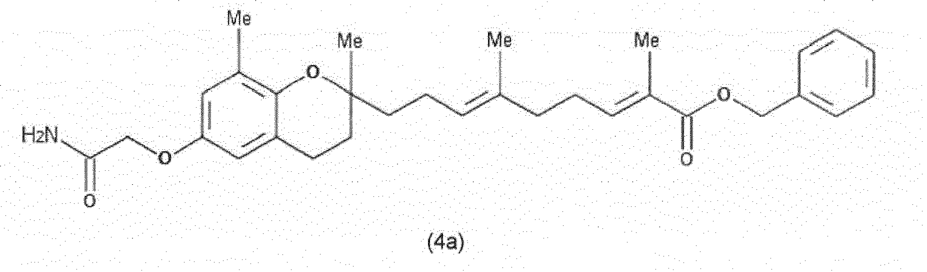 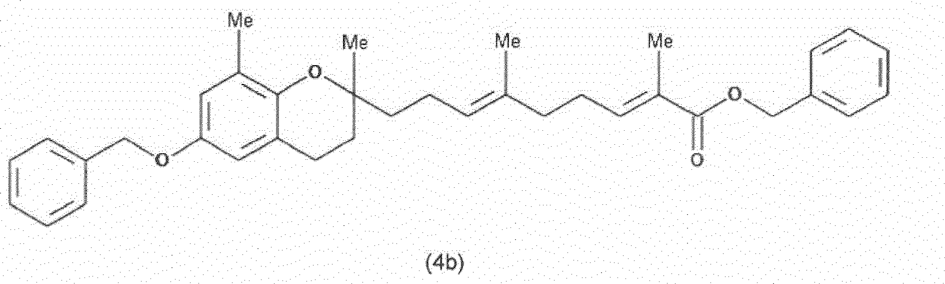 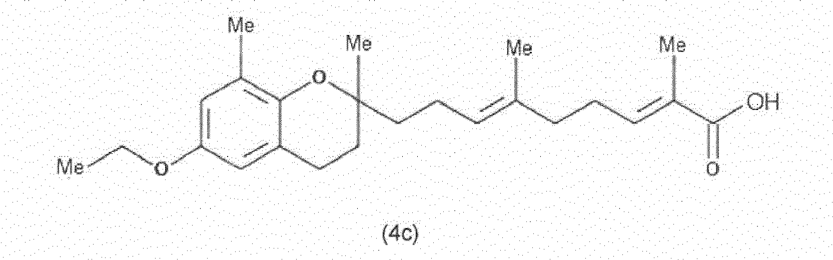 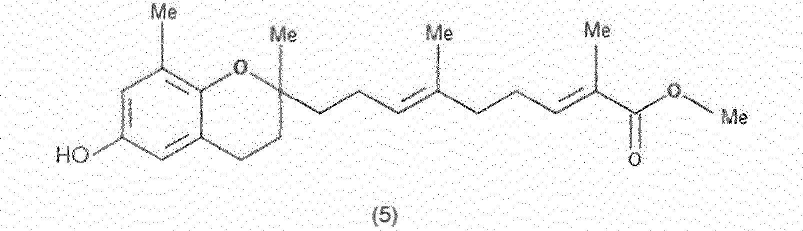 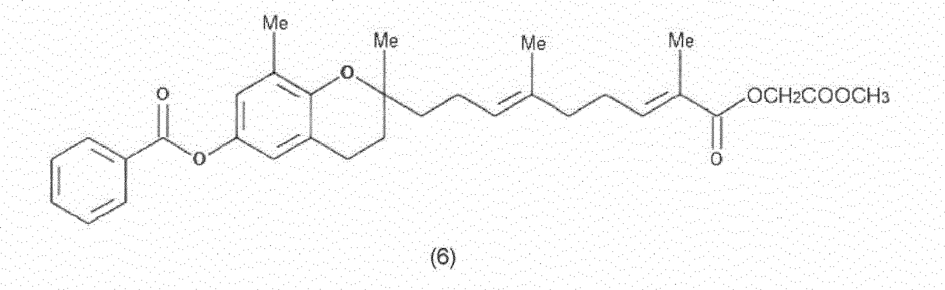 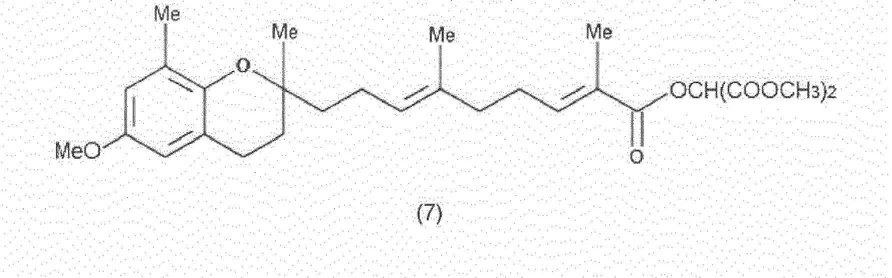 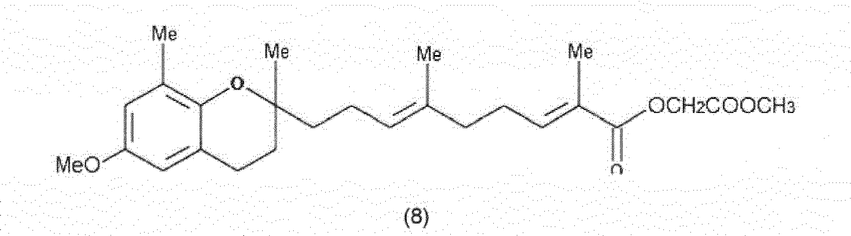 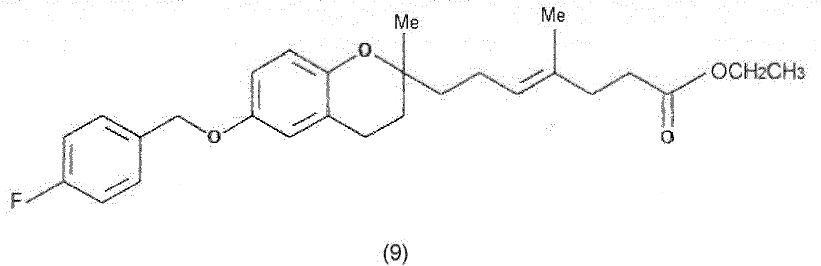 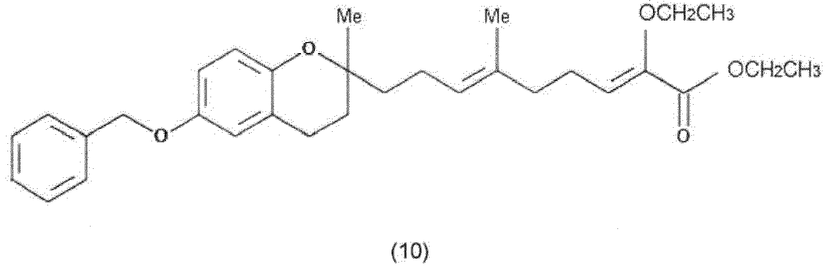 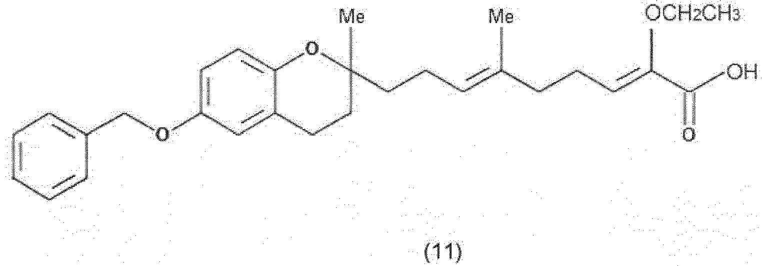 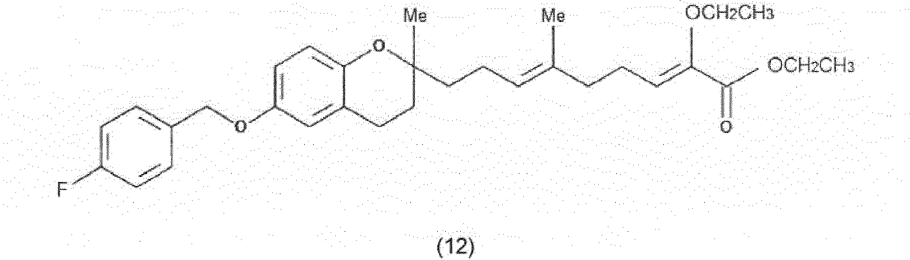 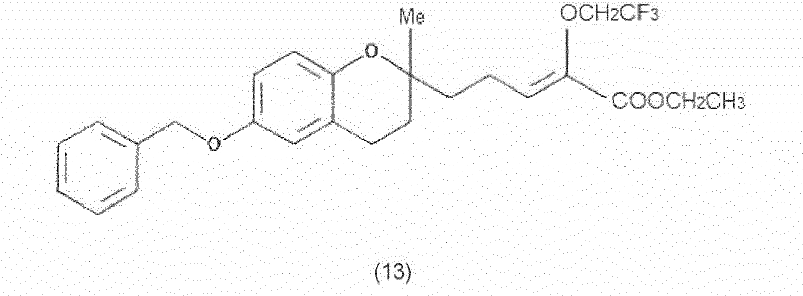 et au moins un excipient pharmaceutiquement acceptable, destiné à être utilisé dans le traitement d'une maladie qui répond à l'administration d'agonistes PPARα et/ou PPARγ, dans lequel la maladie qui répond à l'administration d'agonistes PPARα et/ou PPARγ est indépendamment choisie dans le groupe constitué de troubles cardiovasculaires, de diabète de type 2 (DT2), d'obésité, de syndrome métabolique, d'hypercholestérolémie, d'hypertriglycéridémie, de chylomicronémie primaire, d'hyperlipoprotéinémie, de dysbétalipoprotéinémie familiale, d'inflammation, et de maladies neurodégénératives.
